# EUROPEAN PATENT SPECIFICATION

(11) **EP 4 049 665 B1**
(45) Date of publication and mention of the grant of the patent: **12.03.2025**
(21) Application number: 21214003.2
(22) Date of filing: 15.03.2017
(51) Int. Cl.: A61K 35/28, C12N 5/077, C12N 5/0789, A61P 1/04, A61P 1/12, A61P 3/00, A61P 5/00, A61P 7/00, A61P 7/04, A61P 7/06, A61P 13/02, A61P 17/00, A61P 17/14, A61P 3/10, A61P 19/08, A61P 25/00, A61P 31/18

(54) **METHODS AND COMPOSITIONS RELATING TO HEMATOPOIETIC STEM CELL EXPANSION**
VERFAHREN UND ZUSAMMENSETZUNGEN IM ZUSAMMENHANG MIT DER EXPANSION HÄMATOPOETISCHER STAMMZELLEN
PROCÉDÉS ET COMPOSITIONS ASSOCIÉES À L'EXPANSION DE CELLULES SOUCHES HÉMATOPOÏÉTIQUES

(30) Priority: 15.03.2016 US 201662308324 P; 16.03.2016 US 201662309140 P
(43) Date of publication of application: 31.08.2022
(62) Divisional of application: 17767443.9
(73) Proprietor: The Children's Medical Center Corporation, Boston, MA 02115 (US)
(72) Inventor: ROSSI, Derrick J., Newton, MA 02459 (US); EBINA, Wataru, New York, NY 10017 (US)
(74) Representative: Keltie LLP

(56) References cited:
- WO-A1-2016/210292
- LAURE A. MOUTOUH-DE PARSEVAL ET AL: "Pomalidomide and lenalidomide regulate erythropoiesis and fetal hemoglobin production in human CD34+ cells", JOURNAL OF CLINICAL INVESTIGATION, vol. 118, no. 1, 2 January 2008 (2008-01-02), GB, pages 248 - 258, XP055611665, ISSN: 0021-9738, DOI: 10.1172/JCI32322
- WATARU EBINA ET AL: "Combinatorial Pathway Modulation toward Ex Vivo Maintenance and Propagation of Hematopoietic Stem Cells", HARVARD UNIVERSITY, 1 March 2016 (2016-03-01), XP055611673, ISBN: 978-0-355-03038-9, Retrieved from the Internet <URL:https://dash.harvard.edu/bitstream/handle/1/33493367/EBINA-DISSERTATION-2016.pdf?sequence=4&isAllowed=y> [retrieved on 20190808]
- YOSHIDA TOSHIMI ET AL: "Ikaros fingers on lymphocyte differentiation", INTERNATIONAL JOURNAL OF HEMATOLOGY, ELSEVIER SCIENCE PUBLISHERS, NL, vol. 100, no. 3, 2 August 2014 (2014-08-02), pages 220 - 229, XP035387771, ISSN: 0925-5710, [retrieved on 20140802], DOI: 10.1007/S12185-014-1644-5

## Description

### TECHNICAL FIELD

The technology described herein relates to compositions for the *ex vivo* expansion, enrichment, and maintenance of hematopoietic stem cells.

### BACKGROUND

While hematopoietic stem cells have significant therapeutic potential, a limitation that has hindered their use in the clinic has been the difficulty associated with obtaining sufficient numbers of these cells. In particular, hematopoietic stem cells are resistant to maintenance, propagation, and expansion *ex vivo.* Another challenge to be overcome in order to further develop the use of hematopoietic stem cells (HSCs) as a therapeutic modality is the loss of multi-potency that can occur when these cells are cultured *ex vivo.* There is currently a need for compositions and methods for the *ex vivo* maintenance, propagation, and expansion of HSCs that preserve the multi-potency and hematopoietic functionality of these cells. Described herein is the inventors' discovery of a number of compounds that permit hematopoietic stem cell *ex vivo* maintenance, propagation, expansion and enrichment. The compositions of the invention address the challenges posed by conventional HSC therapies by providing strategies for maintaining, propagating and expanding these cells and also enriching heterogeneous cell populations with HSCs while preserving the ability of the *ex vivo* cultured cells to self-renew and differentiate into a multitude of different blood cell types both *in vitro* and upon transplantation into a recipient.

Laure A. Moutouh-de Parseval et al: "Pomalidomide and lenalidomide regulate erythropoiesis and fetal hemoglobin production in human CD34+ cells", Journal of Clinical Investigation, vol. 118, no. 1, pages 248-258, describes that pomalidomide, used alone or in combination with hydroxyurea, may improve erythropoiesis and increase the ratio of fetal to adult hemoglobin, and supports the evaluation of pomalidomide as an innovative new therapy for β-hemoglobinopathies.

### SUMMARY OF THE INVENTION

In a first aspect, the invention provides a composition comprising:
a. one or more agents that reduce the level of an ikaros family member transcription factor in a cell, wherein the one or more agents are selected from the group consisting of pomalidomide and lenalidomide; and
b. one or more agents that inhibit TGFβ signaling, wherein the one or more agents that inhibit TGFβ signaling comprise a TGFβ receptor inhibitor, and wherein said TGFβ receptor inhibitor is selected from the group consisting of ALK5 inhibitor II, LY364947, DMH1, and A83-01.

In some embodiments, the composition is for producing an expanded population of hematopoietic stem cells ex vivo.

In some embodiments, the composition further comprises one or more of:
c. one or more compounds listed in Table 1;
d. one or more agents that inhibit histone demethylation;
e. one or more agents that inhibit histone deacetylation; and
f. one or more agents that inhibit aryl hydrocarbon receptor signaling.

In some embodiments, said one or more compounds listed in Table 1 comprise UM171.

In some embodiments, said one or more agents that inhibit histone demethylation comprise a histone demethylase inhibitor, and wherein said histone demethylase inhibitor is a LSD1 inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine.

In some embodiments, said one or more agents that inhibit histone deacetylation comprise a histone deacetylase inhibitor selected from the group consisting of Trichostatin A, valproic acid, butyrylhydroxamic acid, and istodax.

In some embodiments, said one or more agents that inhibit aryl hydrocarbon receptor signaling comprise SR1.

In some embodiments, said composition further comprising one or more agents selected from the group consisting of:
a. a compound that inhibits a protein that propagates p38 signaling comprising SB203580 and SB202190; and
b. a compound that inhibits a protein that promotes β-catenin degradation selected from the group consisting of CHIR99021, lithium chloride, BIO, and FGF2.

In some embodiments, said one or more agents or compounds are present in amounts that are sufficient to (i) produce a population of cells enriched with hematopoietic stem cells, or (ii) maintain the hematopoietic stem cell functional potential of said population of hematopoietic stem cells for at least two days.

In some embodiments, said one or more agents or compounds are present in an aqueous solution; or as a lyophilized solid.

In some embodiments, said one or more agents or compounds are present in amounts that are sufficient to:
(i) stimulate expansion of said population of cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture;
(ii) enrich the population of cells with hematopoietic stem cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture;
(iii) maintain long term engraftment potential of said hematopoietic stem cells post-transplantation after having contacted said cells in culture for two or more days; or
(iv) increase expression of a Notch target gene in said population of hematopoietic stem cells, relative to a control population of hematopoietic stem cells cultured under, same conditions but without the one or more agents recited above, and for the same time, optionally wherein the Notch target gene is Hes 1 or Myc.

Further described is a method of producing an expanded population of hematopoietic stem cells ex vivo, the method including contacting a population of hematopoietic stem cells with one or more agents that reduce the level of an ikaros family member transcription factor in a cell, wherein the one or more agents are present in amounts that are sufficient to produce an expanded population of hematopoietic stem cells.

Also described is a method of enriching a population of cells with hematopoietic stem cells *ex vivo,* the method including contacting a population of hematopoietic stem cells with one or more agents that reduce the level of an ikaros family member transcription factor in a cell, wherein the one or more agents are present in amounts that are sufficient to produce a population of cells enriched with hematopoietic stem cells.

Further described is a method of maintaining the hematopoietic stem cell functional potential of a population of hematopoietic stem cells *ex vivo* for at least two days, the method including contacting a population of hematopoietic stem cells with one or more agents that reduce the level of an ikaros family member transcription factor in a cell, wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the one or more agents.

In some examples, the one or more agents are present in amounts that are sufficient to increase expression of a Notch target gene in the population of hematopoietic stem cells relative to a control population of hematopoietic stem cells cultured under the same conditions and for the same time as the first population of hematopoietic stem cells but not contacted with the one or more agents.

In some examples, the Notch target gene is selected from the group consisting of Hes1 and Myc.

In some examples, the ikaros family member transcription factor is selected from the group consisting of ikaros, aiolos, and helios. In some examples, the one or more agents that reduce the level of an ikaros family member transcription factor in a cell promote ubiquitination of the ikaros family member transcription factor.

In some examples, the one or more agents that reduce the level of an ikaros family member transcription factor in a cell include a compound represented by formula (**1**), 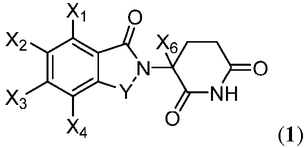
wherein Y is C=O or CH₂;
each of X₁, X₂, X₃, and X₄ is independently hydrogen or -NHX₅;
X₅ is hydrogen or an alkyl group of from 1 to 8 carbon atoms; and
X₆ is hydrogen, an alkyl group of from 1 to 8 carbon atoms, a benzyl group, or a halogen atom.

In some examples, the compound represented by formula (1) is selected from the group consisting of pomalidomide, lenalidomide, and thalidomide.

In some examples, the method includes contacting the population of hematopoietic stem cells with one or more compounds selected from UM171, structural analogs thereof, and the compounds listed in Table 1. In some examples, the one or more compounds listed in Table 1 includes UM171.

In some examples, the method further includes contacting the population of hematopoietic stem cells with one or more agents that inhibit TGFβ signaling. The one or more agents that inhibit TGFβ signaling may include a TGFβ receptor inhibitor. In some examples, the TGFβ receptor inhibitor is selected from the group consisting of ALK5 inhibitor II, LY364947, DMH1, and A83-01. In some examples, the TGFβ receptor inhibitor is A83-01.

In some examples, the method includes contacting the population of hematopoietic stem cells with one or more agents that modulate histone methylation. In some examples, the one or more agents that modulate histone methylation activate histone methylation, maintain histone methylation, or inhibit histone demethylation. For instance, the one or more agents that modulate histone methylation may include a histone demethylase inhibitor. In some examples, the histone demethylase inhibitor is a LSD1 inhibitor, such as a LSD1 inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine. In some examples, the LSD1 inhibitor is Tranylcypromine.

In some examples, the method includes contacting the population of hematopoietic stem cells with one or more agents that modulate histone acetylation. In some examples, the one or more agents that modulate histone acetylation activate histone acetylation, maintain histone acetylation, or inhibit histone deacetylation. In some examples, the one or more agents that that modulate histone acetylation include a histone deacetylation inhibitor, such as a histone deacetylase inhibitor selected from the group consisting of Trichostatin A, valproic acid, butyrylhydroxamic acid, and istodax. In some examples, the histone deacetylase inhibitor is Trichostatin A.

In some examples, the method includes contacting the population of hematopoietic stem cells with one or more agents that exhibit one or more activities selected from the group consisting of:
a. inhibition of p38 signaling; and
b. activation of canonical Wnt signaling.

In some examples, the method includes contacting the population of hematopoietic stem cells with one or more agents that inhibit aryl hydrocarbon receptor signaling, such as SR1.

Also described is a method of producing an expanded population of hematopoietic stem cells *ex vivo,* the method including contacting a population of hematopoietic stem cells with one or more compounds listed in Table 1 and one or more agents that exhibit one or more activities selected from the group consisting of:
a. modulation of histone methylation;
b. inhibition of TGFβ signaling;
c. inhibition of p38 signaling;
d. activation of canonical Wnt signaling;
e. modulation of histone acetylation; and
f. inhibition of aryl hydrocarbon receptor signaling,
wherein the one or more compounds and one or more agents are present in amounts that are sufficient to produce an expanded population of hematopoietic stem cells.

Further described is a method of enriching a population of cells with hematopoietic stem cells *ex vivo,* the method including contacting a population of hematopoietic cells that contains one or more hematopoietic stem cells with one or more compounds listed in Table 1 and one or more agents that exhibit one or more activities selected from the group consisting of::
a. modulation of histone methylation;
b. inhibition of TGFβ signaling;
c. inhibition of p38 signaling;
d. activation of canonical Wnt signaling;
e. modulation of histone acetylation; and
f. inhibition of aryl hydrocarbon receptor signaling,
wherein the one or more compounds and one or more agents are present in amounts that are sufficient to produce a population of cells enriched with hematopoietic stem cells.

Further described is a method of maintaining the hematopoietic stem cell functional potential of a population of hematopoietic stem cells *ex vivo* for at least two days, the method including contacting a first population of hematopoietic stem cells with one or more compounds listed in Table 1 and one or more agents that exhibit one or more activities selected from the group consisting of::
a. modulation of histone methylation;
b. inhibition of TGFβ signaling;
c. inhibition of p38 signaling;
d. activation of canonical Wnt signaling;
e. modulation of histone acetylation; and
f. inhibition of aryl hydrocarbon receptor signaling,
wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the one or more compounds and one or more agents.

Also described is a method of producing an expanded population of hematopoietic stem cells *ex vivo,* the method including contacting a population of hematopoietic stem cells with one or more agents that inhibit aryl hydrocarbon receptor signaling and one or more agents that exhibit one or more activities selected from the group consisting of:
a. modulation of histone methylation;
b. inhibition of TGFβ signaling;
c. inhibition of p38 signaling;
d. activation of canonical Wnt signaling; and
e. modulation of histone acetylation,
wherein the one or more agents are present in amounts that are sufficient to produce an expanded population of hematopoietic stem cells.

Further described is a method of enriching a population of cells with hematopoietic stem cells *ex vivo,* the method including contacting a population of hematopoietic cells that contains one or more hematopoietic stem cells with one or more agents that inhibit aryl hydrocarbon receptor signaling and one or more agents that exhibit one or more activities selected from the group consisting of:
a. modulation of histone methylation;
b. inhibition of TGFβ signaling;
c. inhibition of p38 signaling;
d. activation of canonical Wnt signaling; and
e. modulation of histone acetylation,
wherein the one or more agents are present in amounts that are sufficient to produce a population of cells enriched with hematopoietic stem cells.

Also described is a method of maintaining the hematopoietic stem cell functional potential of a population of hematopoietic stem cells *ex vivo* for at least two days, the method including contacting a first population of hematopoietic stem cells with one or more agents that inhibit aryl hydrocarbon receptor signaling and one or more agents that exhibit one or more activities selected from the group consisting of:
a. modulation of histone methylation;
b. inhibition of TGFβ signaling;
c. inhibition of p38 signaling;
d. activation of canonical Wnt signaling; and
e. modulation of histone acetylation,
wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the one or more agents.

Further described is a method of producing an expanded population of hematopoietic stem cells *ex vivo,* the method including contacting a population of hematopoietic stem cells with one or more agents that activate a ubiquitin ligase complex including cereblon, wherein the one or more agents are present in amounts that are sufficient to produce an expanded population of hematopoietic stem cells.

Also described is a method of enriching a population of cells with hematopoietic stem cells *ex vivo,* the method including contacting a population of hematopoietic stem cells with one or more agents that activate a ubiquitin ligase complex including cereblon, wherein the one or more agents are present in amounts that are sufficient to produce a population of cells enriched with hematopoietic stem cells.

Further described is a method of maintaining the hematopoietic stem cell functional potential of a population of hematopoietic stem cells *ex vivo* for at least two days, the method including contacting a first population of hematopoietic stem cells with one or more agents that activate a ubiquitin ligase complex including cereblon, wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the one or more agents.

In some examples, the one or more agents that activate a ubiquitin ligase complex including cereblon are represented by formula (**1**). In some examples, the one or more agents that activate a ubiquitin ligase complex including cereblon are selected from the group consisting of pomalidomide, lenalidomide, and thalidomide.

In some examples, the method includes contacting the population of hematopoietic stem cells with one or more compounds selected from UM171, structural analogs thereof, and the compounds listed in Table 1.

In some examples, the method includes contacting the population of hematopoietic stem cells with a TGFβ receptor inhibitor selected from the group consisting of ALK5 inhibitor II, LY364947, DMH1, and A83-01.

In some examples, the method includes contacting the population of hematopoietic stem cells with a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine.

In some examples, the method includes contacting the population of hematopoietic stem cells with a histone deacetylase inhibitor selected from the group consisting of Trichostatin A, valproic acid, butyrylhydroxamic acid, and istodax.

In some examples, the method includes contacting the population of hematopoietic stem cells with one or more agents selected from the group consisting of:
a. a compound that inhibits a protein that propagates p38 signaling including SB203580; and
b. a compound that inhibits a protein that promotes β-catenin degradation selected from the group consisting of CHIR99021, lithium chloride, BIO, and FGF2.

In some examples, the method includes contacting the population of hematopoietic stem cells with one or more agents that inhibit aryl hydrocarbon receptor, such as SR1.

In some examples, the method includes contacting the population of hematopoietic stem cells with a compound that inhibits BMP signaling.

Also described is a method of producing an expanded population of hematopoietic stem cells *ex vivo,* the method including contacting a population of hematopoietic stem cells with one or more compounds listed in Table 1 and one or more agents that inhibit the activity of one or more proteins selected from the group consisting of:
a. a histone demethylase;
b. a protein that propagates TGFβ signaling;
c. a protein that propagates p38 signaling;
d. a protein that promotes β-catenin degradation;
e. a histone deacetylase; and
f. aryl hydrocarbon receptor,
wherein the one or more compounds and one or more agents are present in amounts that are sufficient to produce an expanded population of hematopoietic stem cells.

Further described is a method of enriching a population of cells with hematopoietic stem cells *ex vivo,* the method including contacting a population of hematopoietic cells that contains one or more hematopoietic stem cells with one or more compounds listed in Table 1 and one or more agents that inhibit the activity of one or more proteins selected from the group consisting of:
a. a histone demethylase;
b. a protein that propagates TGFβ signaling;
c. a protein that propagates p38 signaling;
d. a protein that promotes β-catenin degradation;
e. a histone deacetylase; and
f. aryl hydrocarbon receptor,
wherein the one or more compounds and one or more agents are present in amounts that are sufficient to produce a population of cells enriched with hematopoietic stem cells.

Also described is a method of maintaining the hematopoietic stem cell functional potential of a population of hematopoietic stem cells *ex vivo* for at least two days, the method including contacting a first population of hematopoietic stem cells with one or more compounds listed in Table 1 and one or more agents that inhibit the activity of one or more proteins selected from the group consisting of:
a. a histone demethylase;
b. a protein that propagates TGFβ signaling;
c. a protein that propagates p38 signaling;
d. a protein that promotes β-catenin degradation;
e. a histone deacetylase; and
f. aryl hydrocarbon receptor,
wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the one or more compounds and one or more agents.

Further described is a method of producing an expanded population of hematopoietic stem cells *ex vivo,* the method including contacting a population of hematopoietic stem cells with one or more agents that inhibit aryl hydrocarbon receptor and one or more agents that inhibit the activity of one or more proteins selected from the group consisting of:
a. modulation of histone methylation;
b. inhibition of TGFβ signaling;
c. inhibition of p38 signaling;
d. activation of canonical Wnt signaling; and
e. modulation of histone acetylation,
wherein the one or more agents are present in amounts that are sufficient to produce an expanded population of hematopoietic stem cells.

Further described is a method of enriching a population of cells with hematopoietic stem cells *ex vivo,* the method including contacting a population of hematopoietic cells that contains one or more hematopoietic stem cells with one or more agents that inhibit aryl hydrocarbon receptor and one or more agents that inhibit the activity of one or more proteins selected from the group consisting of:
a. modulation of histone methylation;
b. inhibition of TGFβ signaling;
c. inhibition of p38 signaling;
d. activation of canonical Wnt signaling; and
e. modulation of histone acetylation,
wherein the one or more agents are present in amounts that are sufficient to produce a population of cells enriched with hematopoietic stem cells.

Also described is a method of maintaining the hematopoietic stem cell functional potential of a population of hematopoietic stem cells *ex vivo* for at least two days, the method including contacting a first population of hematopoietic stem cells with one or more agents that inhibit aryl hydrocarbon receptor and one or more agents that inhibit the activity of one or more proteins selected from the group consisting of:
a. modulation of histone methylation;
b. inhibition of TGFβ signaling;
c. inhibition of p38 signaling;
d. activation of canonical Wnt signaling; and
e. modulation of histone acetylation,
wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the one or more agents.

Further described is a method of producing an expanded population of hematopoietic stem cells *ex vivo,* the method including contacting a population of hematopoietic stem cells with a compound represented by formula (**1**).

Also described is a method of enriching a population of cells with hematopoietic stem cells *ex vivo,* the method including contacting a population of hematopoietic cells that contains one or more hematopoietic stem cells with a compound represented by formula (**1**).

Further described is a method of maintaining the hematopoietic stem cell functional potential of a population of hematopoietic stem cells *ex vivo* for at least two days, the method including contacting a first population of hematopoietic stem cells with a compound represented by formula (**1**), wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the first and second agents.

In some examples, the compound represented by formula (**1**) is selected from the group consisting of pomalidomide, lenalidomide, and thalidomide.

In some examples, the method further includes contacting the population of hematopoietic stem cells with one or more compounds selected from UM171, structural analogs thereof, and the compounds listed in Table 1.

In some examples, the method further including contacting the population of hematopoietic stem cells with a TGFβ receptor inhibitor selected from the group consisting of ALK5 inhibitor II, LY364947, DMH1, and A83-01.

In some examples, the method includes contacting the population of hematopoietic stem cells with a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine.

In some examples, the method includes contacting the population of hematopoietic stem cells with a histone deacetylase inhibitor selected from the group consisting of Trichostatin A, valproic acid, butyrylhydroxamic acid, and istodax.

In some examples, the method includes contacting the population of hematopoietic stem cells with one or more compounds selected from the group consisting of:
a. a compound that inhibits a protein that propagates p38 signaling including SB203580; and
b. a compound that inhibits a protein that promotes β-catenin degradation selected from the group consisting of CHIR99021, lithium chloride, BIO, and FGF2.

In some examples, the method includes contacting the population of hematopoietic stem cells with SR1.

Also described is a method of producing an expanded population of hematopoietic stem cells *ex vivo,* the method including contacting a population of hematopoietic stem cells with one or more compounds selected from UM171, structural analogs thereof, and the compounds listed in Table 1; and one or more agents selected from the group consisting of:
a. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
b. a TGFβ receptor inhibitor selected from the group consisting of ALK5 inhibitor II, LY364947, DMH1, and A83-01;
c. a compound that inhibits a protein that propagates p38 signaling comprising SB203580;
d. a compound that inhibits a protein that promotes β-catenin degradation selected from the group consisting of CHIR99021, lithium chloride, BIO, and FGF2;
e. a histone deacetylase inhibitor selected from the group consisting of Trichostatin A, valproic acid, butyrylhydroxamic acid, and istodax; and
f. an aryl hydrocarbon receptor inhibitor comprising SR1,
wherein the one or more compounds selected from UM171, structural analogs thereof, and the compounds listed in Table 1 and the one or more agents are present in amounts that are sufficient to produce an expanded population of hematopoietic stem cells.

Further described is a method of enriching a population of cells with hematopoietic stem cells *ex vivo,* the method including contacting a population of hematopoietic cells that contains one or more hematopoietic stem cells with UM171 and one or more agents selected from the group consisting of::
a. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
b. a TGFβ receptor inhibitor selected from the group consisting of ALK5 inhibitor II, LY364947, DMH1, and A83-01;
c. a compound that inhibits a protein that propagates p38 signaling comprising SB203580;
d. a compound that inhibits a protein that promotes β-catenin degradation selected from the group consisting of CHIR99021, lithium chloride, BIO, and FGF2;
e. a histone deacetylase inhibitor selected from the group consisting of Trichostatin A, valproic acid, butyrylhydroxamic acid, and istodax; and
f. an aryl hydrocarbon receptor inhibitor comprising SR1,
wherein the UM171 and the one or more agents are present in amounts that are sufficient to produce a population of cells enriched with hematopoietic stem cells.

Further described is a method of maintaining the hematopoietic stem cell functional potential of a population of hematopoietic stem cells *ex vivo* for at least two days, the method including contacting a first population of hematopoietic stem cells with one or more compounds selected from UM171, structural analogs thereof, and the compounds listed in Table 1 and one or more agents selected from the group consisting of:
a. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
b. a TGFβ receptor inhibitor selected from the group consisting of ALK5 inhibitor II, LY364947, DMH1, and A83-01;
c. a compound that inhibits a protein that propagates p38 signaling comprising SB203580;
d. a compound that inhibits a protein that promotes β-catenin degradation selected from the group consisting of CHIR99021, lithium chloride, BIO, and FGF2;
e. a histone deacetylase inhibitor selected from the group consisting of Trichostatin A, valproic acid, butyrylhydroxamic acid, and istodax; and
f. an aryl hydrocarbon receptor inhibitor comprising SR1,
wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the UM171 and the one or more agents.

Also described is a method of producing an expanded population of hematopoietic stem cells *ex vivo,* the method including contacting a population of hematopoietic stem cells with SR1 and one or more agents selected from the group consisting of:
a. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
b. a TGFβ receptor inhibitor selected from the group consisting of ALK5 inhibitor II, LY364947, DMH1, and A83-01;
c. a compound that inhibits a protein that propagates p38 signaling comprising SB203580;
d. a compound that inhibits a protein that promotes β-catenin degradation selected from the group consisting of CHIR99021, lithium chloride, BIO, and FGF2; and
e. a histone deacetylase inhibitor selected from the group consisting of Trichostatin A, valproic acid, butyrylhydroxamic acid, and istodax,
wherein the SR1 and the one or more agents are present in amounts that are sufficient to produce an expanded population of hematopoietic stem cells.

Further described is a method of enriching a population of cells with hematopoietic stem cells *ex vivo,* the method including contacting a population of hematopoietic cells that contains one or more hematopoietic stem cells with SR1 and one or more agents selected from the group consisting of:
a. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
b. a TGFβ receptor inhibitor selected from the group consisting of ALK5 inhibitor II, LY364947, DMH1, and A83-01;
c. a compound that inhibits a protein that propagates p38 signaling comprising SB203580;
d. a compound that inhibits a protein that promotes β-catenin degradation selected from the group consisting of CHIR99021, lithium chloride, BIO, and FGF2; and
e. a histone deacetylase inhibitor selected from the group consisting of Trichostatin A, valproic acid, butyrylhydroxamic acid, and istodax,
wherein the SR1 and the one or more agents are present in amounts that are sufficient to produce a population of cells enriched with hematopoietic stem cells.

Also described is a method of maintaining the hematopoietic stem cell functional potential of a population of hematopoietic stem cells *ex vivo* for at least two days, the method including contacting a first population of hematopoietic stem cells with SR1 and one or more agents selected from the group consisting of:
a. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
b. a TGFβ receptor inhibitor selected from the group consisting of ALK5 inhibitor II, LY364947, DMH1, and A83-01;
c. a compound that inhibits a protein that propagates p38 signaling comprising SB203580;
d. a compound that inhibits a protein that promotes β-catenin degradation selected from the group consisting of CHIR99021, lithium chloride, BIO, and FGF2; and
e. a histone deacetylase inhibitor selected from the group consisting of Trichostatin A, valproic acid, butyrylhydroxamic acid, and istodax,
wherein the first population of hematopoietic stem cells exhibits a hematopoietic stem cell functional potential after two or more days that is greater than that of a control population of hematopoietic stem cells cultured under the same conditions and for the same time as the first population of hematopoietic stem cells but not contacted with the SR1 and the one or more agents.

In some examples, the one or more agents or compounds are present in amounts that are sufficient to stimulate expansion of the population of cells by 10% or more relative to a population of hematopoietic stem cells not contacted with the one or more agents or compounds after seven or more days of culture (e.g., after seven, ten, twelve, fourteen, fifteen, twenty, or more days of culture).

In some examples, the one or more agents or compounds are present in amounts that are sufficient to stimulate expansion of the population of cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture (e.g., after seven, ten, twelve, fourteen, fifteen, twenty, or more days of culture).

In some examples, the one or more agents or compounds are present in amounts that are sufficient to enrich the population of cells with hematopoietic stem cells by 10% or more relative to a population of hematopoietic stem cells not contacted with the one or more agents or compounds after seven or more days of culture (e.g., after seven, ten, twelve, fourteen, fifteen, twenty, or more days of culture).

In some examples, the one or more agents or compounds are present in amounts that are sufficient to enrich the population of cells with hematopoietic stem cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture (e.g., after seven, ten, twelve, fourteen, fifteen, twenty, or more days of culture).

In some examples, the first population of hematopoietic stem cells exhibits a hematopoietic stem cell functional potential after three or more days of culture (e.g., three, five, seven, ten, twelve, fourteen, fifteen, twenty, or more days) that is greater than that of the control population of hematopoietic stem cells.

In some examples, the hematopoietic stem cells are mammalian cells, such as human cells. In some examples, the hematopoietic stem cells are CD34+ cells. In some examples, at least 10% of the CD34+ cells are CD34+, CD34+CD38-, CD34+CD38-CD90+, CD34+CD38-CD90+CD45RA-, or CD34+CD38-CD90+CD45RA-CD49F+ cells. In some examples, the hematopoietic stem cells are from human cord blood. In some examples, the hematopoietic stem cells are from human mobilized peripheral blood. In some examples, the hematopoietic stem cells are from human bone marrow. In some examples, the hematopoietic stem cells are freshly isolated from the human. In some examples, the hematopoietic stem cells have been previously cryopreserved. In some examples, the mammalian cells are murine cells.

In some examples, the hematopoietic stem cells are cultured for two or more days (e.g., three, five, seven, ten, twelve, fourteen, fifteen, twenty, or more days). In some examples, the hematopoietic stem cells contact the one or more agents or compounds for two or more days (e.g., three, five, seven, ten, twelve, fourteen, fifteen, twenty, or more days). In some examples, the hematopoietic stem cells are contacted with the one or more agents or compounds simultaneously. In some examples, the hematopoietic stem cells are contacted with the one or more agents or compounds at different times.

In some examples, the hematopoietic stem cells maintain hematopoietic stem cell functional potential after two days (e.g., three, five, seven, ten, twelve, fourteen, fifteen, twenty, or more days) in culture. In some examples, the hematopoietic stem cells maintain hematopoietic stem cell functional potential following transplantation after two days (e.g., three, five, seven, ten, twelve, fourteen, fifteen, twenty, or more days) in culture.

In some examples, the hematopoietic stem cells maintain long term engraftment potential after two days (e.g., three, five, seven, ten, twelve, fourteen, fifteen, twenty, or more days) in culture.

In some examples, upon transplantation into a patient, the hematopoietic stem cells give rise to recovery of a population of cells selected from the group consisting of neutrophils, platelets, red blood cells, monocytes, macrophages, antigen-presenting cells, microglia, osteoclasts, dendritic cells, and lymphocytes (such as natural killer cells, T cells (e.g., CD4+ or CD8+ cells), and B cells).

In some examples, the hematopoietic stem cells are capable of localizing to hematopoietic tissue to reestablish productive hematopoiesis in a transplanted recipient.

In some examples, the hematopoietic stem cells are cultured on a plastic surface or on a substrate that includes vitronectin, fibronectin, or matrigel.

In some examples, the hematopoietic stem cells are cultured in the presence of 2-20% oxygen. In some examples, the hematopoietic stem cells are cultured in the presence of 2-12% oxygen. In some examples, the hematopoietic stem cells are cultured in the presence of about 5% oxygen.

In some examples, the hematopoietic stem cells are originally within a mononuclear cell fraction prior to treatment with the one or more agents or compounds. In some examples, the hematopoietic stem cells are originally within a CD34+, CD34+CD38-, CD34+CD38-CD90+, CD34+CD38-CD90+CD45RA-, or CD34+CD38-CD90+CD45RA-CD49F+, or CD34+CD38-CD90+CD45RA-CD49F+EPCR+ enriched cell fraction prior to contacting the one or more agents or compounds. In some examples, the hematopoietic stem cells are originally within an un-enriched cell fraction prior to contacting the one or more agents or compounds.

Further described is a method of introducing a polynucleotide into a population of hematopoietic stem cells, the method including:
a. inserting the polynucleotide into the population of hematopoietic stem cells; and
b. expanding the population of hematopoietic stem cells according to the method of any one of the above examples, or maintaining the hematopoietic stem cell functional potential of the population of hematopoietic stem cells according to the method of any one of the above examples.

In some examples, step (a) above precedes step (b). In some examples, step (b) precedes step (a).

In some examples, the method includes providing one or more reagents that cleave a nucleic acid in the cells, such as a zinc finger nuclease, a transcription activator-like nuclease, a CRISPR-associated protein, or a meganuclease.

In some examples, the method includes contacting the hematopoietic stem cells with a vector selected from the group consisting of a viral vector (such as retrovirus, adenovirus, parvovirus, coronavirus, rhabdovirus, paramyxovirus, picornavirus, alphavirus, herpes virus, or poxvirus) and a transposable element (such as a piggybac transposon or sleeping beauty transposon)

In some examples, the method includes introducing the polynucleotide into the hematopoietic stem cells by electroporation, Nucleofection^{™}, or squeeze-poration.

In some examples, the method includes contacting the cells with a transformation agent selected from the group consisting of a cationic polymer (e.g., diethylaminoethyl-dextran), a cationic lipid, calcium phosphate, an activated dendrimer, and a magnetic bead.

In some examples, the method includes introducing the polynucleotide into the hematopoietic stem cells by microinjection or laserfection.

In some examples, the polynucleotide includes a regulatory sequence selected from the group consisting of a promoter, enhancer, or silencer sequence.

In some examples, the polynucleotide encodes a molecule selected from the group consisting of a protein and a RNA (mRNA, tRNA, siRNA, miRNA, shRNA). In some examples, the polynucleotide is a chemically modified RNA.

In some examples, the method includes introducing the population of expanded hematopoietic stem cells or progeny thereof into a recipient.

Also described is a method of treating a recipient with hematopoietic stem cells or progeny thereof, the method including:
a. providing a population of hematopoietic stem cells;
b. expanding the population of hematopoietic stem cells according to the method of any one of the above examples;
c. optionally differentiating the hematopoietic stem cells into common lymphoid progenitor cells, common myeloid progenitor cells, megakaryocyte-erythroid progenitor cells, granulocyte-megakaryocyte progenitor cells, granulocytes, promyelocytes, neutrophils, eosinophils, basophils, erythrocytes, reticulocytes, thrombocytes, megakaryoblasts, platelet-producing megakaryocytes, platelets, monocytes, macrophages, dendritic cells, microglia, osteoclasts, and lymphocytes, NK cells, B-cells and/or T-cells; and
d. introducing the population of expanded hematopoietic stem cells or progeny thereof into the recipient.

Further described is a method of treating a recipient with hematopoietic stem cells or progeny thereof, the method including:
a. providing a population of hematopoietic stem cells;
b. enriching the population of hematopoietic stem cells according to the method of any one of the above examples;
c. optionally differentiating the hematopoietic stem cells into common lymphoid progenitor cells, common myeloid progenitor cells, megakaryocyte-erythroid progenitor cells, granulocyte-megakaryocyte progenitor cells, granulocytes, promyelocytes, neutrophils, eosinophils, basophils, erythrocytes, reticulocytes, thrombocytes, megakaryoblasts, platelet-producing megakaryocytes, platelets, monocytes, macrophages, dendritic cells, microglia, osteoclasts, and lymphocytes, NK cells, B-cells and/or T-cells; and
d. introducing the population of cells enriched with hematopoietic stem cells or progeny thereof into the recipient.

Also described is a method of treating a recipient with hematopoietic stem cells or progeny thereof, the method including:
a. providing a population of hematopoietic stem cells;
b. maintaining the hematopoietic stem cell functional potential of the population of hematopoietic stem cells according to the method of any one of the above examples;
c. optionally differentiating the hematopoietic stem cells into common lymphoid progenitor cells, common myeloid progenitor cells, megakaryocyte-erythroid progenitor cells, granulocyte-megakaryocyte progenitor cells, granulocytes, promyelocytes, neutrophils, eosinophils, basophils, erythrocytes, reticulocytes, thrombocytes, megakaryoblasts, platelet-producing megakaryocytes, platelets, monocytes, macrophages, dendritic cells, microglia, osteoclasts, and lymphocytes, NK cells, B-cells and/or T-cells; and
d. introducing the population of hematopoietic stem cells or progeny thereof into the recipient.

Also described is a method of treating a recipient with hematopoietic stem cells or progeny thereof, the method including:
a. providing a population of hematopoietic stem cells produced by the method of any one of the above examples;
b. optionally differentiating the hematopoietic stem cells into common lymphoid progenitor cells, common myeloid progenitor cells, megakaryocyte-erythroid progenitor cells, granulocyte-megakaryocyte progenitor cells, granulocytes, promyelocytes, neutrophils, eosinophils, basophils, erythrocytes, reticulocytes, thrombocytes, megakaryoblasts, platelet-producing megakaryocytes, platelets, monocytes, macrophages, dendritic cells, microglia, osteoclasts, and lymphocytes, NK cells, B-cells and/or T-cells; and
c. introducing the population of hematopoietic stem cells or progeny thereof into the recipient.

In some examples, the recipient is a human.

In some examples, the hematopoietic stem cells are derived from one or more hematopoietic stem cells isolated from a human donor. In some examples, the hematopoietic stem cells are from mobilized peripheral blood of the donor.

In some examples, the donor has been previously administered one or more mobilizing agents selected from the group consisting of a CXCR4 antagonist (e.g., AMD3100), GCSF, and GROβ.

In some examples, the hematopoietic stem cells are additionally contacted with a prostaglandin, such as dmPGE2 or an analog thereof.

In some examples, the hematopoietic stem cells are additionally contacted with an agonist of Notch signaling.

In some examples, the hematopoietic stem cells are additionally contacted with an inhibitor of SIRT1. In some examples, the inhibitor or SIRT1 is selected from the group consisting of nicotinamide, cambinol, and analogs thereof.

In some examples, the recipient is a human patient suffering from a disease selected from the group consisting of Acute Lymphoblastic Leukemia (ALL), Acute Myelogenous Leukemia (AML), Chronic Myelogenous Leukemia (CML), Chronic Lymphocytic Leukemia (CLL), Hodgkin Lymphoma (HL), Non-Hodgkin Lymphoma (NHL), Myelodysplastic Syndrome (MDS), Multiple myeloma, Aplastic anemia, Bone marrow failure, Myeloproliferative disorders such as Myelofibrosis, Essential thrombocytopenia or Polycythemia vera, Fanconi anemia, Dyskeratosis congenita, Common variable immune deficiency (CVID, such as CVID 1, CVID 2, CVID 3, CVID 4, CVID 5, and CVID 6), Hemophagocytic lymphohistiocystosis, Solid tumors such as Neuroblastoma, Germ cell tumors, Breast cancer, Wilms' tumor, Medulloblastoma, and Neuroectodermal tumors, Autoimmune diseases such as Scleroderma, Multiple sclerosis, Ulcerative colitis, Systemic lupus erythematosus or Type I diabetes, Human immunodeficiency virus (HIV), or protein deficiencies such as Adrenoleukodystrophy (ALD), Metachromatic leukodystrophy (MLD), Hemophilia A & B, Hurler syndrome, Hunter syndrome, Fabry disease, Gaucher disease, Epidermolysis bullosa, Amyloidosis, Globoid Cell Leukodystrophy, Sanfillipo syndrome, and Morquio syndrome.

In some examples, the recipient is a human patient suffering from a disease selected from the group consisting of Sickle cell anemia, Alpha thalassemia, Beta thalassemia, Delta thalassemia, Hemoglobin Ethalassemia, Hemoglobin S/thalassemia, Hemoglobin C/thalassemia, Hemoglobin D/thalassemia, Chronic granulomatous disease (X-linked Chronic granulomatous disease, autosomal recessive (AR) chronic granulomatous disease, chronic granulomatous disease AR I NCF1,, Chronic granulomatous disease AR CYBA, Chronic granulomatous disease AR II NCF2, Chronic granulomatous disease AR III NCF4), X-linked Severe Combined Immune Deficiency (SCID), ADA SCID, IL7-RA SCID, CD3 SCID, Rag1/Rag2 SCID, Artemis SCID, CD45 SCID, Jak3 SCID, Congenital agranulocytosis,, Congenital agranulocytosis-congenital neutropenia- SCN1, Congenital agranulocytosis-congenital neutropenia-SCN2, Familial hemophagocytic lymphohistiocystosis (FHL), Familial hemophagocytic lymphohistiocytosis type 2 (FHL2, perforin mutation), Agammaglobulinemia (X-linked Agammaglobulinemia), Wiskott-Aldrich syndrome, Chediak-Higashi syndrome, Hemolytic anemia due to red cell pyruvate kinase deficiency, Paroxysmal nocturnal hemoglobinuria, X-linked Adrenoleukodystrophy (X-ALD), X-linked lymphoproliferative disease, Unicentric Castleman's Disease, Multicentric Castleman's Disease, Congenital amegakaryocytic thrombocytopenia (CAMT) type I, Reticular dysgenesis, Fanconi anemia, Acquired idiopathic sideroblastic anemia, Systemic mastocytosis, Von willebrand disease (VWD), Congenital dyserythropoietic anemia type 2, Cartilage-hair hypoplasia syndrome, Hereditary spherocytosis, Blackfan-Diamond syndrome, Shwachman-Diamond syndrome, Thrombocytopenia-absent radius syndrome, Osteopetrosis, Infantile osteopetrosis, Mucopolysaccharidoses, Lesch-Nyhan syndrome, Glycogen storage disease, Congenital mastocytosis, Omenn syndrome, X-linked Immunodysregulation, polyendocrinopathy, and enteropathy (IPEX), IPEX characterized by mutations in FOXP3, *X-linked* syndrome of polyendocrinopathy, immune dysfunction, and diarrhea (XPID), X-Linked Autoimmunity-Allergic Dysregulation Syndrome (*XLAAD*), IPEX-like syndrome, Hyper IgM type 1, Hyper IgM type 2, Hyper IgM type 3, Hyper IgM type 4, Hyper IgM type 5, X linked hyperimmunoglobulin M, Bare lymphocyte Syndrome type I, and Bare lymphocyte Syndrome type II (Bare lymphocyte Syndrome type II, MHC class I deficiency; Bare lymphocyte Syndrome type II, complementation group A; Bare lymphocyte Syndrome type II, complementation group C; Bare lymphocyte Syndrome type II complementation group D; Bare lymphocyte Syndrome type II, complementation group E).

In some examples, the recipient is a human patient suffering from a hematolymphoid malignancy, a non-hematolymphoid malignancy, or a protein deficiency, or a tissue or cell transplantation recipient (e.g., to induce tolerance to transplanted tissue or cells).

In some examples, the hematopoietic stem cells are autologous or syngeneic. In some examples, the hematopoietic stem cells are allogeneic.

Further described is a composition including:
a. one or more agents that reduce the level of an ikaros family member transcription factor in a cell; and one or more of
b. one or more agents that inhibit TGFβ signaling;
c. one or more compounds listed in Table 1;
d. one or more agents that modulate histone methylation;
e. one or more agents that modulate histone acetylation; and
f. one or more agents that inhibit aryl hydrocarbon receptor signaling.

In some examples, the ikaros family member transcription factor is selected from the group consisting of ikaros, aiolos, and helios.

In some examples, the one or more agents that reduce the level of an ikaros family member transcription factor in a cell promote ubiquitination of the ikaros family member transcription factor. In some examples, the one or more agents that reduce the level of an ikaros family member transcription factor in a cell include a compound represented by formula (**1**). In some examples, the compound represented by formula (**1**) is selected from the group consisting of pomalidomide, lenalidomide, and thalidomide.

In some examples, the one or more compounds listed in Table 1 include UM171.

In some examples, the one or more agents that inhibit TGFβ signaling include a TGFβ receptor inhibitor. In some examples, the TGFβ receptor inhibitor is selected from the group consisting of ALK5 inhibitor II, LY364947, DMH1, and A83-01. In some examples, the TGFβ receptor inhibitor is A83-01.

In some examples, the one or more agents that modulate histone methylation activate histone methylation, maintain histone methylation, or inhibit histone demethylation. In some examples, the one or more agents that modulate histone methylation include a histone demethylase inhibitor. In some examples, the histone demethylase inhibitor is a LSD1 inhibitor. In some examples, the LSD1 inhibitor is selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine. In some examples, the LSD1 inhibitor is Tranylcypromine.

In some examples, the one or more agents that modulate histone acetylation activate histone acetylation, maintain histone acetylation, or inhibit histone deacetylation. In some examples, the one or more agents that modulate histone acetylation include a histone deacetylase inhibitor. In some examples, the histone deacetylase inhibitor is selected from the group consisting of Trichostatin A, valproic acid, butyrylhydroxamic acid, and istodax. In some examples, the histone deacetylase inhibitor is Trichostatin A.

In some examples, the one or more agents that inhibit aryl hydrocarbon receptor signaling include SR1.

Also described is a composition including:
a. one or more compounds listed in Table 1; and one or more of
b. one or more agents that inhibit TGFβ signaling;
c. one or more agents that modulate histone methylation;
d. one or more agents that modulate histone acetylation; and
e. one or more agents that inhibit aryl hydrocarbon receptor signaling.

Further described is a composition including:
a. one or more agents that inhibit aryl hydrocarbon receptor signaling; and one or more of
b. one or more agents that inhibit TGFβ signaling;
c. one or more agents that modulate histone methylation; and
d. one or more agents that modulate histone acetylation.

Further described is a composition including:
a. one or more agents that activate a ubiquitin ligase complex including cereblon; and one or more of
b. a TGFβ receptor inhibitor;
c. a compound listed in Table 1;
d. a histone demethylase inhibitor;
e. a histone deacetylase inhibitor; and
f. an aryl hydrocarbon receptor inhibitor.

In some examples, the one or more agents that activate a ubiquitin ligase complex including cereblon are represented by formula (**1**). In some examples, the one or more agents that activate a ubiquitin ligase complex including cereblon are selected from the group consisting of pomalidomide, lenalidomide, and thalidomide.

In some examples, the compound listed in Table 1 is UM171.

In some examples, the TGFβ receptor inhibitor is selected from the group consisting of ALK5 inhibitor II, LY364947, DMH1, and A83-01.

In some examples, the histone demethylase inhibitor is selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine.

In some examples, the histone deacetylase inhibitor is selected from the group consisting of Trichostatin A, valproic acid, butyrylhydroxamic acid, and istodax.

In some examples, the composition includes one or more agents selected from the group consisting of:
a. a compound that inhibits a protein that propagates p38 signaling including SB203580; and
b. a compound that inhibits a protein that promotes β-catenin degradation selected from the group consisting of CHIR99021, lithium chloride, BIO, and FGF2.

In some examples, the aryl hydrocarbon receptor inhibitor is SR1.

In some examples, the composition includes a compound that inhibits BMP signaling.

Also described is a composition including:
a. a compound listed in Table 1; and one or more of
b. a TGFβ receptor inhibitor;
c. a histone demethylase inhibitor;
d. a histone deacetylase inhibitor; and
e. an aryl hydrocarbon receptor inhibitor.

Further described is a composition including:
a. an aryl hydrocarbon receptor inhibitor; and one or more of
b. a TGFβ receptor inhibitor;
c. a compound listed in Table 1;
d. a histone demethylase inhibitor; and
e. a histone deacetylase inhibitor.

Also described is a composition including:
a. a compound represented by formula (1); and one or more of
b. a TGFβ receptor inhibitor selected from the group consisting of ALK5 inhibitor II, LY364947, DMH1, and A83-01;
c. UM171, a structural analog thereof, or a compound listed in Table 1;
d. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
e. a histone deacetylase inhibitor selected from the group consisting of Trichostatin A, valproic acid, butyrylhydroxamic acid, and istodax; and
f. SR1.

In some examples, the compound represented by formula (**1**) is selected from the group consisting of pomalidomide, lenalidomide, and thalidomide.

In some examples, the composition includes one or more agents selected from the group consisting of:
a. a compound that inhibits a protein that propagates p38 signaling including SB203580; and
b. a compound that inhibits a protein that promotes β-catenin degradation selected from the group consisting of CHIR99021, lithium chloride, BIO, and FGF2.

Further described is a composition including:
a. UM171, a structural analog thereof, or a compound listed in Table 1; and one or more of
b. a TGFβ receptor inhibitor selected from the group consisting of ALK5 inhibitor II, LY364947, DMH1, and A83-01;
c. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine;
d. a histone deacetylase inhibitor selected from the group consisting of Trichostatin A, valproic acid, butyrylhydroxamic acid, and istodax; and
e. SR1.

Also described is a composition including
a. SR1; and one or more of
b. a TGFβ receptor inhibitor selected from the group consisting of ALK5 inhibitor II, LY364947, DMH1, and A83-01;
c. a histone demethylase inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine; and
d. a histone deacetylase inhibitor selected from the group consisting of Trichostatin A, valproic acid, butyrylhydroxamic acid, and istodax.

In some examples, the one or more agents or compounds are present in amounts that are sufficient to produce an expanded population of hematopoietic stem cells. In some examples, the one or more agents or compounds are present in amounts that are sufficient to produce a population of cells enriched with hematopoietic stem cells. In some examples, the one or more agents or compounds are present in amounts sufficient to maintain the hematopoietic stem cell functional potential of the population of hematopoietic stem cells for at least two days.

In some examples, the one or more agents or compounds are present in an aqueous solution. In some examples, the one or more agents or compounds are present as a lyophilized solid.

In some examples, the one or more agents or compounds are present in amounts that are sufficient to stimulate expansion of the population of cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture (e.g., after seven, ten, twelve, fourteen, fifteen, twenty, or more days of culture).

In some examples, the one or more agents or compounds are present in amounts that are sufficient to enrich the population of cells with hematopoietic stem cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture (e.g., after seven, ten, twelve, fourteen, fifteen, twenty, or more days of culture).

In some examples, the one or more agents or compounds are present in amounts that are sufficient to maintain long term engraftment potential of the hematopoietic stem cells post-transplantation after having contacted the cells in culture for two or more days (e.g., three, five, seven, ten, twelve, fourteen, fifteen, twenty, or more days).

Also described is a cell culture medium including the composition of any one of the above examples. In some examples, the cell culture medium is substantially free of serum.

In some examples, the composition further includes a population of hematopoietic stem cells in contact with the one or more agents or compounds.

In some examples, the hematopoietic stem cells have been cultured in the presence of the one or more agents or compounds for two or more days (e.g., three, five, seven, ten, twelve, fourteen, fifteen, twenty, or more days).

Further described is a method of producing an expanded population of hematopoietic stem cells *ex vivo,* the method including contacting a population of hematopoietic stem cells with (1) a first agent that reduces the level of an ikaros family member transcription factor in a cell; and (2) a second agent selected from the group consisting of SR1 or an analog thereof, a prostaglandin, an agonist of Notch signaling, or an inhibitor of SIRT1, wherein the first and second agents are present in amounts that together are sufficient to produce an expanded population of hematopoietic stem cells.

Also described is a method of enriching a population of cells with hematopoietic stem cells *ex vivo,* the method including contacting a population of hematopoietic cells that contains one or more hematopoietic stem cells with (1) a first agent that reduces the level of an ikaros family member transcription factor in a cell; and (2) a second agent selected from the group consisting of SR1 or an analog thereof, a prostaglandin, an agonist of Notch signaling, or an inhibitor of SIRT1, wherein the first and second agents are present in amounts that together are sufficient to produce a population of cells enriched with hematopoietic stem cells.

Further described is a method of maintaining the hematopoietic stem cell functional potential of a population of hematopoietic stem cells *ex vivo* for at least two days, the method including contacting a first population of hematopoietic stem cells with(1) a first agent that reduces the level of an ikaros family member transcription factor in a cell; and (2) a second agent selected from the group consisting of SR1 or an analog thereof, a prostaglandin, an agonist of Notch signaling, or an inhibitor of SIRT1, wherein the population of hematopoietic stem cells exhibits a hematopoietic stem cell functional potential after two or more days that is greater than that of a control population of hematopoietic stem cells cultured under the same conditions and for the same time as the population of hematopoietic stem cells but not contacted with the first and second agents.

Also described is a population of hematopoietic stem cells produced by the method of any one of the above examples.

Also described is a kit including the composition of any one of the above examples. The kit may include a package insert, such as a package insert instructing a user of the kit to expand, enrich, or maintain the hematopoietic stem cell functional potential of a population of hematopoietic stem cells *ex vivo.* In some examples, the package insert instructs the user to express a polynucleotide in the hematopoietic stem cells. In some examples, the package insert instructs the user to administer the hematopoietic stem cells or progeny thereof to a patient.

### Definitions

For convenience, the meaning of some terms and phrases used in the specification, examples, and appended claims, are provided below. Unless stated otherwise, or implicit from context, the following terms and phrases include the meanings provided below. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs. If there is an apparent discrepancy between the usage of a term in the art and its definition provided herein, the definition provided within the specification shall prevail.

As used herein, the term "expanded population" of hematopoietic stem cells or hematopoietic progenitor cells refers to a population of cells comprising at least one more hematopoietic stem cell, such that the quantity of hematopoietic stem cells in the population is greater (e.g., at least 10% greater, at least 20% greater, at least 30% greater) than the number of HSCs prior to administration of one or more agents as described herein (e.g., one or more agents that reduce the level of an ikaros family transcription factor, modulate TGFβ signaling, modulate lysine methylation, modulate p38 signaling, modulate canonical Wnt signaling, modulate histone methylation, or that modulate histone acetylation).

As used herein, an agent capable of reducing the level of an ikaros family transcription factor in a cell includes any compound or substance (e.g., a small molecule, protein, interfering RNA, messenger RNA, or other natural or synthetic compound, or a composition such as a virus or other material composed of multiple substances) capable of reducing the concentration of activity of an ikaros family transcription factor, such as ikaros, aiolos, and helios. Exemplary agents capable of reducing the level of an ikaros transcription factor include compounds that activate ubiquitin ligases, such as cereblon-containing ubiquitin E3 ligases, that mediate the polyubiquitination of an ikaros family transcription factor. Polyubiquitination of ikaros family transcription factors leads to degradation of the transcription factor by proteolysis. Compounds capable of reducing the level of an ikaros family transcription factor include thalidomide, pomalidomide, and lenalidomide, which are described, e.g., in US 5,798,368; US 5,955,476; US 6,281,230; and US 6,335,349. According to the invention, the one or more agents that reduce the level of an ikaros family member transcription factor in a cell are selected from the group consisting of pomalidomide and lenalidomide.

As used herein, an agent that inhibits histone demethylation refers to a substance or composition (e.g., a small molecule, protein, interfering RNA, messenger RNA, or other natural or synthetic compound, or a composition such as a virus or other material composed of multiple substances) capable of attenuating or preventing the activity of a histone demethylase or other enzyme that catalyzes the formation of an intermediate that leads to histone demethylation. Inhibition can occur by direct interaction or via indirect means, such as by causing a reduction in the quantity of histone demethylase produced in a cell or by inhibition of the interaction between a histone demethylase and a methylated histone substrate. Histone demethylases include lysine-specific demethylases, such as LSD1 and LSD2, as well as other FAD-dependent histone demethylases. Histone demethylases also include dioxygenases that utilize divalent iron (Fe²⁺) to catalyze oxidative demethylation of histone residues, such as AlkB, and Jumonji C (JmjC) domain-containing histone demethylases, such as JHDM1, JHDM2, and members of the JMJD2 superfamily of histone demethylases. Other enzymes that convert methylated histone residues into reactive intermediates that subsequently undergo oxidative demethylation include monoamine oxidases. Histone demethylation inhibitors may directly bind a histone demethylase and compete with a methylated histone substrate for binding at the enzyme active site. Alternatively, an agent that inhibits histone demethylation may bind a histone demethylase at a location remote from the active site and disrupt or prevent the interaction between the enzyme and a methylated histone substrate, e.g., by inducing a conformational change in the enzyme, or disrupt or prevent the catalytic cycle, e.g., by inactivating or displacing enzymatic cofactors.

An agent that inhibits histone demethylation is capable of attenuating or preventing the formation of a demethylated histone residue with a half-maximal inhibitory concentration (IC₅₀) of 100 µM or below (e.g., between 1 nM and 100 µM) as determined from a histone demethylation assay known in the art or described herein. Exemplary assays that can be used to elucidate the biological activity of a histone demethylation inhibitor include, without limitation, cell-based growth inhibition assays and dissociation-enhanced lanthanide fluorescence assays as described in US 8,735,622, time-resolved fluorescence resonance energy transfer assays as described in WO 2014/151945, as well as mass spectrometry-based assays and coupled-enzyme formaldehyde dehydrogenase assays as described in WO 2010/043866, among others.

As used herein, an agent that inhibits the TGFβ signaling pathway refers to a substance or composition (e.g., a small molecule, protein, interfering RNA, messenger RNA, or other natural or synthetic compound, or a composition such as a virus or other material composed of multiple substances) that can attenuate or prevent the transcription of one or more genes that are transcribed due to the activity of a SMAD transcription co-activator protein. An agent that inhibits the TGFβ signaling pathway may disrupt the signal transduction cascade that leads to SMAD-induced gene transcription at one or more points within the pathway. For instance, a TGFβ signaling pathway inhibitor may disrupt or prevent TGFβ or a TGFβ superfamily ligand, such as Activin, Nodal, bone morphogenetic protein (BMP), growth and differentiation factor (GDF), or Mullerian inhibitory factor (MIF), from binding to its endogenous receptor, thus inhibiting the phosphorylation and activation of the receptor-associated SMAD proteins. A TGFβ signaling pathway inhibitor may function by preventing the translocation of one or more SMAD proteins to the nucleus, for example, by binding a SMAD protein and preventing or disrupting the interaction between the SMAD protein and the nucleoporins. A TGFβ signaling pathway inhibitor may stabilize the interaction between one or more SMAD proteins and SMAD Anchor for Receptor Activation (SARA), which sequesters SMAD proteins in the cytoplasm and prevents their translocation into the nucleus. Other examples of TGFβ signaling pathway inhibitors include substances, such as neurogenin, that bind SMAD proteins and sequester them from DNA-bound transcription factors, thus preventing transcription of a target gene. Alternative inhibitors of the TGFβ signaling pathway include substances that promote the ubiquitination of one or more SMAD proteins, thereby marking the protein for degradation by the proteasome and preventing target gene transcription. According to the invention, the one or more agents that inhibit TGFβ signaling comprise a TGFβ receptor inhibitor and the TGFβ receptor inhibitor is selected from the group consisting of ALK5 inhibitor II, LY364947, DMH1, and A83-01.

Exemplary assays that can be used to determine the inhibitory activity of a TGFβ signaling pathway inhibitor include, without limitation, electrophoretic mobility shift assays, antibody supershift assays, as well as TGFβ-inducible gene reporter assays, as described in WO 2006/012954, among others.

As used herein, an agent that inhibits the p38 signaling pathway refers to a substance or composition (e.g., a small molecule, protein, interfering RNA, messenger RNA, or other natural or synthetic compound, or a composition such as a virus or other material composed of multiple substances) that can attenuate or prevent the activity of the p38 mitogen activated protein kinases (MAPKs, e.g., p38α, p38β, p38γ, or p38δ) or any protein that is involved, either directly or indirectly, in activating one or more of these enzymes. An agent that inhibits the p38 signaling pathway may include a substance, such as a monoclonal antibody, that binds to a cytokine receptor, such as IL-1R, and prevents the receptor-mediated activation of p38 MAP kinases. Alternatively, a p38 signaling pathway inhibitor may bind a p38 protein directly and attenuate or prevent the phosphorylation of the p38 activation loop by a MAP kinase. An agent that inhibits the p38 signaling pathway may alternatively disrupt the formation of poly-ubiquitin chains at lysine residues of TNF receptor associated factor (TRAF), which serve as scaffolds for MAPK complexes. Other inhibitors of the p38 signaling pathway include those that promote the phosphorylation of MAP kinases at sites remote from the activation loop and prevent their association with p38, as well as those that acetylate MAP kinases within the activation loop and thus prevent phosphorylation and concomitant activation of the MAP kinase.

Exemplary assays that can be used to determine the inhibitory activity of an agent that inhibits the p38 signaling pathway include, without limitation, fluorescence anisotropy competitive binding assays, as well as time-resolved fluorescence resonance energy transfer assays, as described in WO 2006/012954, among others.

As used herein, an agent that inhibits histone deacetylation refers to a substance or composition (e.g., a small molecule, protein, interfering RNA, messenger RNA, or other natural or synthetic compound, or a composition such as a virus or other material composed of multiple substances) capable of attenuating or preventing the activity of histone deacetylase, more particularly its enzymatic activity either via direct interaction or via indirect means such as by causing a reduction in the quantity of a histone deacetylase produced in a cell or by inhibition of the interaction between a histone deacetylase and an acetylated histone substrate. Inhibiting histone deacetylase enzymatic activity means reducing the ability of a histone deacetylase to catalyze the removal of an acetyl group from a histone residue (e.g., a mono-, di-, or tri-methylated lysine residue; a monomethylated arginine residue, or a symmetric/asymmetric dimethylated arginine residue, within a histone protein). Preferably, such inhibition is specific, such that the an agent that inhibits histone deacetylation reduces the ability of a histone deacetylase to remove an acetyl group from a histone residue at a concentration that is lower than the concentration of the inhibitor that is required to produce another, unrelated biological effect.

As used herein, the terms "histone deacetylase" and "HDAC" refer to any one of a family of enzymes that catalyze the removal of acetyl groups from the ε-amino groups of lysine residues at the N-terminus of a histone. Unless otherwise indicated by context, the term "histone" is meant to refer to any histone protein, including HI, H2A, H2B, H3, H4, and H5, from any species. Human HDAC proteins or gene products, include, but are not limited to, HDAC-1, HDAC-2, HDAC-3, HDAC-4, HDAC-5, HDAC-6, HDAC-7, HDAC-8, HDAC-9, HDAC-10, and HDAC-11.

As used herein, an agent that inhibits a protein that promotes β-catenin degradation include those agents that inhibit β-catenin phosphorylation or ubiquitination. These agents may be any substance (e.g., a small molecule, protein, interfering RNA, messenger RNA, or other natural or synthetic compound, or a composition such as a virus or other material composed of multiple substances) that can reduce the rate or extent of β-catenin degradation, e.g., by attenuating the catalysis of phosphorylation of serine and/or threonine residues that would otherwise render β-catenin a substrate for ubiquitination and proteasome-mediated degradation (for example, at residues Ser33, Ser37 and/or at Thr41). By extending the half life of functional β-catenin, these agents promote a concomitant increase in the rate or extent of transcription of a gene that is transcribed due to the activity of the β-catenin transcription co-activator. Exemplary agents that inhibit β-catenin phosphorylation include agonists of the canonical β-catenin/Wnt signaling pathway, a signal transduction cascade that orchestrates the inhibition of glycogen synthase kinase 3 (GSK3) by providing substrates that compete with β-catenin for phosphorylation.

As used herein, a "Wnt signaling agonist" refers to an agonist of the canonical Wnt signaling pathway. Agonists of this pathway further include Wnt proteins or other compounds that bind directly to the Frizzled and LRP5/6 co-receptor proteins in a manner that promotes an increase in the concentration of β-catenin in the nucleus of a mammalian cell. Alternatively, a β-catenin/Wnt pathway agonist may function by inhibiting one or more secreted Frizzled-related proteins (SFRPs) or Wnt inhibitory protein (WIF), which bind and sequester Wnt proteins from the endogenous Wnt co-receptors.

Exemplary methods that can be used to determine the activity of a β-catenin/Wnt pathway agonist include, without limitation, monitoring the expression of a reporter gene under the control of a TCF/LEF family transcription factor, as well as TOPFlash luciferase reporter assays, as described in US 2014/0044763.

As used herein, a compound that inhibits aryl hydrocarbon receptor signaling include agents that inhibit the signal transduction cascade that is propagated by the binding of the aryl hydrocarbon receptor to an activating ligand thereof. The aryl hydrocarbon receptor is a cytosolic, ligand-inducible transcription factor that, upon binding to an agonistic ligand, translocates into the nucleus and promotes the transcription of target genes containing the distinct sequence motifs, such as the gene encoding cytochrome P450A1 enzyme that contains an upstream dioxin-responsive element. Examples of agents that inhibit aryl hydrocarbon receptor signaling include aryl hydrocarbon receptor inhibitors, which may include compounds, such as SR1, that bind the aryl hydrocarbon receptor directly and thus compete with aryl hydrocarbon receptor ligands for binding to the receptor. Additional examples of agents that inhibit aryl hydrocarbon receptor signaling include agents that interfere with the translocation of the active aryl hydrocarbon receptor to the nucleus, and agents that inhibit the interaction of the aryl hydrocarbon receptor with the DNA (e.g., the promoter regions containing XRE sites) of target genes.

As used herein, an agonist of Notch signaling is an agent that promotes activation of Notch pathway function. The term "Notch pathway function" as used herein refers to a function mediated by the Notch signal transduction pathway including, but not limited to, nuclear translocation of the intracellular domain of Notch, nuclear translocation of RBP-Jκ or its Drosophila homolog Suppressor of Hairless; activation of bHLH genes of the Enhancer of Split complex, e.g., Mastermind; activation of the HES-1 gene or the KBF2 (also referred to as CBF1) gene; inhibition of Drosophila neuroblast segregation; and binding of Notch to a Delta protein, a Jagged/Serrate protein, Fringe, Deltex or RBP-Jκ/Suppressor of Hairless, or homologs or analogs thereof. The Notch signal transduction cascade and the phenotypes effected by Notch signaling are described, e.g., in Kopan et al. Cell 137:216 (2009) and Jarriault et al. Molecular Cell Biology 18:7423 (1998). Examples of Notch agonists are described, e.g., in US 2014/0369973 and in US 7,399,633. Exemplary Notch agonists include, without limitation, Notch proteins, as well as analogs, derivatives, and fragments thereof; other proteins that propagate the Notch signaling pathway, as well as analogs, derivatives, and fragments thereof; activating antibodies that stimulate Notch receptor activity and antigen-binding fragments thereof that retain agonistic activity; nucleic acids encoding proteins that potentiate Notch signaling; as well as proteins, derivatives, and analogs thereof which bind to or otherwise interact with Notch proteins or other proteins in the Notch pathway such that Notch pathway activity is promoted. Such agonists include, but are not limited to, Notch proteins and derivatives thereof containing the Notch intracellular domain, Notch nucleic acids encoding the foregoing, and proteins contacting the Notch-interacting domain of Notch ligands (e.g., the extracellular domain of Delta or Serrate). Other agonists include but are not limited to RBPJκ/Suppressor of Hairless or Deltex. Fringe can additionally be used to enhance Notch activity, for example in conjunction with Delta protein. These proteins, fragments, and derivatives thereof can be recombinantly expressed and isolated or can be chemically synthesized using peptide and protein synthesis techniques known in the art.

As used herein, the term "inhibitor" refers to any compound, natural or synthetic, which can reduce the activity of a target protein or signaling pathway. An inhibitor can be, for example, a peptide, a protein, an antibody, a peptidomimetic, an amino acid, an amino acid analog, a polynucleotide, a polynucleotide analog, an aptamer, a nucleotide, a nucleotide analog, an organic or inorganic compound. An inhibitor may attenuate or prevent the activity of a target protein either directly or indirectly. Direct inhibition can be obtained, for instance, by binding to a protein and preventing the protein from interacting with an endogenous molecule, such as an enzyme, a substrate, or other binding partner, thereby diminishing the activity of the protein. For instance, an inhibitor may bind an enzyme active site and sterically preclude binding of an endogenous substrate at this location, thus decreasing the enzymatic activity of the protein. Alternatively, indirect inhibition can be obtained, for instance, by binding to a protein that promotes the activity of a target protein by inducing a conformational change or catalyzing a chemical modification of the target protein. For instance, indirect inhibition of a target protein may be achieved by binding and inactivating a kinase that catalyzes the phosphorylation of, and thus activates, the target protein.

As used herein, the term "hematopoietic stem cells" (or "HSCs") refer to immature blood cells having the capacity to self-renew and to differentiate into mature blood cells comprising diverse lineages including but not limited to granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells). It is known in the art that such cells may or may not include CD34⁺ cells. CD34⁺ cells are immature cells that express the CD34 cell surface marker. In humans, CD34+ cells are believed to include a subpopulation of cells with the stem cell properties defined above, whereas in mice, HSCs are CD34-. In addition, HSCs also refer to long term repopulating HSC (LT-HSC) and short term repopulating HSC (ST-HSC). LT-HSC and ST-HSC are differentiated, based on functional potential and on cell surface marker expression. For example, human HSC are a CD34+, CD38-, CD45RA-, CD90+, CD49F+, and lin- (negative for mature lineage markers including CD2, CD3, CD4, CD7, CD8, CD10, CD11B, CD19, CD20, CD56, CD235A). In mice, bone marrow LT-HSC are CD34-, SCA-1+, C-kit+, CD135-, Slamfl/CD150+, CD48-, and lin- (negative for mature lineage markers including Ter119, CD11b, Gr1, CD3, CD4, CD8, B220, IL7ra), whereas ST-HSC are CD34+, SCA-1+, C-kit+, CD135-, Slamfl/CD150+, and lin- (negative for mature lineage markers including Ter119, CD11b, Gr1, CD3, CD4, CD8, B220, IL7ra). In addition, ST-HSC are less quiescent (i.e., more active) and more proliferative than LT-HSC under homeostatic conditions. However, LT-HSC have greater self renewal potential (i.e., they survive throughout adulthood, and can be serially transplanted through successive recipients), whereas ST-HSC have limited self renewal (i.e., they survive for only a limited period of time, and do not possess serial transplantation potential). Any of these HSCs can be used in any of the methods described herein. Optionally, ST-HSCs are useful because they are highly proliferative and thus, can more quickly give rise to differentiated progeny.

As used herein, the phrase "hematopoietic stem cell functional potential" refers to the functional properties of hematopoietic stem cells which include 1) multi-potency (which refers to the ability to differentiate into multiple different blood lineages including, but not limited to, granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells), 2) self-renewal (which refers to the ability of hematopoietic stem cells to give rise to daughter cells that have equivalent potential as the mother cell, and further that this ability can repeatedly occur throughout the lifetime of an individual without exhaustion), and 3) the ability of hematopoietic stem cells or progeny thereof to be reintroduced into a transplant recipient whereupon they home to the hematopoietic stem cell niche and re-establish productive and sustained hematopoiesis.

Hematopoietic stem cells are optionally obtained from blood products. A blood product includes a product obtained from the body or an organ of the body containing cells of hematopoietic origin. Such sources include unfractionated bone marrow, umbilical cord, placenta, peripheral blood, or mobilized-peripheral blood. All of the aforementioned crude or unfractionated blood products can be enriched for cells having hematopoietic stem cell characteristics in a number of ways. For example, the more mature, differentiated cells are selected against, via cell surface molecules they express. Optionally, the blood product is fractionated by positively selecting for CD34⁺ cells. CD34⁺ cells include a subpopulation of hematopoietic stem cells capable of self-renewal, multi-potency, and that can be reintroduced into a transplant recipient whereupon they home to the hematopoietic stem cell niche and re-establish productive and sustained hematopoiesis. Such selection is accomplished using, for example, commercially available magnetic anti-CD34 beads (Dynal, Lake Success, NY). Unfractionated blood products are optionally obtained directly from a donor or retrieved from cryopreservative storage. Hematopoietic stem cells can also be optionally obtained from differentiated embryonic stem cells, differentiated induced pluripotent stem cells or from other reprogrammed mature cells types.

The terms "stem cell" or "undifferentiated cell" as used herein, refer to a cell in an undifferentiated or partially differentiated state that has the property of self-renewal and has the developmental potential to differentiate into multiple cell types. A stem cell is capable of proliferation and giving rise to more such stem cells while maintaining its functional potential. Stem cells can divide asymmetrically, which is known as obligatory asymmetrical differentiation, with one daughter cell retaining the functional potential of the parent stem cell and the other daughter cell expressing some distinct other specific function, phenotype and/or developmental potential from the parent cell. The daughter cells themselves can be induced to proliferate and produce progeny that subsequently differentiate into one or more mature cell types, while also retaining one or more cells with parental developmental potential. A differentiated cell may derive from a multipotent cell, which itself is derived from a multipotent cell, and so on. Alternatively, some of the stem cells in a population can divide symmetrically into two stem cells. Accordingly, the term "stem cell" refers to any subset of cells that have the developmental potential, under particular circumstances, to differentiate to a more specialized or differentiated phenotype, and which retain the capacity, under certain circumstances, to proliferate without substantially differentiating. In some examples, the term stem cell refers generally to a naturally occurring parent cell whose descendants (progeny cells) specialize, often in different directions, by differentiation, e.g., by acquiring completely individual characters, as occurs in progressive diversification of embryonic cells and tissues. Some differentiated cells also have the capacity to give rise to cells of greater developmental potential. Such capacity may be natural or may be induced artificially upon treatment with various factors. Cells that begin as stem cells might proceed toward a differentiated phenotype, but then can be induced to "reverse" and re-express the stem cell phenotype, a term often referred to as "dedifferentiation" or "reprogramming" or "retrodifferentiation" by persons of ordinary skill in the art.

As used herein, "homing potential" refers to the capability of a stem cell to localize to sites *in vivo* that can support productive hematopoiesis such as the bone marrow.

As used herein, a cell population that is "enriched" in a particular cell type refers to a population in which the relative proportion of cells of a particular type has increased in comparison with a previous population of cells (for example, in comparison with a population of cells prior to treatment with an agent that reduces the level of an ikaros family transcription factor, an agent that inhibits TGFβ signaling, or an agent that inhibits lysine demethylation).

The term "multipotent" when used in reference to a "multipotent cell" refers to a cell that has the developmental potential to differentiate into multiple different hematopoietic cell types. Hematopoietic stem cells are multi-potent and can form the many different types of blood cells (red, white, platelets, etc.), but it cannot form neurons.

As used herein, the phrases "preserve multi-potency" or "maintain multi-potency" refer to a process by which the degree of multi-potency of a population of cells is preserved over a period of time. The degree of multi-potency of a population of cells describes the number and identity of differentiated cell types into which a population of cells can differentiate. For example, a population of cells exhibiting multi-potency that has been maintained over a period of two days *ex vivo* (e.g., in culture) is capable of differentiating into at least the same number of different cell types as the population was capable of differentiating into at the beginning of the cell culture period.

As used herein, a "mobilizing agent" is an agent capable of inducing the migration of hematopoietic stem cells from the bone marrow of a subject to the peripheral blood. Exemplary mobilizing agents include CXCR4 antagonists, such as AMD3100, as well as GCSF and GROβ.

As used herein, "contacting" a population of cells with one or more agents can be achieved in a variety of ways. For instance, a population of hematopoietic stem cells may be contacted with one or more agents that reduce the level of an ikaros family transcription factor, an agent that inhibits TGFβ signaling, an agent that inhibits lysine demethylation, an agent that inhibits p38 signaling, an agent that activates canonical Wnt signaling, or an agent that modulates histone acetylation (e.g., thalidomide, pomalidomide, lenalidomide, LSD1 inhibitor IV RN-1, LSD1 inhibitor II S2101, LSD1 inhibitor LSD1-C76, LSD1 inhibitor III CBB1007, LSD1 inhibitor I, ALK5 inhibitor II (E-616452), LY364947, A83-01, Trichostatin A, Tranylcypromine, SB203580, CHIR99021, DMH1, sodium acetate, and istodax) by culturing the hematopoietic stem cells in the presence of these agents for a period of time, such as for two or more days. When more than one agent is contacted with a population of cells, the agents can be present in the cell culture medium together, such that the cells are exposed to the agents simultaneously. Alternatively, the agents may be added to the cell culture medium sequentially. For instance, the one or more agents may be added to a population of cells in culture according to a particular regimen, e.g., such that different agents are added to the culture media at different times during a culture period.

As used herein, the term "engraftment potential" is used to refer to the ability of hematopoietic stem and progenitor cells to repopulate a tissue, whether such cells are naturally circulating or are provided by transplantation. The term encompasses all events surrounding or leading up to engraftment, such as tissue homing of cells and colonization of cells within the tissue of interest. The engraftment efficiency or rate of engraftment can be evaluated or quantified using any clinically acceptable parameter as known to those of skill in the art and can include, for example, assessment of competitive repopulating units (CRU); incorporation or expression of a marker in tissue(s) into which stem cells have homed, colonized, or become engrafted; or by evaluation of the progress of a subject through disease progression, survival of hematopoietic stem and progenitor cells, or survival of a recipient. In one examples, engraftment is determined by measuring white blood cell counts in peripheral blood during a post-transplant period. Alternatively, engraftment can be assessed by measuring recovery of marrow cells by donor cells in a bone marrow aspirate sample.

As used herein, the term "self-renewal" refers to the ability of a stem cell to produce daughter stem cells with the same phenotype, characteristics and functional potential as the original stem cell. In particular, self-renewal, as used herein, is defined as the ability to continue proliferation while maintaining an undifferentiated multi-potent stem cell state.

As used herein, a population of cells that has been "freshly isolated" from a donor refers to a population of cells that has been isolated from a donor without having been cryopreserved and thawed prior to infusion. A population of cells may be isolated from a donor and separated into two intermediate populations, one of which may be infused into a patient and the other of which may be cryopreserved. In this instance, the intermediate population that is infused into the patient is considered freshly isolated. A population of cells is considered to be freshly isolated from a donor if the population is cultured *ex vivo* prior to infusion in the patient. For example, this culturing step may be performed in order to expand, enrich, and/or maintain the population of hematopoietic stem cells prior to administration of the resulting cells to a patient. In these instances, the resulting cells are considered to be freshly isolated from the donor, to the extent that the cells that are administered to the patient have not been cryopreserved and thawed prior to infusion into the patient.

The terms "decrease", "reduced", "reduction", or "inhibit" are all used herein to mean a decrease by a statistically significant amount. In some examples, "reduce," "reduction" or "decrease" or "inhibit" typically means a decrease by at least 10% as compared to a reference level (e.g. the absence of a given treatment) and can include, for example, a decrease by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , or more. As used herein, "reduction" or "inhibition" does not encompass a complete inhibition or reduction as compared to a reference level. "Complete inhibition" is a 100% inhibition as compared to a reference level.

As used herein, "CRU" (competitive repopulating unit) refers to a unit of measure of long-term engrafting stem cells, which can be detected after in-vivo transplantation.

As used herein, the term "modulate" means to change or induce an alteration in a particular biological activity. Modulation includes, but is not limited to, stimulating or inhibiting an activity (e.g., by activating a receptor so as to initiate a signal transduction cascade, to inhibit a receptor from propagating a signaling pathway, by activating an endogenous inhibitor that attenuates a biological activity, or by inhibiting the activity of a protein that inhibits a particular biological function. Modulation of a protein that propagates a particular signaling pathway may result in an increase or a decrease in activity (e.g., ikaros family member-mediated transcription, TGFβ signaling, lysine demethylation, p38 signaling, Wnt signaling, histone methylation, or histone acetylation), a change in the affinity of one protein in a pathway for another, or another change in the structural, functional, or immunological properties associated with the activity of a pathway or protein within a pathway.

The terms "increased", "increase", "enhance", or "activate" are all used herein to mean an increase by a statistically significant amount. In some examples, the terms "increased", "increase", "enhance", or "activate" can mean an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level. In the context of a marker, an "increase" is a statistically significant increase in such level.

As used herein, a "subject" means a human or animal. Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, and hamsters. Domestic and game animals include cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. In some examples, the subject is a mammal, e.g., a primate, e.g., a human. The terms, "individual," "patient" and "subject" are used interchangeably herein.

As used herein, a "recipient" is a patient that receives a transplant, such as a transplant containing a population of hematopoietic stem cells or a population of differentiated cells. The transplanted cells administered to a recipient may be, e.g., autologous, syngeneic, or allogeneic cells.

As used herein, a "donor" is a human or animal from which one or more cells are isolated prior to administration of the cells, or progeny thereof, into a recipient. The one or more cells may be, e.g., a population of hematopoietic stem cells to be expanded, enriched, or maintained according to the methods of the disclosure prior to administration of the cells or the progeny thereof into a recipient.

Preferably, the subject is a mammal. The mammal can be a human, non-human primate, mouse, rat, dog, cat, horse, or cow, but is not limited to these examples. Mammals other than humans can be advantageously used as subjects that represent animal models of disease and/or treatment. A subject can be male or female.

As used herein, the terms "protein" and "polypeptide" are used interchangeably herein to designate a series of amino acid residues, connected to each other by peptide bonds between the alpha-amino and carboxy groups of adjacent residues. The terms "protein", and "polypeptide" refer to a polymer of amino acids, including modified amino acids (e.g., phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. The terms "protein" and "polypeptide" are used interchangeably herein when referring to a gene product and fragments thereof. Thus, exemplary polypeptides or proteins include gene products, naturally occurring proteins, homologs, orthologs, paralogs, fragments and other equivalents, variants, fragments, and analogs of the foregoing.

As used herein, the term "polynucleotide", "nucleic acid" or "nucleic acid sequence" refers to any molecule, preferably a polymeric molecule, incorporating units of ribonucleic acid, deoxyribonucleic acid or an analog thereof. The nucleic acid can be either single-stranded or double-stranded. A single-stranded nucleic acid can be one nucleic acid strand of a denatured double- stranded DNA. Alternatively, it can be a single-stranded nucleic acid not derived from any double-stranded DNA. In one example, the nucleic acid can be DNA. In another example, the nucleic acid can be RNA. In another example, the nucleic acid can be chemically modified-RNA. For instance, the nucleic acid can be chemically modified messenger RNA. In another example, the nucleic acid can be RNA synthesized with naturally occurring or synthetic nucleotide analogs. Suitable nucleic acid molecules are DNA, including genomic DNA or cDNA. Other suitable nucleic acid molecules are RNA, including mRNA, tRNA, siRNA, miRNA, or shRNA.

A used herein, the term "siRNA" refers to a double stranded nucleic acid molecule capable of RNA interference or "RNAi", as disclosed, for example, in Bass, Nature 411:428 (2001); Elbashir et al. Nature 411:494 (2001); WO 2000/044895; WO 2001/036646; WO 1999/032619; WO 2000/001846; WO 2001/029058; WO 1999/007409; and WO 2000/044914. As used herein, siRNA molecules are not limited to those molecules containing only RNA, but further encompass chemically modified nucleotides and non-nucleotides having RNAi capacity or activity.

As used herein, the term "miRNA" refers to a class of small, non-coding, single-stranded RNA, typically between 18-23 nucleotides in length. miRNA molecules are capable of regulating gene expression by modulating the stability and translation of mRNA encoding specific proteins. miRNA also influence other nuclear processes, such as heterochromatin formation and genome rearrangement.

As used herein, the term "shRNA" (small hairpin RNA) refers to an RNA duplex containing siRNA, part of which is in the form of a hairpin structure. In addition to the duplex portion, the hairpin structure may contain a loop portion positioned between the two sequences that form the duplex. The loop can vary in length. For instance, the loop may be 5, 6, 7, 8, 9, 10, 11, 12 or 13 nucleotides in length. The hairpin structure can also contain 3' or 5' overhang portions. For example, the overhang may be a 3' or a 5' overhang and may be 0, 1, 2, 3, 4 or 5 nucleotides in length.

As used herein, the term "pharmaceutical composition" refers to the active agent in combination with a pharmaceutically acceptable carrier e.g. a carrier commonly used in the pharmaceutical industry. The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "administering," refers to the placement of a compound, cell, or population of cells as disclosed herein into a subject by a method or route which results in at least partial delivery of the agent at a desired site. Pharmaceutical compositions comprising the compounds or cells disclosed herein can be administered by any appropriate route which results in an effective treatment in the subject.

The term "statistically significant" or "significantly" refers to statistical significance and generally means a two standard deviation (2SD) or greater difference.

Other than in the operating examples, or where otherwise indicated, all numbers expressing quantities of ingredients or reaction conditions used herein should be understood as modified in all instances by the term "about." As used herein, the term "about" indicates a deviation of ±10%.

As used herein the term "comprising" or "comprises" is used in reference to compositions, methods, and respective component(s) thereof, that are essential to the method or composition, yet open to the inclusion of unspecified elements, whether essential or not.

The term "consisting of' refers to compositions, methods, and respective components thereof as described herein, which are exclusive of any element not recited in that description of the embodiment.

As used herein the term "consisting essentially of' refers to those elements required for a given embodiment. The term permits the presence of elements that do not materially affect the basic and novel or functional characteristic(s) of that embodiment.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a nonlimiting example. Thus, the abbreviation "e.g." is synonymous with the term "for example."

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims. Definitions of common terms in immunology and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737).

As used herein, "thalidomide" refers to a compound having the structure of Formula (**2**), below.

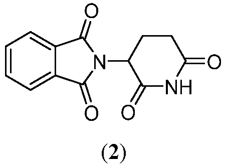

As used herein, "pomalidomide" refers to a compound having the structure of Formula (**3**), below. The synthesis and biological activity of pomalidomide is described, e.g., in US 6,335,349.

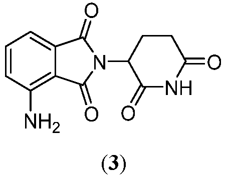

As used herein, "lenalidomide" refers to a compound having the structure of Formula (**4**), below. The synthesis and biological activity of lenalidomide is described, e.g., in US 6,281,230.

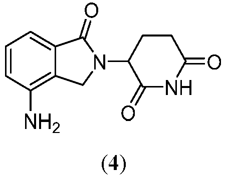

As used herein, "UM171" refers to a compound having the structure of Formula (**5**) or a pharmaceutically acceptable salt thereof, such as the hydrochloride salt or the hydrobromide salt thereof. The synthesis and biological activity of UM171 is described, e.g., in US 2015/0011543.

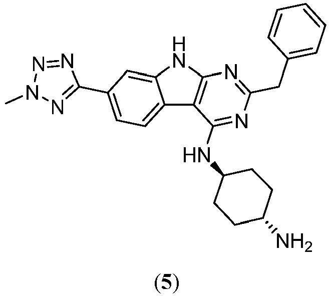

As used herein, "LSD1 inhibitor II S2101" refers to a compound having the structure of Formula (**6**) or a pharmaceutically acceptable salt thereof. The synthesis of LSD1 inhibitor II S2101 as well as the structure and synthesis of related compounds, e.g., derivatives, including those with similar biological activity are known in the art, see, e.g. WO 1998/009625; WO 2003/016309; WO 2004/087646; WO 2005/033066; WO 2006/034769; WO 2007/068330; WO 2009/147217; WO 2014/194280; US 2013/0178520; US 5,426,196; EP 764640; and EP 764632.

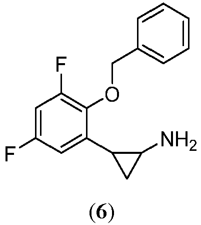

As used herein, "LSD1 inhibitor IV RN-1" refers to a compound having the structure of Formula (**7**) or a pharmaceutically acceptable salt thereof. The synthesis of LSD1 inhibitor IV RN-1 as well as the structure and synthesis of related compounds, e.g., derivatives, including those with similar biological activity are known in the art, see, e.g. WO 2015/003643; WO 2014/205266; WO 2014/19428; WO 2012/122405; WO 2014/018375; WO 2009/147217; WO 2007/068330; WO 2006/034769; WO 2005/033066; WO 2004/087646; WO 2003/016309; WO 1998/009625; WO 1997/010822; WO 1997/010825; WO 1995/017408; and US 2013/0178520.

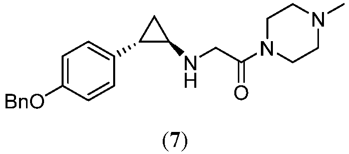

As used herein, "LSD1 inhibitor LSD1-C76" refers to a compound having the structure of Formula (**8**) or a pharmaceutically acceptable salt thereof. The synthesis of LSD1 inhibitor LSD1-C76 as well as the structure and synthesis of related compounds, e.g., derivatives, including those with similar biological activity are known in the art.

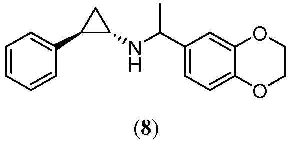

As used herein, "LSD1 inhibitor III CBB1007" refers to a compound having the structure of Formula (**9**) or a pharmaceutically acceptable salt thereof. The synthesis of LSD1 inhibitor III CBB1007 as well as the structure and synthesis of related compounds, e.g., derivatives, including those with similar biological activity are known in the art.

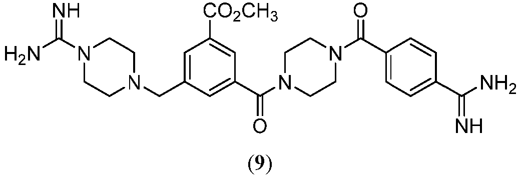

As used herein, "LSD1 inhibitor I" refers to a compound having the structure of Formula (**10**) or a pharmaceutically acceptable salt thereof. LSD1 inhibitor I is also referred to in the art as BHC110 Inhibitor I, Histone Lysine Demethylase Inhibitor III, and/or KDM1 Inhibitor I. The synthesis of LSD1 inhibitor I as well as the structure and synthesis of related compounds, e.g., derivatives, including those with similar biological activity are known in the art.

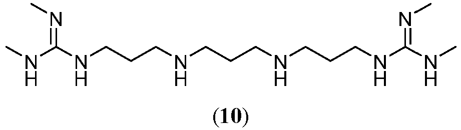

As used herein, "ALK5 inhibitor II" (also referred to as "RepSox" or "E-616452" refers to a compound having the structure of Formula (**11**) or a pharmaceutically acceptable salt thereof. ALK5 inhibitor II (E-616452) is also referred to in the art as Transforming Growth Factor-b Type I Receptor Kinase Inhibitor II or Repsox.

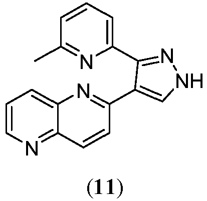

As used herein, "LY364947" refers to a compound having the structure of Formula (**12**) or a pharmaceutically acceptable salt thereof. LY364947 is also referred to in the art as ALK5 Inhibitor I TbR-I Inhibitor Transforming Growth Factor-b Type I Receptor Kinase Inhibitor. The synthesis of LY364947 and ALK5 inhibitor II as well as the structure and synthesis of related compounds, e.g., derivatives, including those with similar biological activity are known in the art, see, e.g. US 6,028,072; WO 2002/062794; WO 2004/026302; WO 2004/026306; WO 2004/072033; WO 2007/088651; WO 2007/070866; WO 2007/039151; and WO 2007/052943.

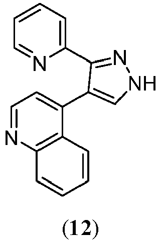

As used herein, "A83-01" refers to a compound having the structure of Formula (**13**) or a pharmaceutically acceptable salt thereof. The synthesis of A83-01 as well as the structure and synthesis of related compounds, e.g., derivatives, including those with similar biological activity are known in the art, see, e.g., US 2003/0064997; US 2003/0064997; US 2003/0064997; US 5,777,097; US 5,871,934; GB 2306108; WO 1993/014081; WO 1995/003297; WO 1997/33883; WO 1997/35855; and WO 1993/014081.

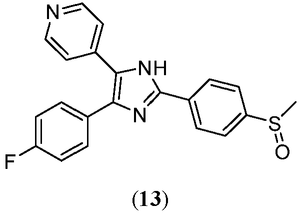

As used herein, "trichostatin A" refers to a compound having the structure of Formula (**14**) or a pharmaceutically acceptable salt thereof. The synthesis of trichostatin A as well as the structure and synthesis of related compounds, e.g., derivatives, including those with similar biological activity are known in the art, see, e.g., US 4,690,918; US 4,946,999; EP 0827946; JP 07206670; and JP 60149520.

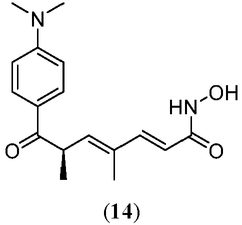

As used herein, "tranylcypromine" refers to a compound having the structure of Formula (**15**) or a pharmaceutically acceptable salt thereof. The synthesis of tranylcypromine as well as the structure and synthesis of related compounds, e.g., derivatives, including those with similar biological activity are known in the art, see, e.g.US 2,993,931; US 2,997,422; US 3,011,945; US 3,079,403; US 3,134,676; and US 3,244,596.

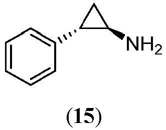

As used herein, "SB203580" refers to a compound having the structure of Formula (**16**) or a pharmaceutically acceptable salt thereof. The synthesis of SB203580 as well as the structure and synthesis of related compounds, e.g., derivatives, including those with similar biological activity are known in the art, see, e.g. WO 2007/070866; WO 2008/022182; WO 2010/065917; WO 2010/077955; and WO 2010/102267.

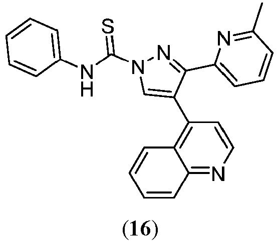

As used herein, "CHIR99021" refers to a compound having the structure of Formula (**17**) or a pharmaceutically acceptable salt thereof. The synthesis of CHIR99021 as well as the structure and synthesis of related compounds, e.g., derivatives, including those with similar biological activity are known in the art, see, e.g. WO 1999/065897; WO 2002/020495; WO 2005/003948; WO 2006/001863; WO 2006/117212; WO 2007/016485; WO 2007/075911; WO 2007/083978; and US 2002/0156087.

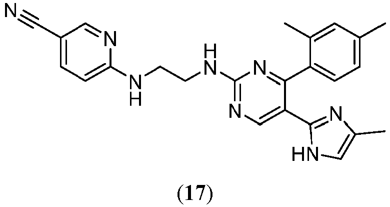

As used herein, "DMH1" refers to a compound having the structure of Formula (**18**) or a pharmaceutically acceptable salt thereof. The synthesis of DMH1 as well as the structure and synthesis of related compounds, e.g., derivatives, including those with similar biological activity are known in the art, see, e.g. WO 2012/115120; WO 2013/016452; WO 2013/163228; WO 2013/166488; WO2014/138088; WO 2014/176606; WO 2014/200115; WO2014/062138; US 2014/0248696; and US 8,822,684.

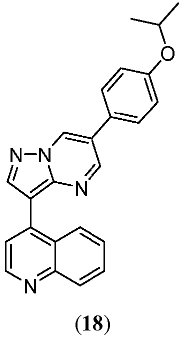

As used herein, "istodax" or "romidepsin" refers to a compound having the structure of Formula (**19**) or a pharmaceutically acceptable salt thereof. The synthesis of istodax as well as the structure and synthesis of related compounds, e.g., derivatives, including those with similar biological activity are known in the art, see, e.g., WO14/102731; WO12/009336; WO13/106696; WO02/20817; US 4,977,138; US 7,611,721; US 7,608,280; and US 2012/046442.

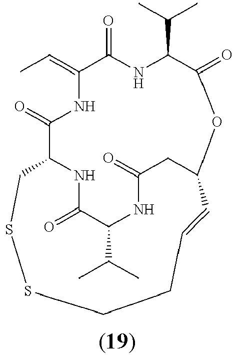

Other terms are defined herein within the description of the disclosure.

### BRIEF DESCRIPTION OF THE DRAWINGS

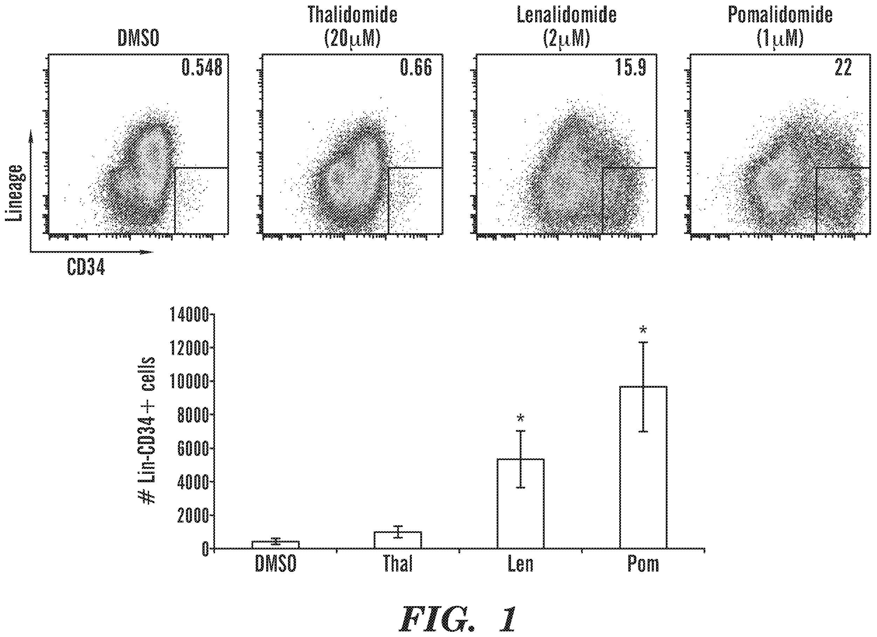

Figure 1 shows a series of fluorescence activated cell sorting (FACS) diagrams and a bar graph obtained from analysis of hematopoietic stem cells isolated from human mobilized peripheral blood and cultured in the presence of thalidomide, lenalidomide, or pomalidomide at the indicated concentrations for 14 days. * indicates statistically significant increase versus control DMSO cultured cells.

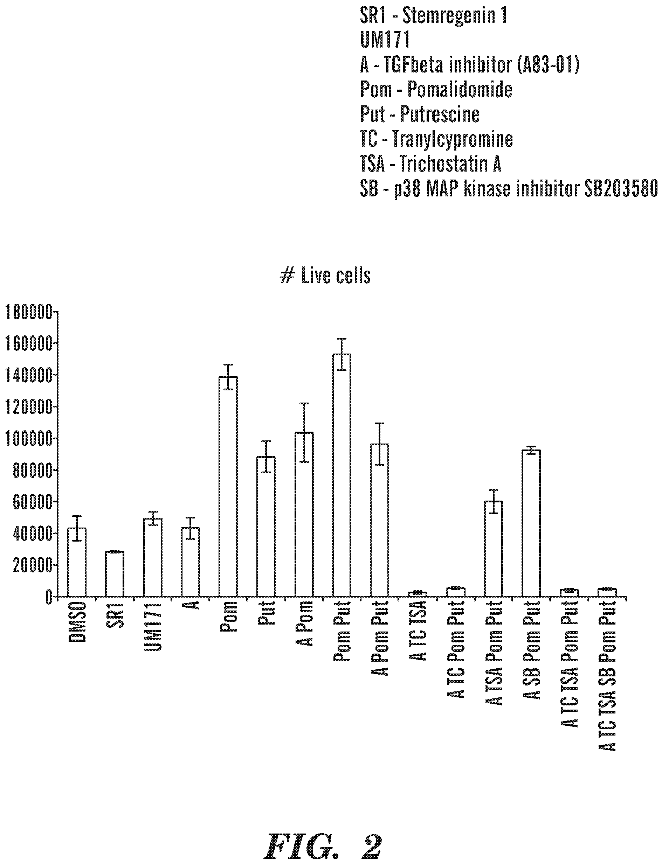

Figure 2 is a bar graph showing the quantity of live cells present following the culture of hematopoietic stem cells isolated from human cord blood and cultured in the presence of the indicated reagents for 12 days.

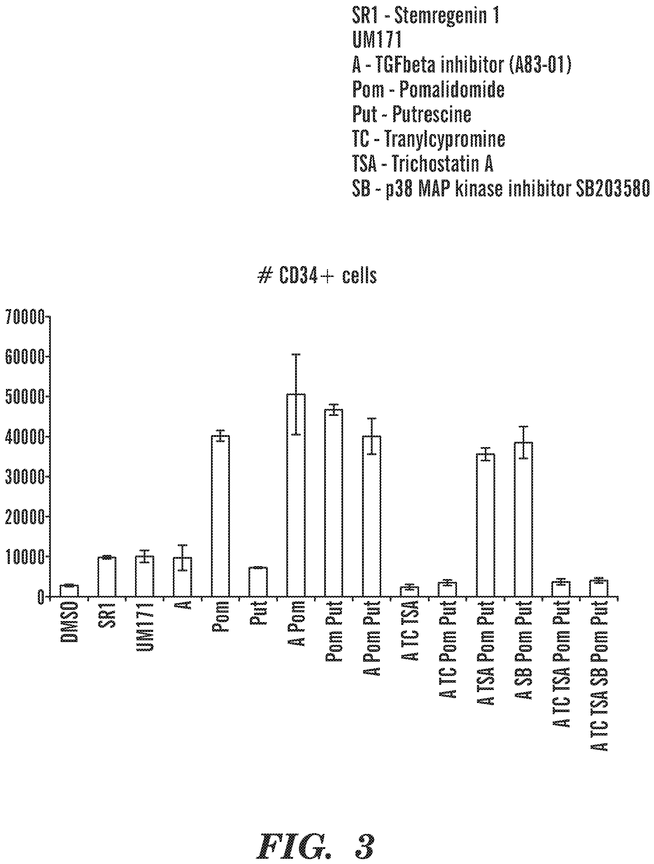

Figure 3 is a bar graph showing the quantity of CD34+ cells present following the culture of hematopoietic stem cells isolated from human cord blood and cultured in the presence of the indicated reagents for 12 days.

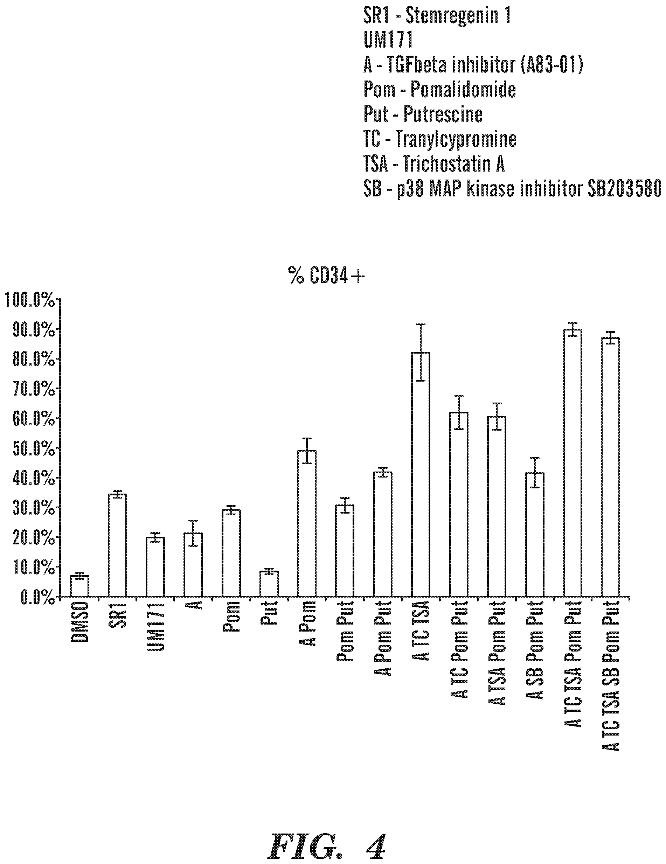

Figure 4 is a bar graph showing the percentage of CD34+ cells present in a population of cells obtained following the culture of hematopoietic stem cells isolated from human cord blood and cultured in the presence of the indicated reagents for 12 days.

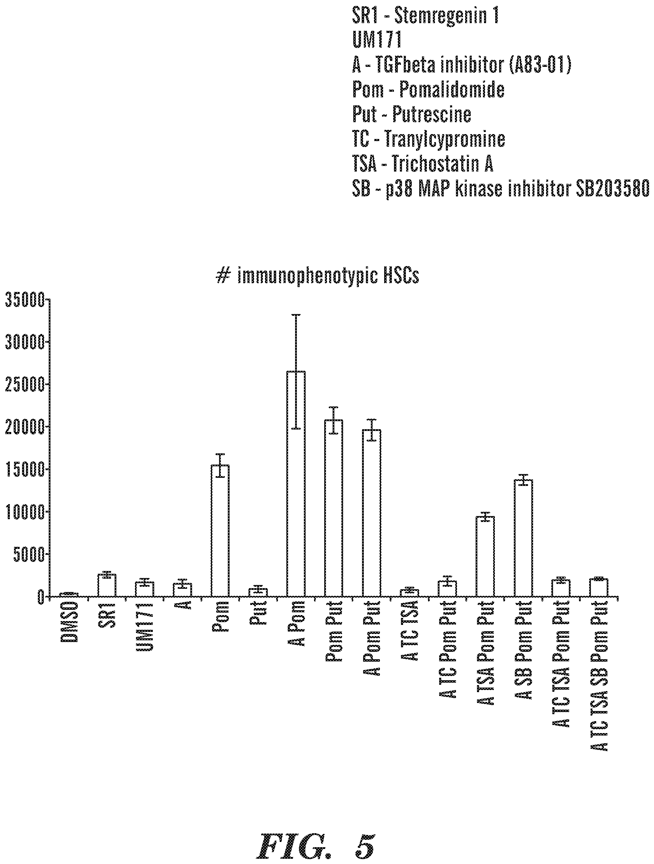

Figure 5 is a bar graph showing the quantity of hematopoietic stem cells (as defined as lineage-, CD34+, CD38-, CD45RA-, CD90+, CD49f+) present following the culture of hematopoietic stem cells isolated from cord blood and cultured in the presence of the indicated reagents for 12 days.

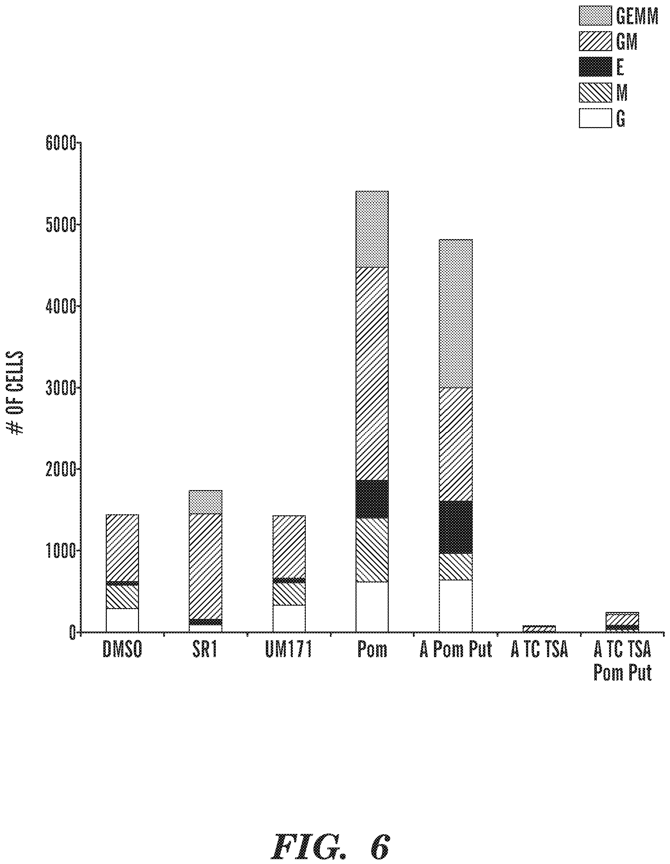

Figure 6 is a bar graph showing the total colony number and composition of colonies obtained following the culture of hematopoietic stem cells isolated from human cord blood and cultured in the presence of the indicated reagents for 12 days. Cells were seeded into methocult media (H4434, Stem Cell Technologies) and colonies were scored 16 days post-seeding. Reagent names are as indicated in Figures 2-5.

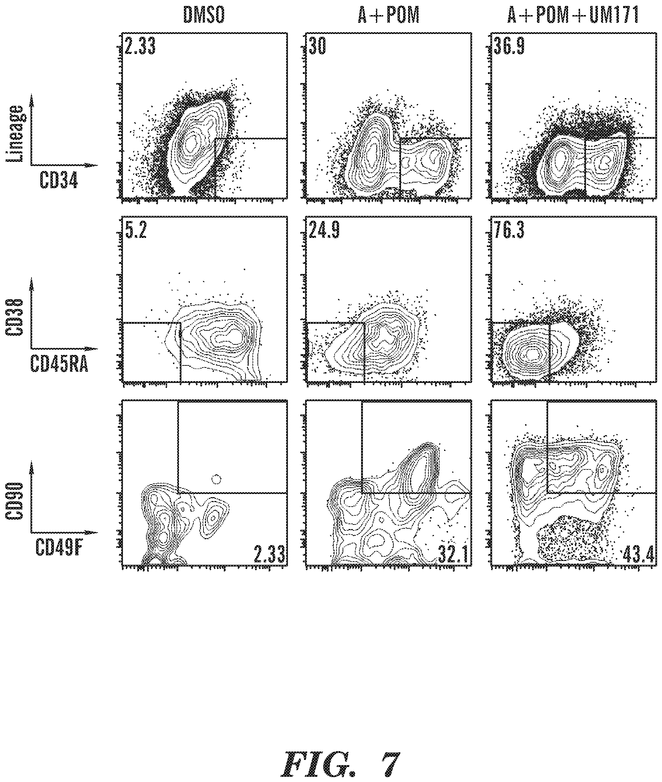

Figure 7 shows a series of FACS diagrams obtained from the analysis of CD34+ cells isolated from human mobilized peripheral blood and cultured in the presence of the indicated reagents for 21 days. Reagent names are as indicated in Figures 2-5.

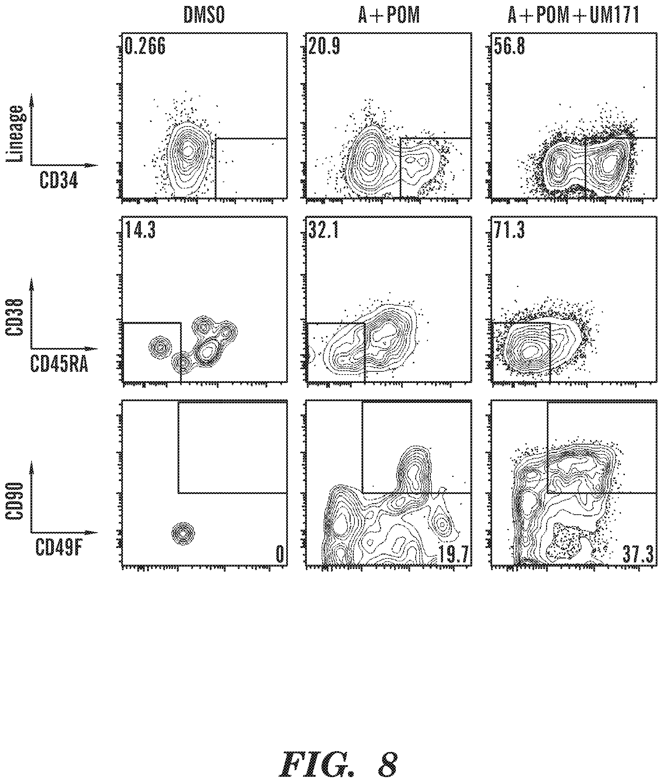

Figure 8 shows a series of FACS diagrams obtained from the analysis of hematopoietic stem cells isolated from human mobilized peripheral blood and cultured in the presence of the indicated reagents for 21 days. Reagent names are as indicated in Figures 2-5.

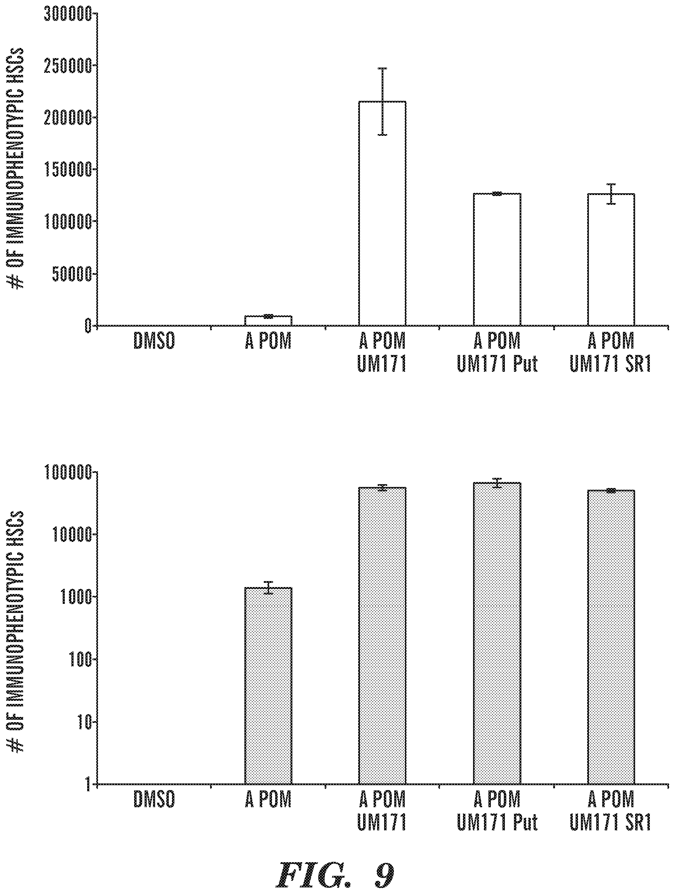

Figure 9 shows a series of bar graphs indicating the quantity of hematopoietic stem cells present in a population of cells obtained from 500 starting CD34+ cells (top) or 50 starting hematopoietic stem cells (bottom) from human peripheral blood and culturing the cells in the presence of the indicated reagents for 21 days. Reagent names are as indicated in Figures 2-5.

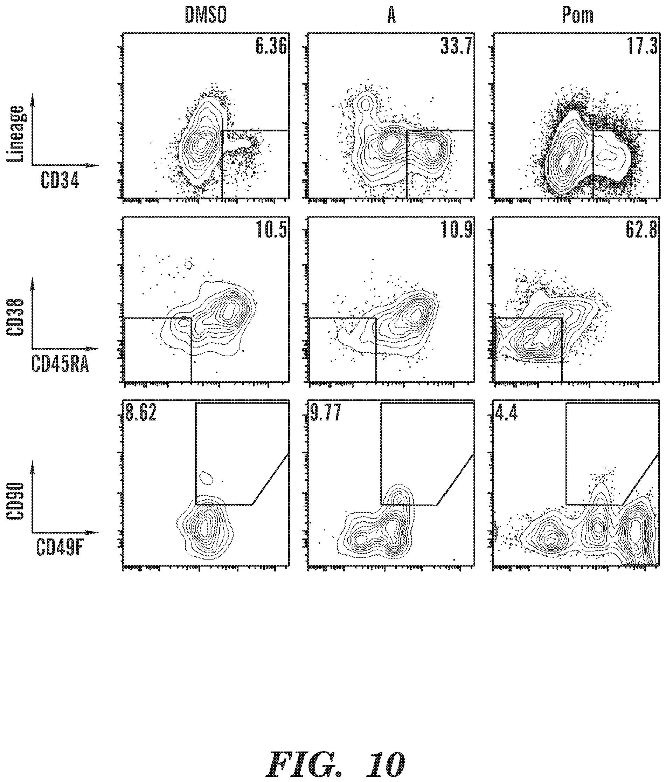

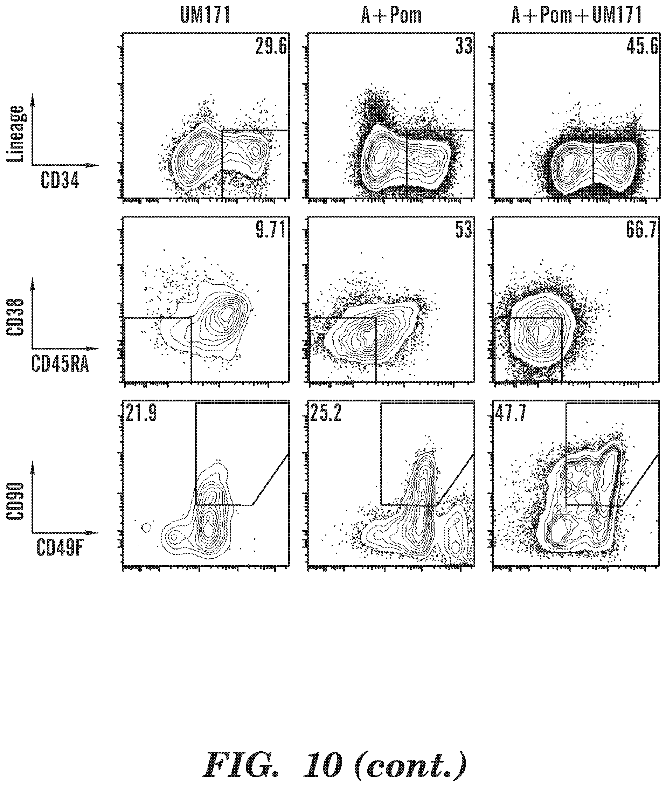

Figure 10 shows a series of FACS diagrams obtained from the analysis of hematopoietic stem cells obtained from human mobilized peripheral blood and cultured in the presence of the indicated reagents for 14 days. Reagent names are as indicated in Figures 2-5.

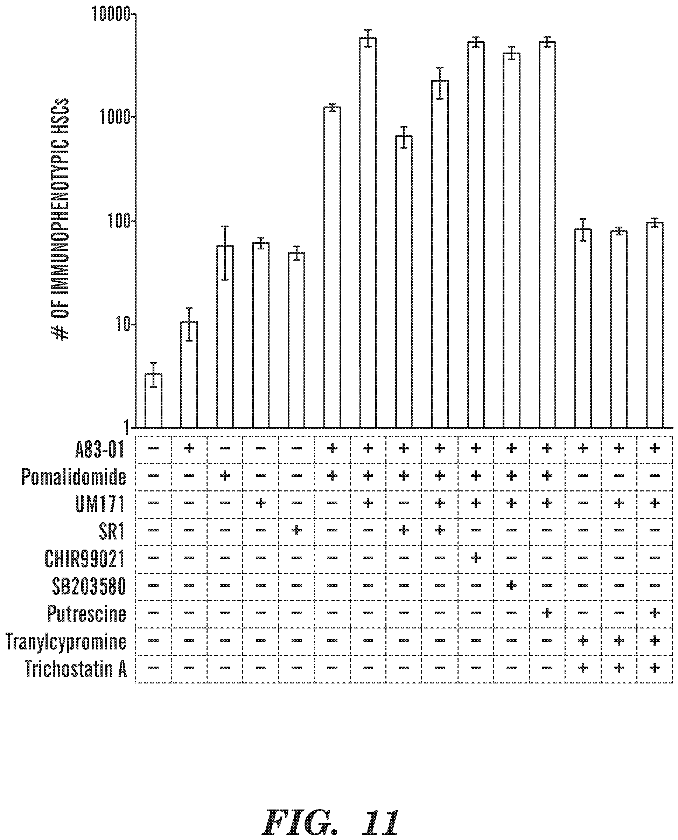

Figure 11 is a bar graph showing the quantity of hematopoietic stem cells having the phenotype Lin-CD34+CD38-CD45RA-CD90+CD49F+CD41- obtained from hematopoietic stem cells isolated from human mobilized peripheral blood and cultured in the presence of the indicated reagents for 14 days.

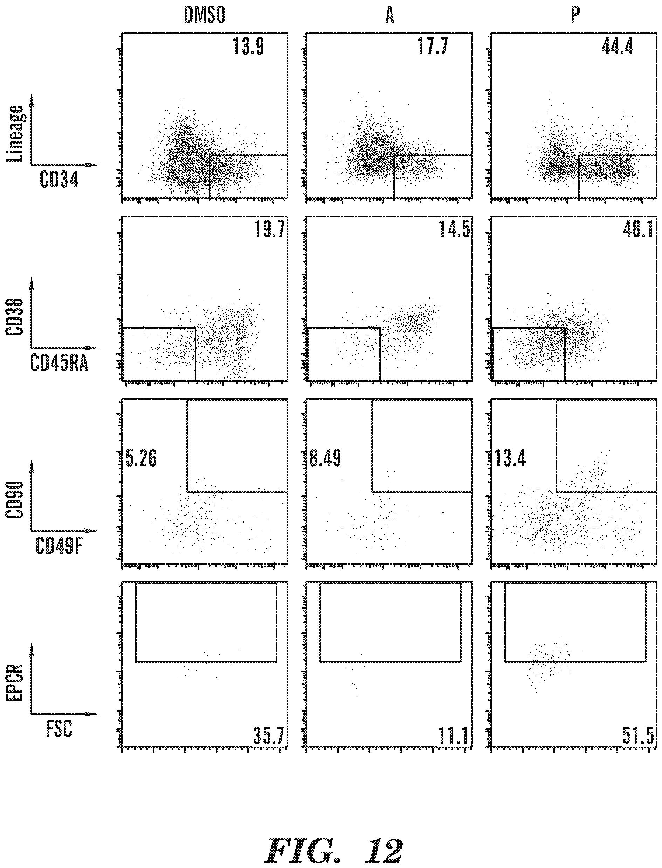

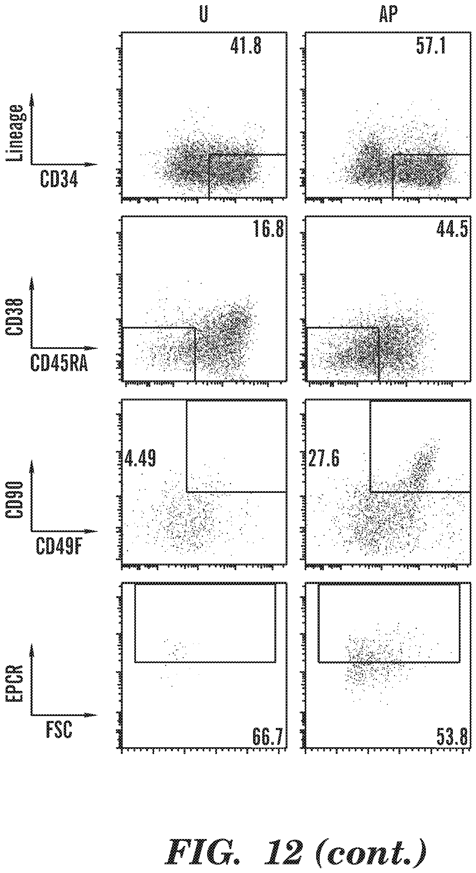

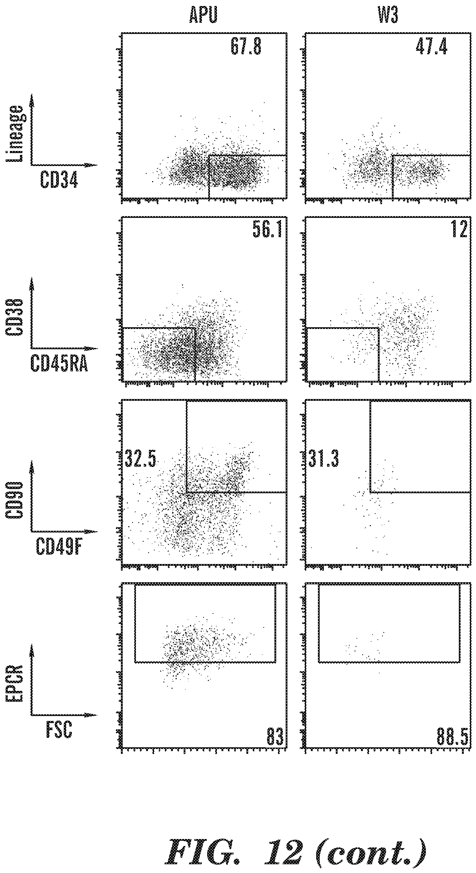

Figure 12 shows a series of FACS diagrams obtained from the analysis of CD34+ cells obtained from human cord blood and cultured in the presence of the indicated reagents for 14 days. Reagent names are as indicated in Figures 2-5. W3 indicates a mixture of A83-01, Tranylcypromine, and Trichostatin A.

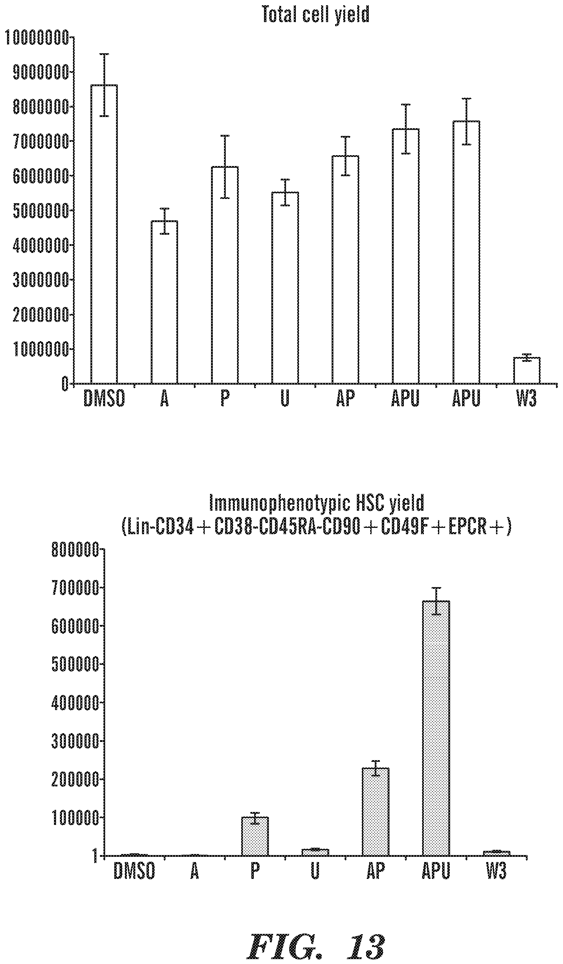

Figure 13 shows a series of bar graphs indicating total cell yield (left) and hematopoietic stem cell yield (right) following culturing of CD34+ cells isolated from human cord blood in the presence of the indicated reagents for 14 days. Reagent names are as indicated in Figures 2-5. Reagent names are as indicated in Figures 2-5 and 12.

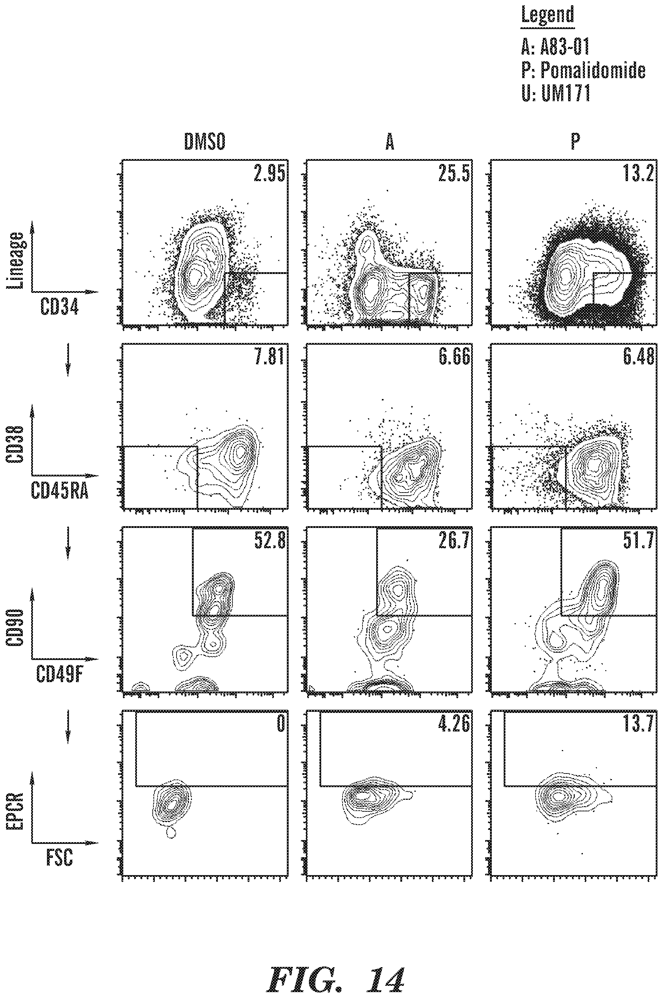

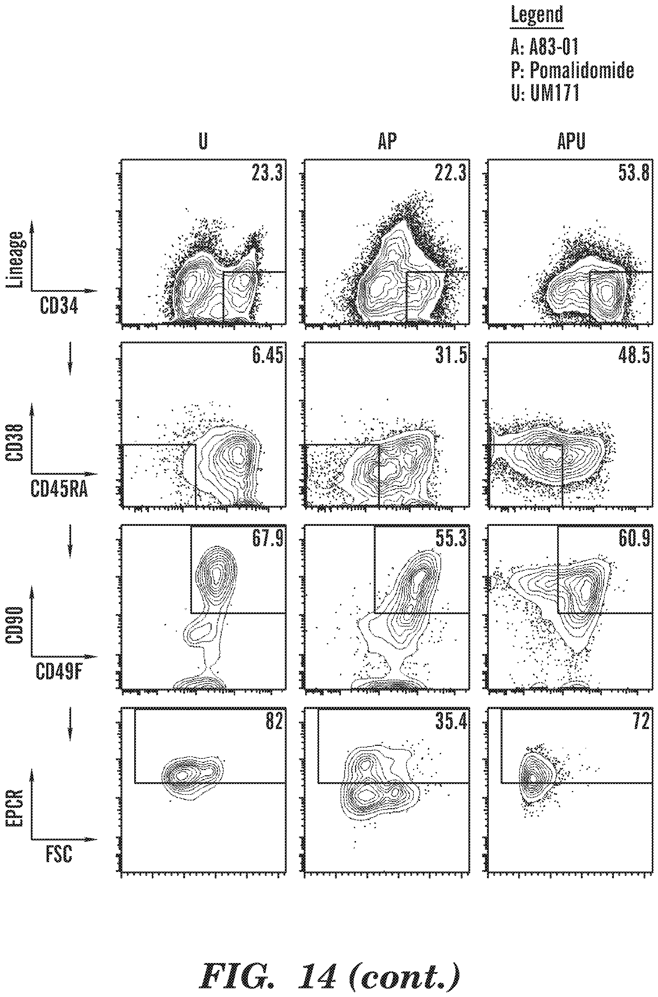

Figure 14 shows a series of FACS diagrams obtained from the analysis of hematopoietic stem cells obtained from human bone marrow and cultured in the presence of the indicated reagents for 14 days.

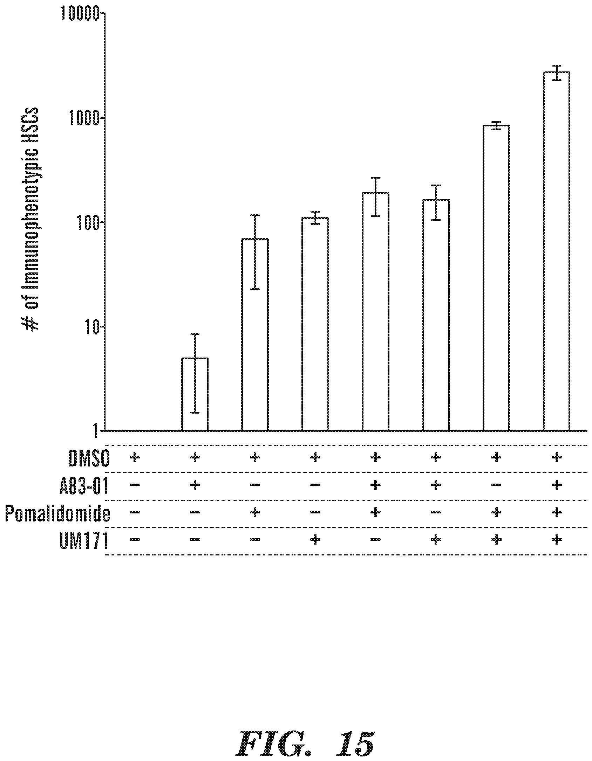

Figure 15 is a bar graph indicating the total quantity of hematopoietic stem cells obtained from the isolation of hematopoietic stem cells from human bone marrow and the culturing of these cells in the presence of the indicated reagents for 14 days.

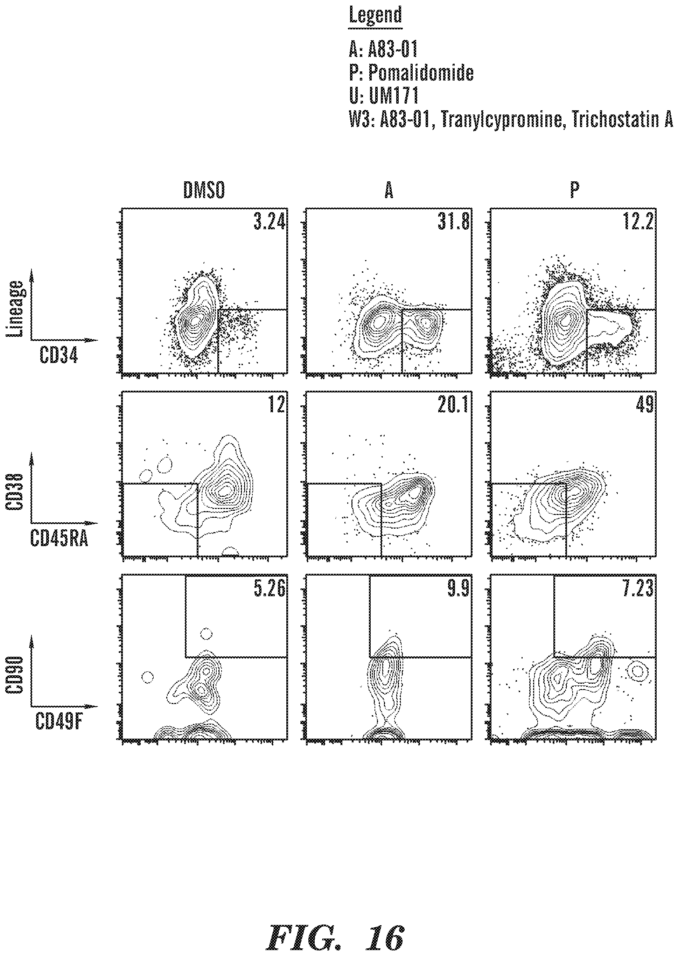

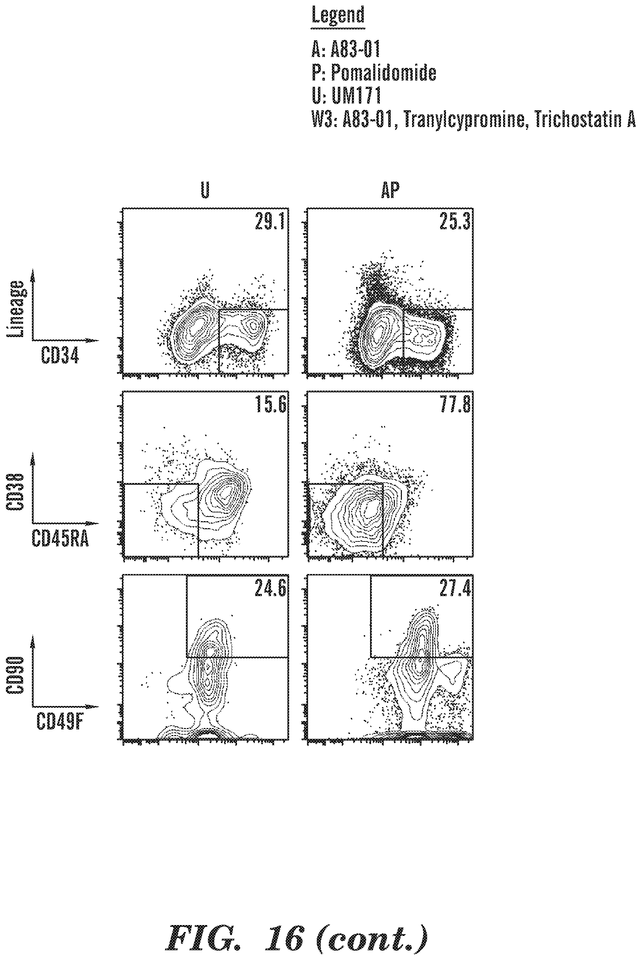

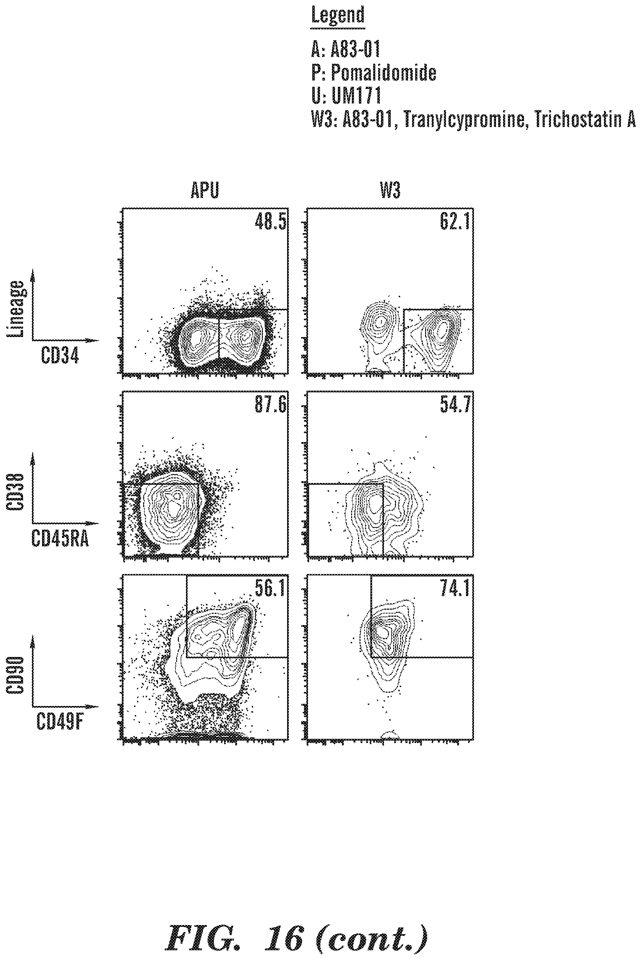

Figure 16 shows a series of FACS diagrams obtained from the analysis of hematopoietic stem cells obtained from human mobilized peripheral blood and cultured in the presence of the indicated reagents for 14 days.

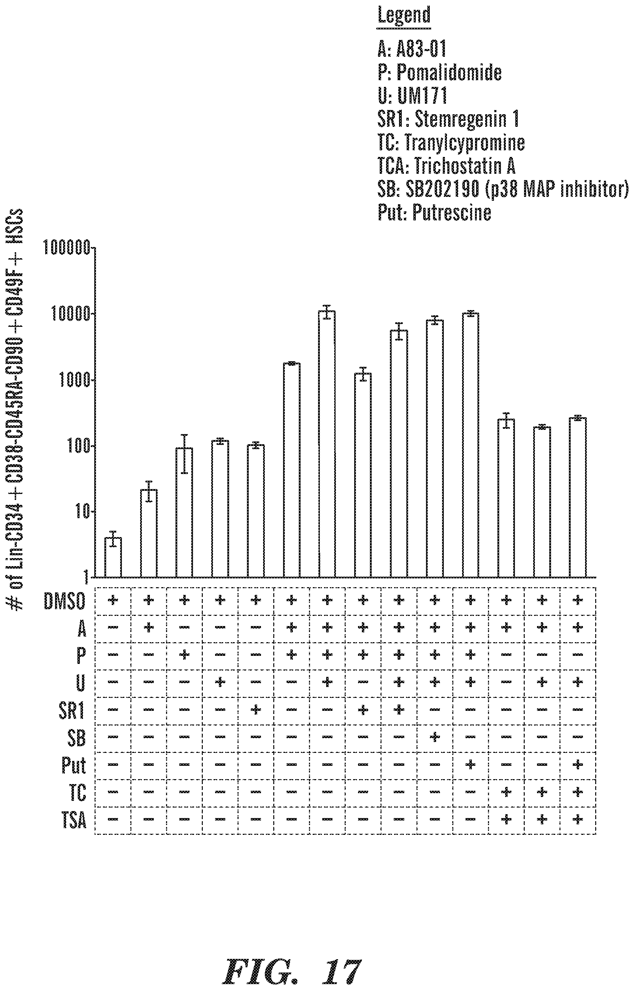

Figure 17 is a bar graph showing the total quantity of phenotypic hematopoietic stem cells present in a population of cells obtained from the isolation of hematopoietic stem cells from human mobilized peripheral blood and the culturing of these cells in the presence of the indicated reagents for 14 days.

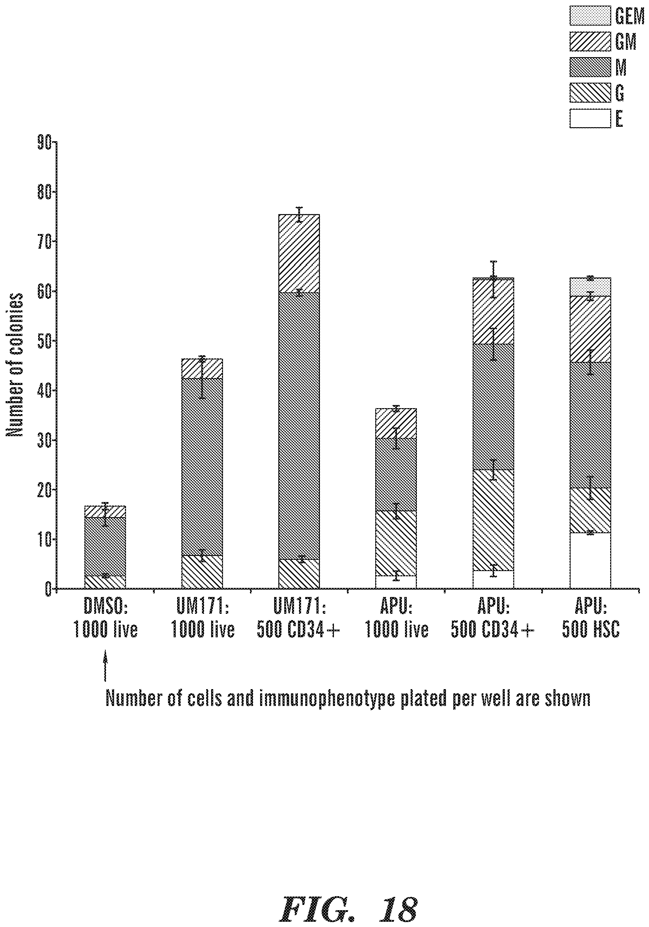

Figure 18 is a bar graph showing the quantity and composition of colonies obtained from cultures of hematopoietic stem cells from human mobilized peripheral blood cultured in the presence of the indicated reagents for 21 days. Cells were passaged every 3 days starting at day 15. The indicated numbers of cells were isolated from the day 21 cultures and were seeded into methocult media (H4434, Stem Cell Technologies) and colonies were scored 16 days post-seeding.

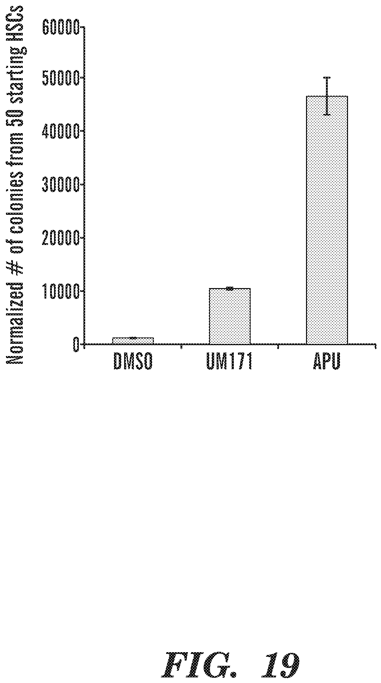

Figure 19 is a bar graph showing the normalized quantity of colonies obtained from the isolation of hematopoietic stem cells from human mobilized peripheral blood and the culturing of these cells in the presence of the indicated reagents for 21 days. Cells were passaged every 3 days starting at day 15. Cells were isolated from the day 21 cultures and seeded into methocult media (H4434, Stem Cell Technologies) and colonies were scored 16 days post-seeding. Total colony number is shown.

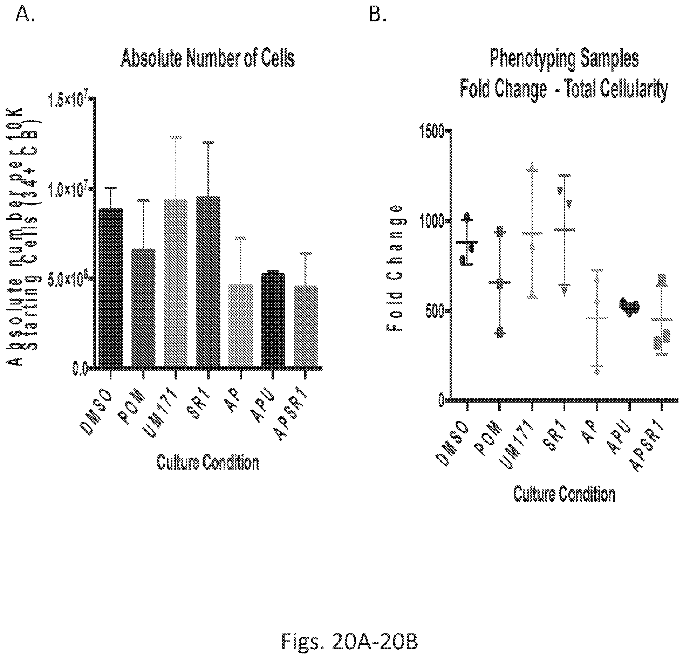

Figs. 20A-20B depict the absolute number and fold change in cellularity of CD34+ cord blood cells after 12 days in culture. CD34+ enriched cord blood cells were obtained from AllCells. Ten thousand cells were cultured in StemSpan SFEMII containing recombinant human SCF, TPO, IL-3 and FLT3L (all at 100 ng/ml). Cells were cultured with cytokine containing media with the vehicle control (DMSO) or the indicated small molecules. Media was changed every three days. On day twelve, cells were harvested, counted, and analyzed by flow cytometry. Fig. 20A depicts the absolute number of live cells after 12 days of culture. No significant differences detected using One-way ANOVA. Fig. 20B depicts the fold change from 10,000 stating cells. POM, pomalidomide; SR1, StemRegenin 1; A, A83-01; AP, A + POM; APU, A+POM+UM171; APSR1, A+POM+SR1.

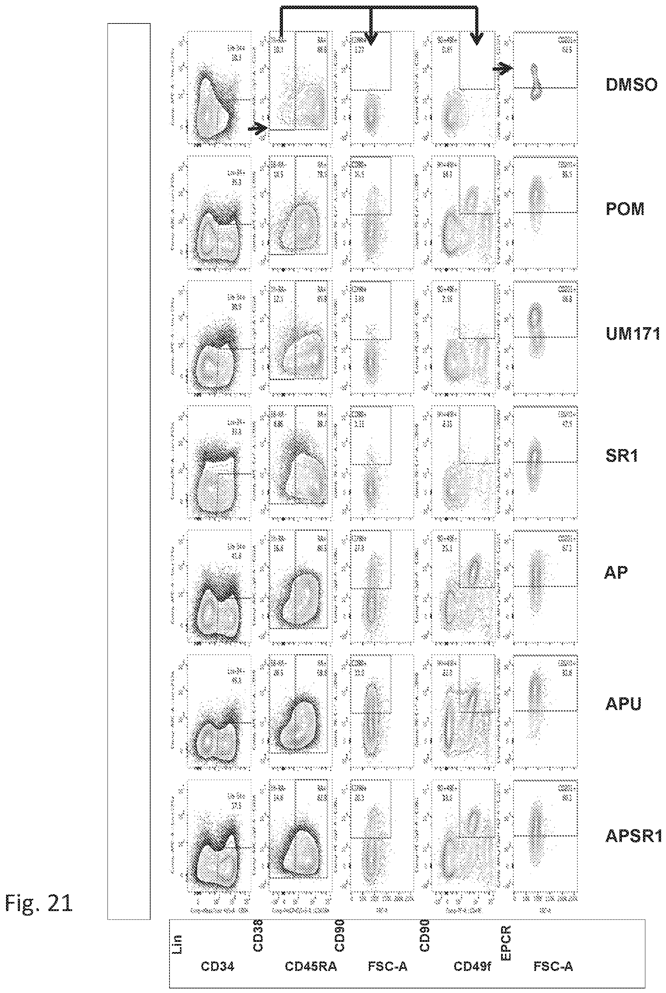

Fig. 21 depicts representative Dot Plots and gating strategy for expanded CD34+ cord blood cells. On day 12 after culture in the indicated compounds, samples were analyzed by flow cytometry. Plots show samples previously gated through live (PI-) and singlet gates. Gating strategy for immunophenotypic hematopoietic stem cells (HSCs) defined as Lin-CD34+CD45RA-CD90+ and Lin-CD34+CD45RA-CD90+CD49f+EPCR+ HSCs is shown. Percentages indicate the frequency of the parent (previous) gate. POM, pomalidomide; SR1, StemRegenin 1; A, A83-01; AP, A + POM; APU, A+POM+UM171; APSR1, A+POM+SR1.

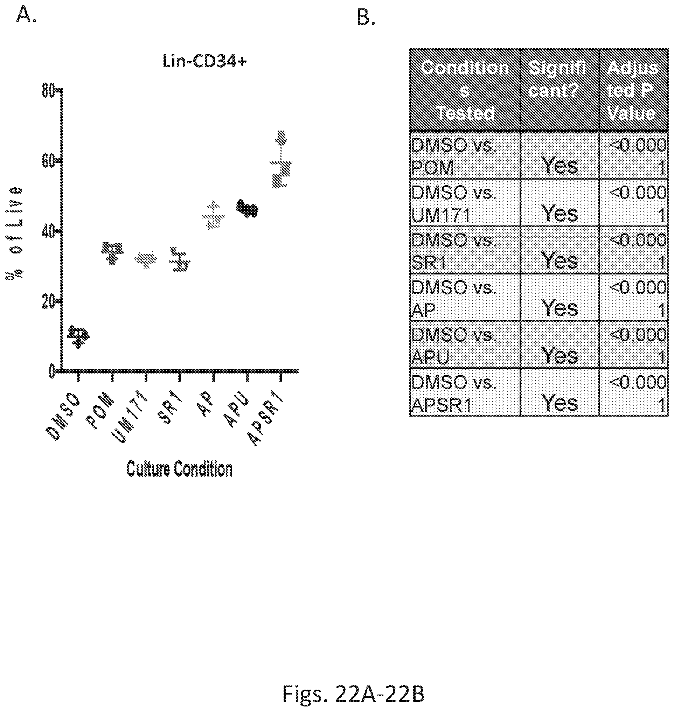

Figs. 22A-22B depict the frequency of Lin-CD34+ Cells after 12 Days in Culture. Fig. 22A depicts a plot of the frequency of Lin-CD34+ cells after 12 days of culture in the indicated compounds. Fig. 22B depicts a table of the statistical significance for compound expanded cells compared to DMSO. Statistics were generated using one-way ANOVA and Tukey's multiple comparisons tests. POM, pomalidomide; SR1, StemRegenin 1; A, A83-01; AP, A + POM; APU, A+POM+UM171; APSR1, A+POM+SR1.

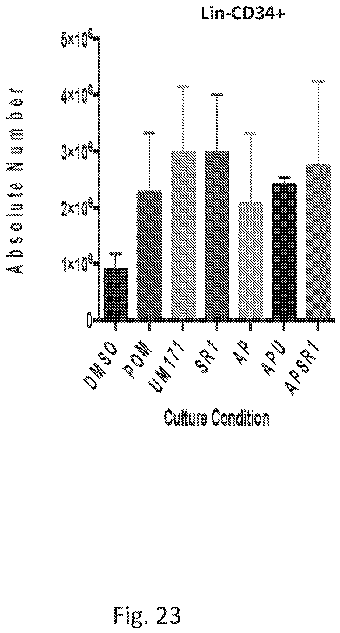

Fig. 23 depicts the absolute Number of Lin-CD34+ Cells after 12 Days in Culture. Plot shows the absolute number of Lin-CD34+ cells after 12 days of culture in the indicated compounds. No significant differences were detected using One-way ANOVA and Tukey's Multiple Comparisons. POM, pomalidomide; SR1, StemRegenin 1; A, A83-01; AP, A + POM; APU, A+POM+UM171; APSR1, A+POM+SR1.

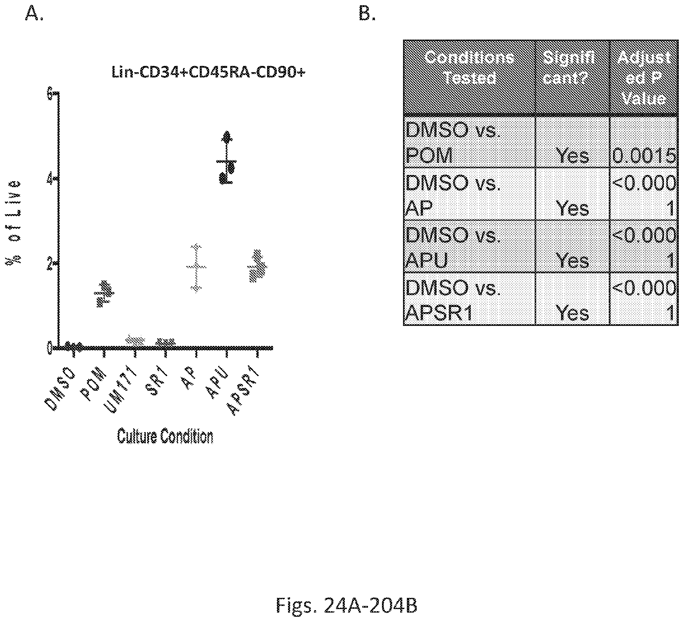

Figs. 24A-24B demonstrate the frequency of Lin-CD45RA-CD90+ HSCs after 12 Days in Culture. Fig. 24A depicts a graph of the frequency of immunophenotypic HSCs (Lin-CD34+CD45RA-CD90+) in each well after twelve days of culture in the indicated conditions. Fig. 24B depicts a table of statistical significance of compound expanded cells compared to DMSO expanded cells. Statistics are based on one-way ANOVA and Tukey's multiple comparisons. POM, pomalidomide; SR1, StemRegenin 1; A, A83-01; AP, A + POM; APU, A+POM+UM171; APSR1, A+POM+SR1.

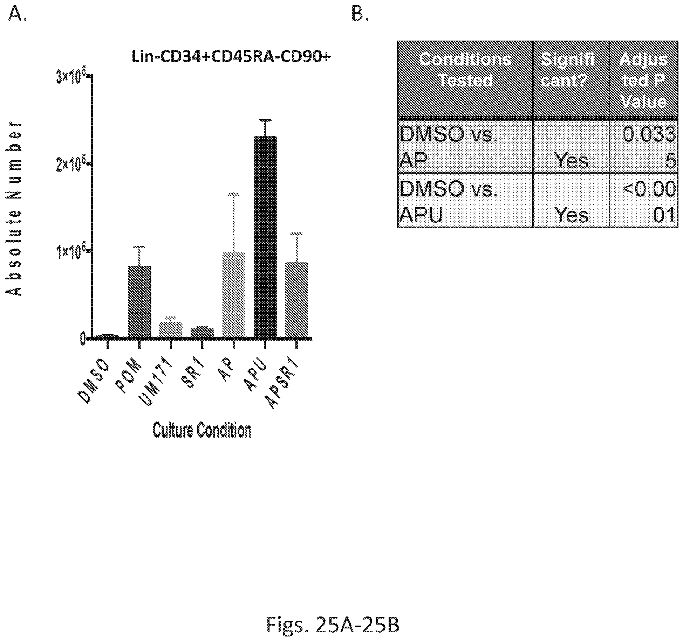

Figs. 25A-25B demonstrate the absolute Number of Lin-CD45RA-CD90+ HSCs after 12 Days in Culture. Fig. 25A depicts a graph of the absolute number of immunophenotypic HSCs (Lin-CD34+CD45RA-CD90+) in each well after twelve days of culture in the indicated conditions. Fig. 25B depicts a table of the statistical significance of compound expanded cells compared to DMSO expanded cells. Statistics are based on one-way ANOVA and Tukey's multiple comparisons. POM, pomalidomide; SR1, StemRegenin 1; A, A83-01; AP, A + POM; APU, A+POM+UM171; APSR1, A+POM+SR1.

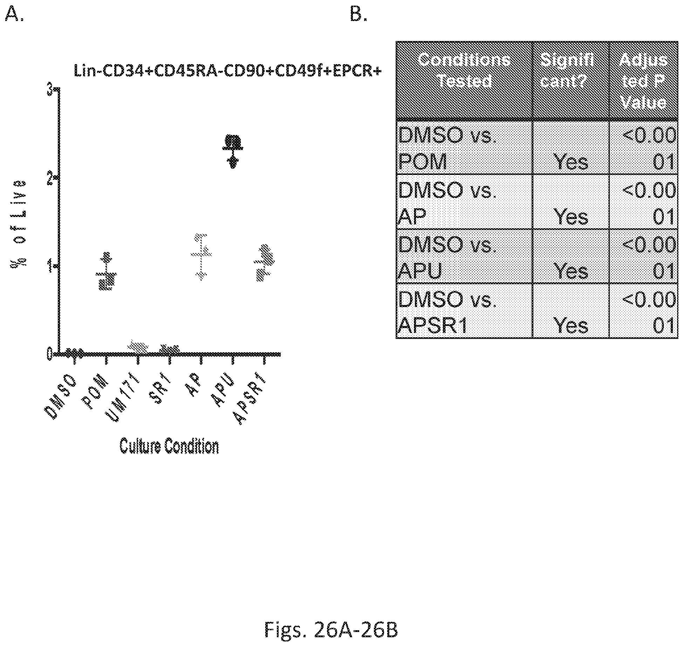

Figs. 26A-26B demonstrate the frequency of Lin-CD45RA-CD90+CD49f+EPCR+ HSCs after 12 Days in Culture. Fig. 26A depicts a graph of the frequency of Lin-CD34+CD45RA-CD90+CD49f+EPCR+ HSCs per well after twelve days of culture in the indicated conditions. Fig. 26B depicts a table of the statistical significance of compound expanded cells compared to DMSO expanded cells. Statistics are based on one-way ANOVA and Tukey's multiple comparisons. POM, pomalidomide; SR1, StemRegenin 1; A, A83-01; AP, A + POM; APU, A+POM+UM171; APSR1, A+POM+SR1.

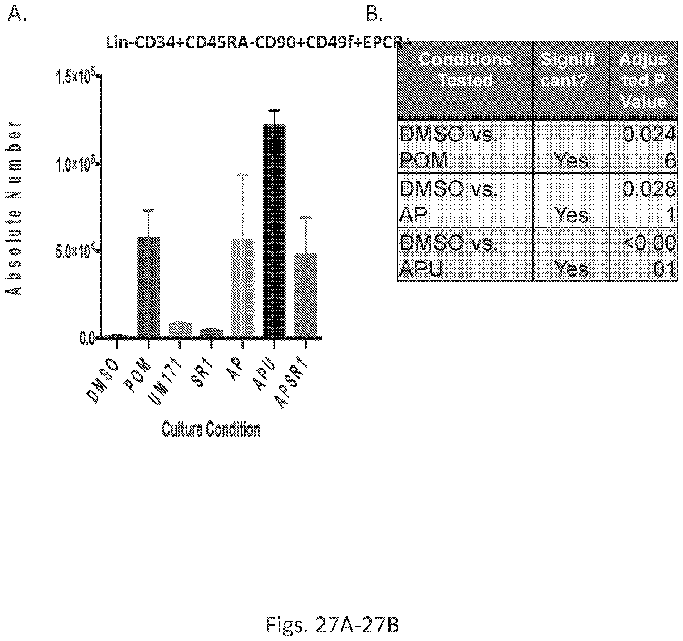

Figs. 27A-27B demonstrate the absolute number of Lin-CD45RA-CD90+CD49f+EPCR+ HSCs arising from CD34+ enriched CB after 12 Days in Culture. Fig. 27A depicts a graph of the absolute number of Lin-CD34+CD45RA-CD90+CD49f+EPCR+ HSCs per well after twelve days of culture in the indicated conditions. Fig. 27B depicts a table of the statistical significance of compound expanded cells compared to DMSO expanded cells. Statistics are based on one-way ANOVA and Tukey's multiple comparisons. POM, pomalidomide; SR1, StemRegenin 1; A, A83-01; AP, A + POM; APU, A+POM+UM171; APSR1, A+POM+SR1.

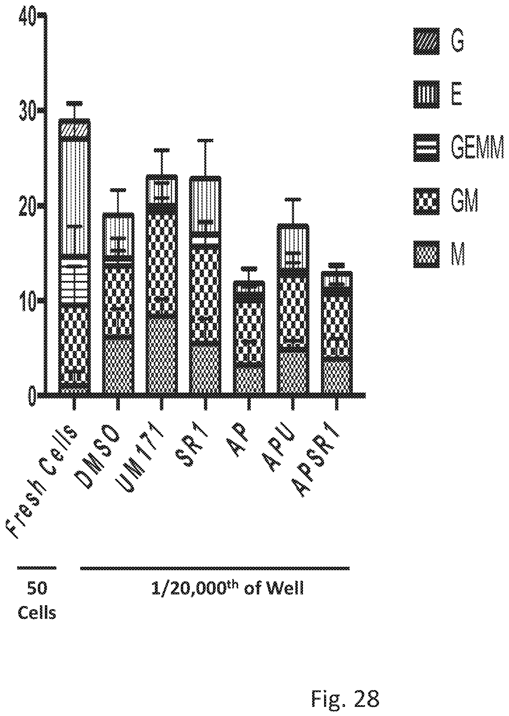

Fig. 28 demonstrates the colony forming potential of fresh CD34+ cord blood cells and cells cultured for 12 days in the presence of small moleculesOn Day 0, 50 fresh cells were plated in triplicate in methylcellulose media. Triplicate cultures were set up on three separate days. After twelve days of culturing CD34+ enriched cord blood cells in media containing cytokines and small molecules, 1/20,000th of the well was plated into methylcelulose media containing cytokines (Stem Cell Technologies). The total number of colonies in the lower panel is calculated at the number of colonies counted x 20,000 in order to obtain an estimate of the colony forming potential of the entire well. For all conditions, colonies were counted and scored on day 14 post-plating. POM, pomalidomide; SR1, StemRegenin 1; A, A83-01; AP, A + POM; APU, A+POM+UM171; APSR1, A+POM+SR1.

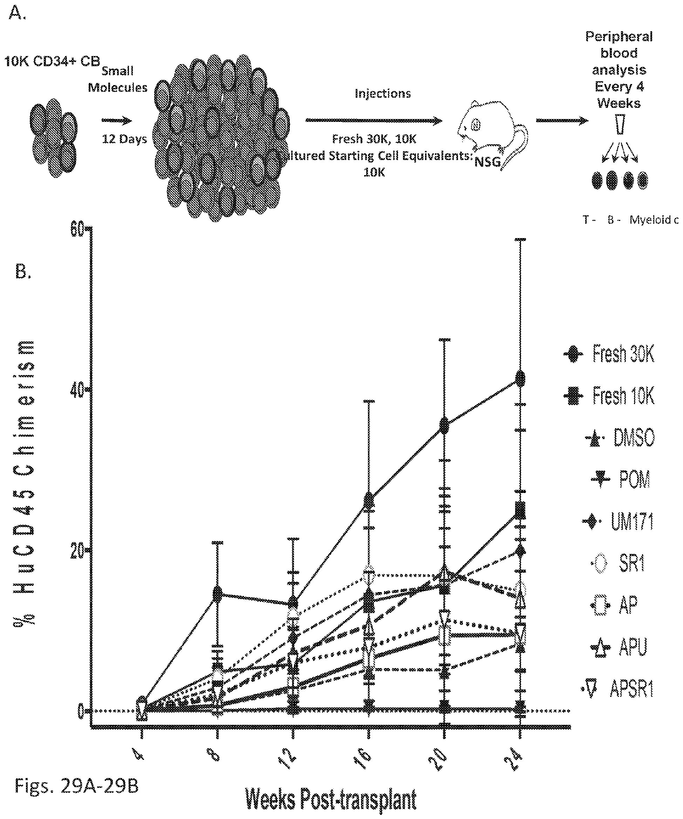

Figs. 29A-29B demonstrate a transplantation assay to measure HSC function for expanded human CD34+ cord blood cells. Fig. 29A depicts a schematic showing in vivo experimental design to test the function of fresh and expanded human CD34+ enriched cord blood cells. On day 0, (culture initiation), 30,000 or 10,000 CD34+ enriched cord blood cells were injected into sublethally irradiated NSG recipients or 10,000 cells were plated per well for expansion for 12 days in the indicated compounds. On day 12 after culture in the indicated compounds, 10,000 starting cell equivalents were injected into each sublethally irradiated NSG recipient mouse. Mice were bled every 4 weeks for 24 weeks, and engraftment was analyzed in the peripheral blood by flow cytometry for human CD45+ cells. Fig. 29B depicts human CD45+ chimerism in the peripheral blood of NSG mice at 4, 8, 12, 16, 20, and 24 weeks post-transplantatation for fresh and cultured cells. POM, pomalidomide; SR1, StemRegenin 1; A, A83-01; AP, A + POM; APU, A+POM+UM171; APSR1, A+POM+SR1.

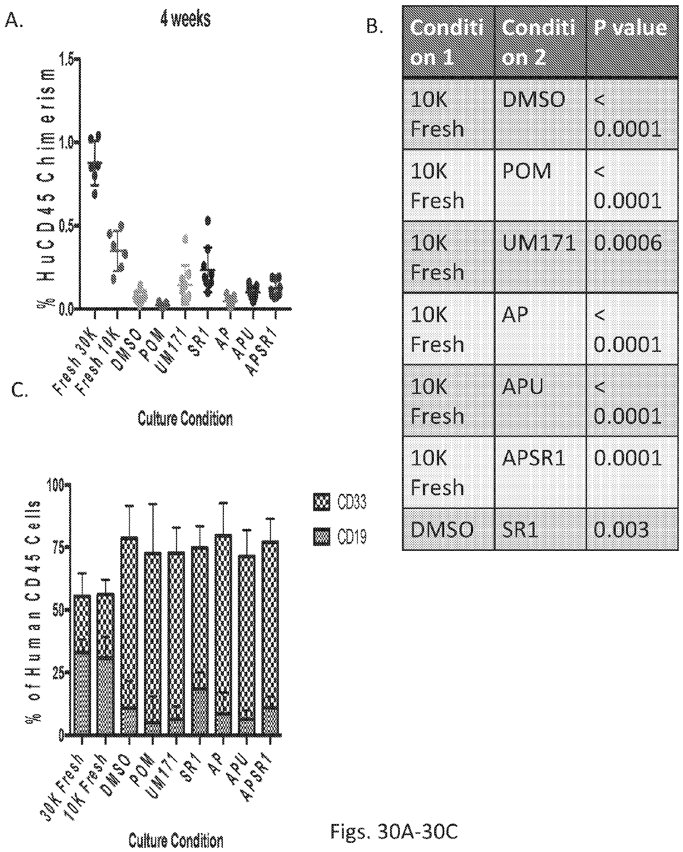

Figs. 30A-30C demonstrate peripheral blood analysis at 4 weeks post-transplantation. Fig. 30A depicts a plot demonstrating peripheral blood chimerism of human CD45+ cells at 4 weeks post-transplant. Fig. 30B depicts a table of statistically significant p values for data in Fig. 30A, based on one-way ANOVA and Tukey's multiple comparisons tests. Fig. 30B depicts the frequency of human CD45+ B cells (CD19+), T cells (CD3+) and myeloid cells (CD33+) in the peripheral blood at 4 weeks post-transplant. POM, pomalidomide; SR1, StemRegenin 1; A, A83-01; AP, A + POM; APU, A+POM+UM171; APSR1, A+POM+SR1.

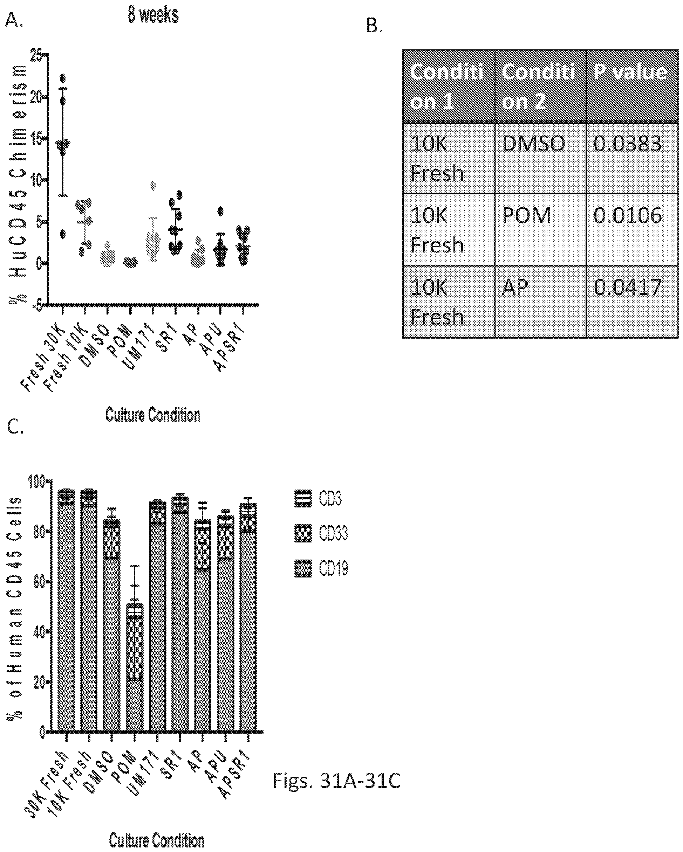

Figs. 31A-31B demonstrate peripheral blood analysis at 8 weeks post-transplantation. Fig. 31A is a plot demonstrating peripheral blood chimerism of human CD45+ cells at 8 weeks post-transplant. Fig. 31B depicts a table of statistically significant p values for data in Fig. 31A, based on one-way ANOVA and Tukey's multiple comparisons tests. Fig. 31C depicts the the frequency of human CD45+ B cells (CD19+), T cells (CD3+) and myeloid cells (CD33+) in the peripheral blood at 8 weeks post-transplant. POM, pomalidomide; SR1, StemRegenin 1; A, A83-01; AP, A + POM; APU, A+POM+UM171; APSR1, A+POM+SR1.

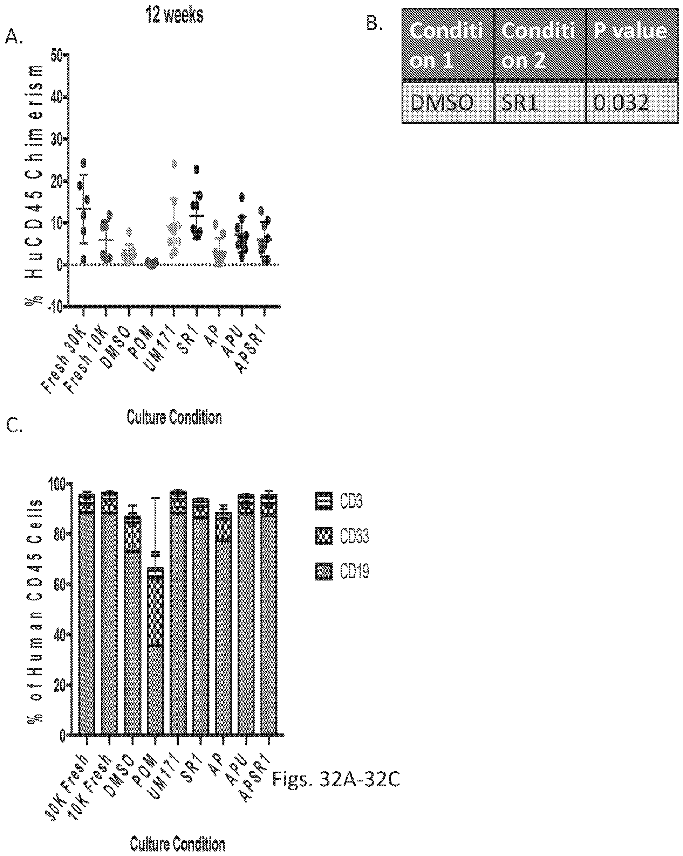

Figs. 32A-32C demonstrate peripheral blood analysis at 12 weeks post-transplantation. Fig. 32A depicts a plot of peripheral blood chimerism of human CD45+ cells at 12 weeks post-transplant. Fig. 32B depicts a table of statistically significant p values for data in Fig. 32A, based on one-way ANOVA and Tukey's multiple comparisons tests. Fig. 32C depicts frequency of human CD45+ B cells (CD19+), T cells (CD3+) and myeloid cells (CD33+) in the peripheral blood at 12 weeks post-transplant. POM, pomalidomide; SR1, StemRegenin 1; A, A83-01; AP, A + POM; APU, A+POM+UM171; APSR1, A+POM+SR1.

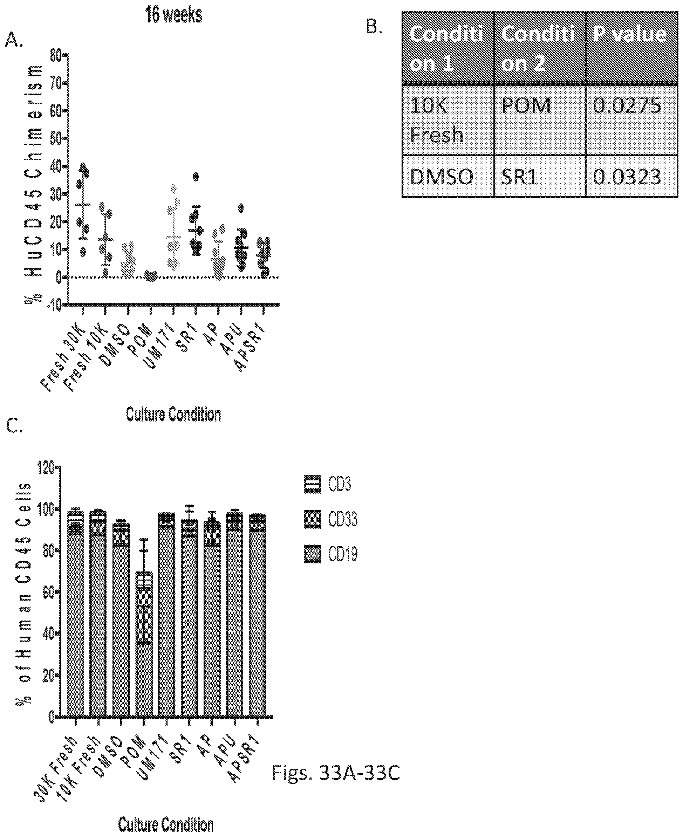

Figs. 33A-33C demonstrate that transplantation Assays indicate HSC Maintenance for AP, APU and APSR1 Expanded Cells at 16 weeks post-transplantation. Fig. 33A depicts peripheral blood chimerism of human CD45+ cells at 16 weeks post-transplant. Fig. 33B depicts a table of statistically significant p values for data in Fig. 33A, based on one-way ANOVA and Tukey's multiple comparisons tests. Fig. 33C depicts the frequency of human CD45+ B cells (CD19+), T cells (CD3+) and myeloid cells (CD33+) in the peripheral blood at 16 weeks post-transplant. POM, pomalidomide; SR1, StemRegenin 1; A, A83-01; AP, A + POM; APU, A+POM+UM171; APSR1, A+POM+SR1.

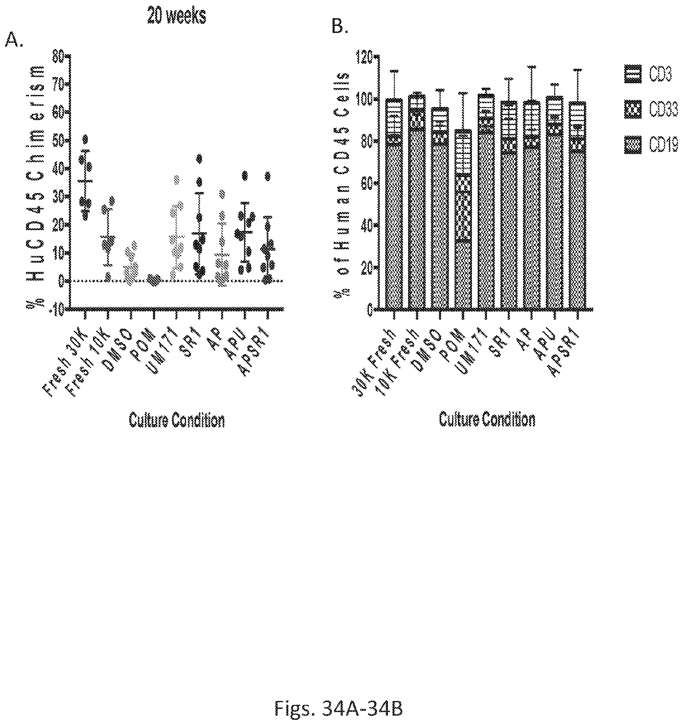

Figs. 34A-34B demonstrate that Transplantation Assays indicate HSC Maintenance for AP, APU and APSR1 Expanded Cells at 16 weeks post-transplantation. Fig. 34A depicts a plot of peripheral blood chimerism of human CD45+ cells at 20 weeks post-transplant. No statistical significance detected. No statistically significant differences detected using one-way ANOVA and Tukey's multiple comparisons tests. Fig. 34B depicts the frequency of human CD45+ B cells (CD19+), T cells (CD3+) and myeloid cells (CD33+) in the peripheral blood at 20 weeks post-transplant. POM, pomalidomide; SR1, StemRegenin 1; A, A83-01; AP, A + POM; APU, A+POM+UM171; APSR1, A+POM+SR1.

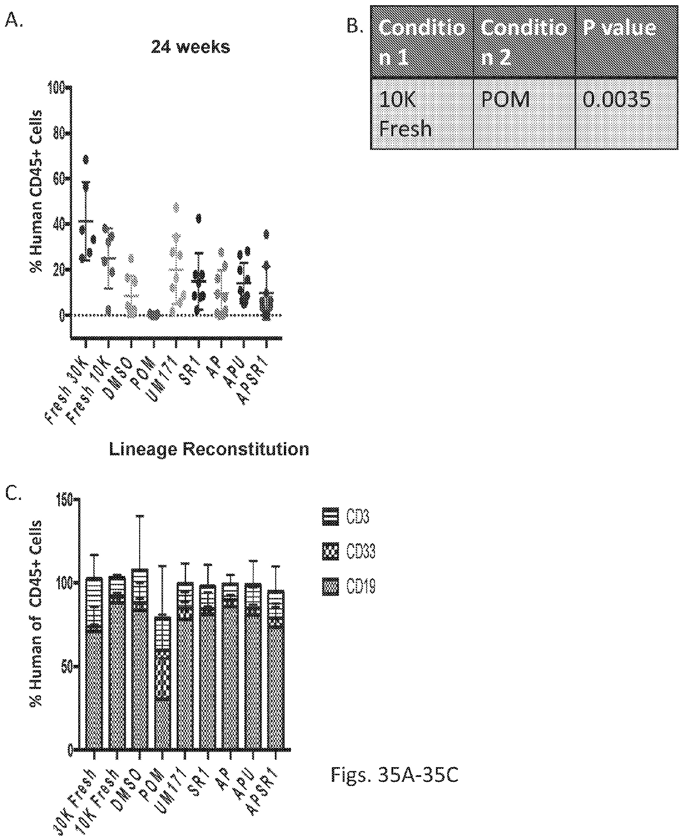

Figs. 35A-35C demonstrate that Transplantation Assays indicate HSC Maintenance for AP, APU and APSR1 Expanded Cells at 16 weeks post-transplantation. Fig. 35A depicts a plot of peripheral blood chimerism of human CD45+ cells at 24 weeks post-transplant. Fig. 35B depicts a table of statistically significant p values for data in Fig. 35A, based on one-way ANOVA and Tukey's multiple comparisons tests. Fig. 35C depicts the frequency of human CD45+ B cells (CD19+), T cells (CD3+) and myeloid cells (CD33+) in the peripheral blood at 24 weeks post-transplant. POM, pomalidomide; SR1, StemRegenin 1; A, A83-01; AP, A + POM; APU, A+POM+UM171; APSR1, A+POM+SR1.

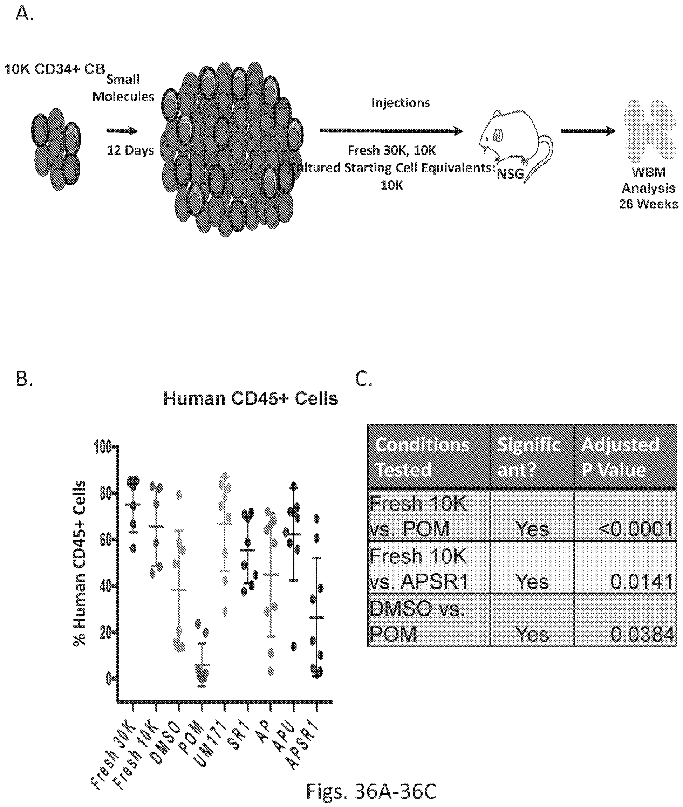

Figs. 36A-36C demonstrate bone marrow analysis of NSG mice transplanted with fresh or compound expanded human CD34+ cord blood cells. Fig. 36A depicts a schematic showing experimental design to assess human chimerism on the bone marrow following expansion of human CD34+ cord blood samples. On day 0 (culture initiation), 30,000 or 10,000 CD34+ enriched cord blood cells were injected into sublethally irradiated NSG recipients or 10,000 cells were plated per well to be expansed for 12 days in the indicated compounds. On day 12 after culture in the indicated compounds, 10,000 starting cell equivalents were injected into each sublethally irradiated NSG recipient mouse. Whole bone marrow analysis was carried out at 26 weeks post-transplant, and human engraftment was analyzed by flow cytometry. Fig. 36B depicts a plot of bone marrow chimerism of human CD45+ cells at 26 weeks post-transplant. Fig. 36C depicts a table of all statistically significantly p values for 10K fresh or DMSO cells versus compound expanded cells. Statistics were generated using one-way ANOVA and Tukey's multiple comparisons tests. POM, pomalidomide; SR1, StemRegenin 1; A, A83-01; AP, A + POM; APU, A+POM+UM171; APSR1, A+POM+SR1.

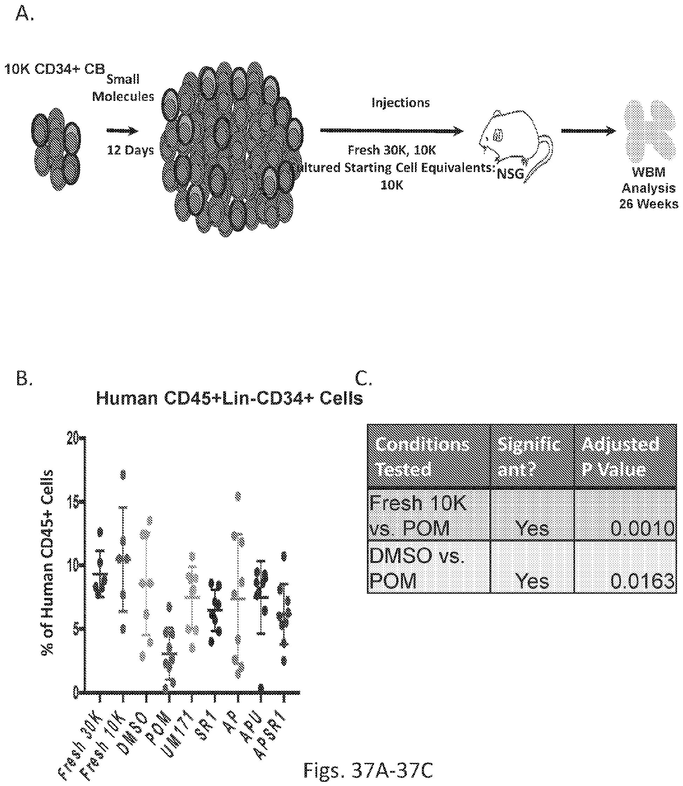

Figs. 37A-37C demonstrate Bone marrow analysis of NSG mice transplanted with fresh or compound expanded human CD34+ cord blood cells. Fig. 37A depicts a schematic showing experimental design of expanded human CD34+ cord blood samples. On day 0, culture initiation, 30,000 or 10,000 CD34+ enriched cord blood cells were injected into sublethally irradiated NSG recipients or 10,000 cells were cultured per well for 12 days in the indicated compounds. On day 12 after culture in the indicated compounds, 10,000 started cell equivalents were injected into each sublethally irradiated NSG recipient mouse. Whole bone marrow analysis was carried out at 26 weeks post-transplant, and human engraftment was analyzed by flow cytometry. Fig. 37B depicts a plot of bone marrow chimerism of human CD45+Lin-CD34+ HSPCs in the bone marrow at 26 weeks post-transplant. Fig. 37C depicts a table of all statistically significantly p values for 10K fresh or DMSO cells versus compound expanded cells. Statistics were generated using one-way ANOVA and Tukey's multiple comparisons tests. POM, pomalidomide; SR1, StemRegenin 1; A, A83-01; AP, A + POM; APU, A+POM+UM171; APSR1, A+POM+SR1.

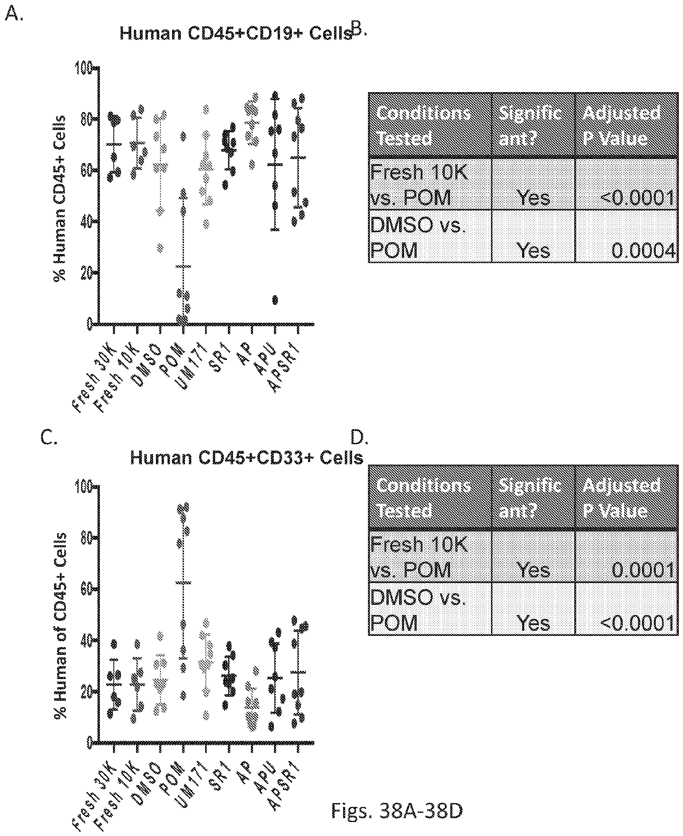

Figs. 38A-38D demonstrate bone marrow analysis for human B cell and myeloid cells from NSG mice transplanted with fresh or compound expanded human CD34+ cord blood cells. Fig. 38A depicts a plot of bone marrow chimerism of human CD45+CD19+ B cells at 26 weeks post-transplant. Fig. 38B depicts a table of all statistically significantly p values for 10K fresh or DMSO cells versus compound expanded cells in Fig. 38A . Statistics were generated using one-way ANOVA and Tukey's multiple comparisons tests. Fig. 38C depicts the frequency of human CD45+CD33+ myeloid cells in the bone marrow at 26 weeks post-transplant. Fig. 38D depicts all statistically significantly p values for 10K fresh or DMSO cells versus compound expanded cells in Fig. 38C. Statistics were generated using one-way ANOVA and Tukey's multiple comparisons tests. POM, pomalidomide; SR1, StemRegenin 1; A, A83-01; AP, A + POM; APU, A+POM+UM171; APSR1, A+POM+SR1.

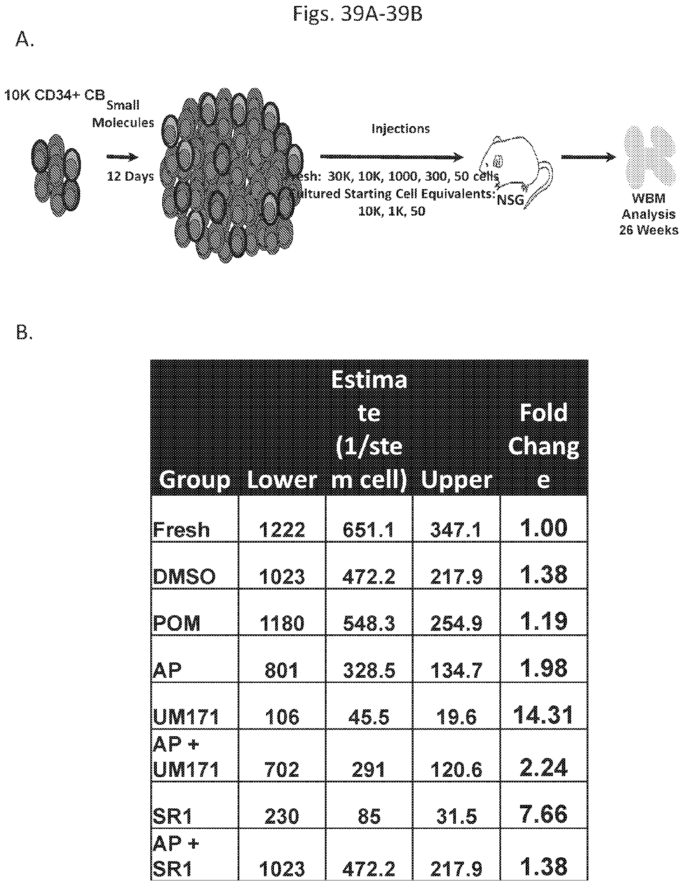

Figs. 39A-39B depict Limit Dilution analysis demonstrateing that AP expands functional human hematopoietic stem cells. Fig. 39A depicts a schematic showing experimental design of the limit dilution analysis of fresh and expanded human CD34+ cord blood samples. On day 0 (culture initiation), 30,000, 10,000, 1000, 300, or 50 fresh CD34+ enriched cord blood cells were injected into sublethally irradiated NSG recipients or 10,000 cells were cultured per well for 12 days in the indicated compounds. On day 12 after culture in the indicated compounds, 10,000, 1000, or 50 starting cell equivalents were injected into each sublethally irradiated NSG recipient mice. Whole bone marrow analysis was carried out at 26 weeks post-transplant, and human engraftment was analyzed by flow cytometry. Fig. 39B depicts a chart of the limit dilution analysis for these data, with 95% confidence intervals (lower and upper) as well as the fold change in functional expansion in each condition compared to fresh cells. POM, pomalidomide; SR1, StemRegenin 1; A, A83-01; AP, A + POM; APU, A+POM+UM171; APSR1, A+POM+SR1.

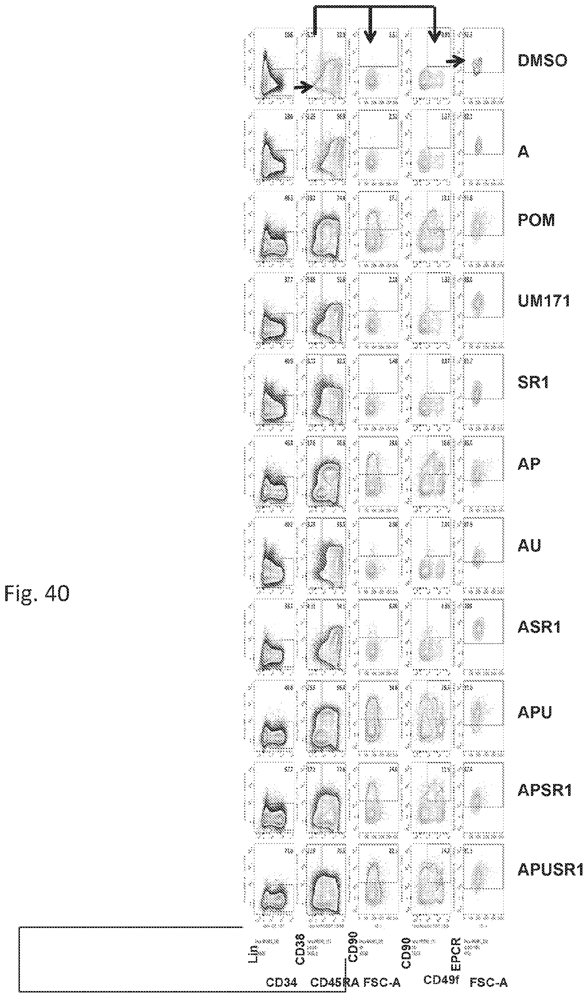

Fig. 40 depicts representative dot plots and gating strategy for expanded CD34+ cord blood cells. On day 12 after culture in the indicated compounds, samples were analyzed by flow cytometry. Plots show samples previously gated through live (PI-) and singlet gates. Gating strategy for immunophenotypic hematopoietic stem cells (HSCs) as Lin-CD34+CD45RA-CD90+ and Lin-CD34+CD45RA-CD90+CD49f+EPCR+ are shown. Percentages indicate the frequency of cells in the parent population. POM, pomalidomide; SR1, StemRegenin 1; A, A83-01; AP, A + POM; AU, A+UM171; ASR1; A+SR1; APU, A+POM+UM171; APSR1, A+POM+SR1; APUSR1, A+POM+UM171+SR1.

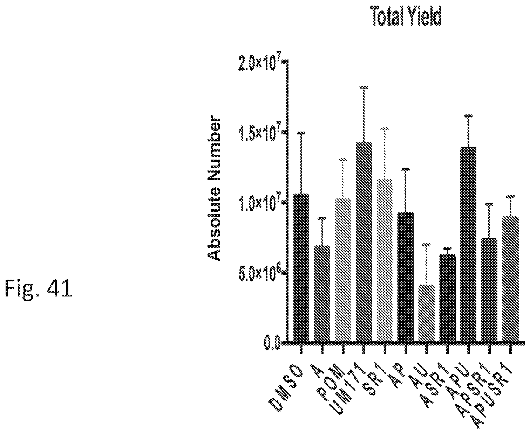

Fig. 41 depicts a graph of the absolute Number of cells after 12 Days in Culture. Depicted is the total number of live cells after 12 days of culturing 10,000 CD34+ enriched cord blood cells in the indicated conditions. No significant differences detected between DMSO and small molecule expanded cells based on one-way ANOVA and Tukey's multiple comparisons tests.

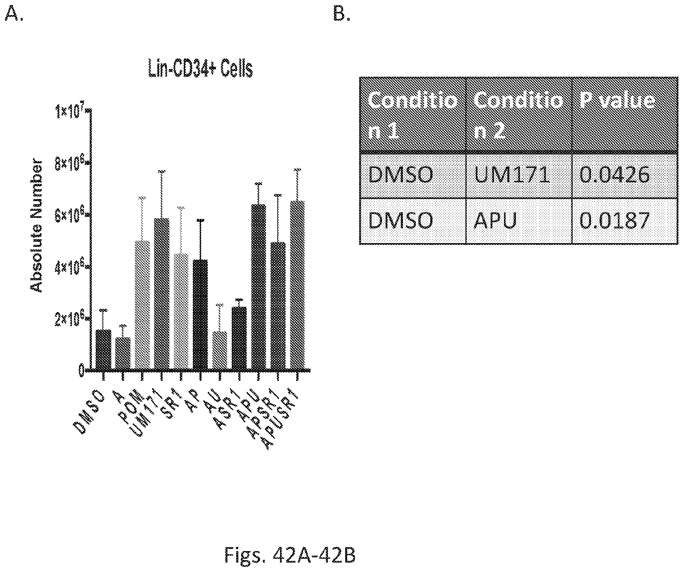

Figs. 42A-42B depict the absolute Number of Lin-CD34+ Cells after 12 Days in Culture. Fig. 42 depicts a plot of the absolute number of human Lineage-CD34+ HSPCs arising from 10,000 CD34+ enriched cord blood cells after 12 days of culture in the indicated media conditions. Fig. 42B depicts a table of significant p values for data in Fig. 42A, based on one-way ANOVA and Tukey's multiple comparisons tests.

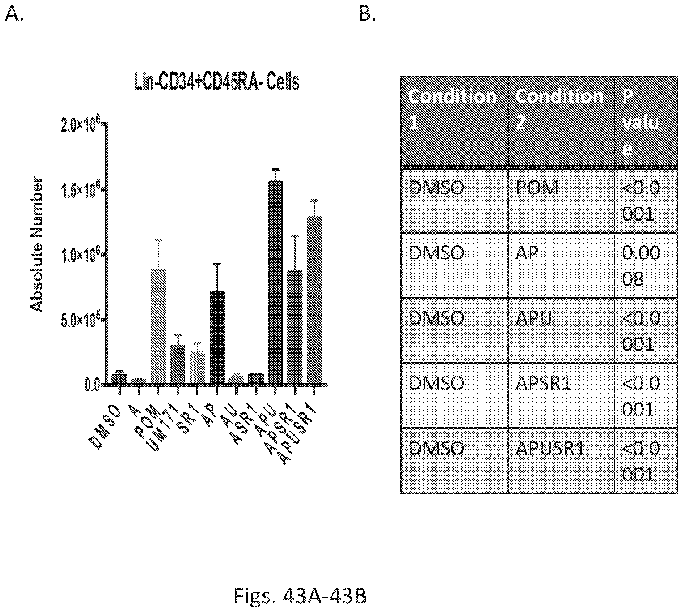

Figs. 43A-43B demonstrate the absolute number of Lin-CD34+CD45RA- progenitor cells After 12 days in culture. Fig. 43A depicts a plot of the absolute number of human Lineage-CD34+CD45RA- HSPCs arising after12 days of culturing 10,000 CD34+ enriched cord blood cells in the indicated media conditions. Fig. 43B depicts a table of statistically significant p values for data in Fig. 43A, based on one-way ANOVA and Tukey's multiple comparisons tests.

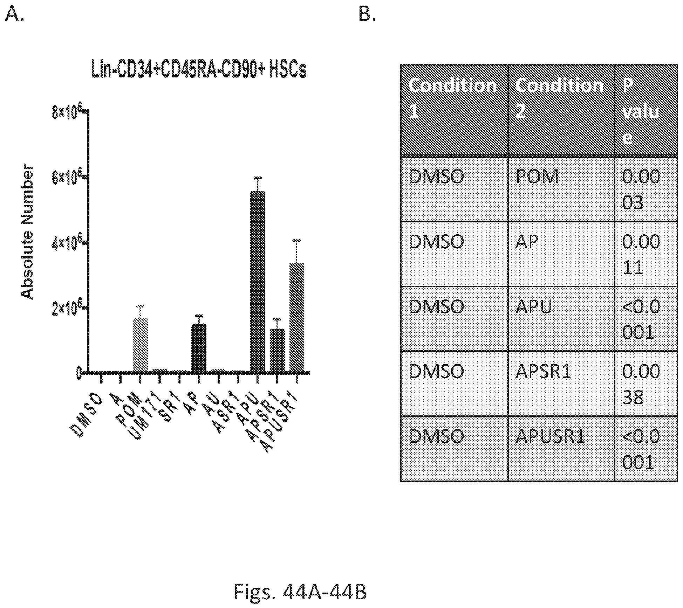

Figs. 44A-44B demonstrate the absolute Number of Lin-CD45RA-CD90+ HSCs after 12 Days in Culture. Fig. 44A depicts the absolute number of immunophenotypic HSCs (Lin-CD34+CD45RA-CD90+) arising from 10,0000 CD34+ enriched cord blood cells in each well after twelve days of culture in the indicated conditions. Fig. 44B depicts a table of all statistically significant p values for data in Fig. 44A, based on one-way ANOVA and Tukey's multiple comparisons tests.

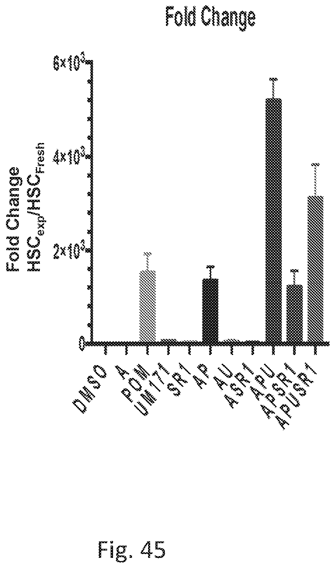

Fig. 45 depicts a graph of Fold Change for Lin-CD45RA-CD90+ HSCs after 12 Days in Culture. Graph shows the fold change in the absolute number of HSCs (Lin-CD34+CD45RA-CD90+) in cultured cells compared to fresh cells. HSC-exp, HSC-expansion condition.

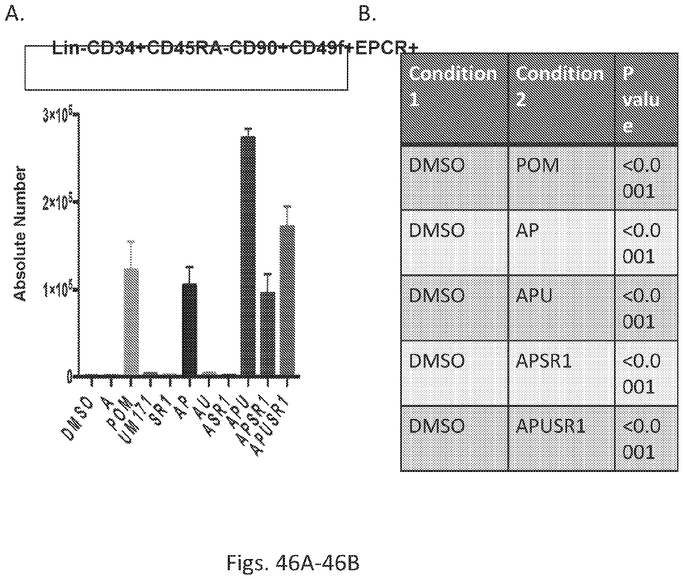

Figs. 46A-46B demonstrate the absolute number of Lin-CD45RA-CD90+CD49f+EPCR+ HSCs after 12 Days in Culture. Fig. 46A depicts the absolute number of immunophenotypic HSCs (Lin-CD34+CD45RA-CD90+CD49f+EPCR+) arising from 10,000 CD34+ enriched cord blood cells in each well after twelve days of culture in the indicated conditions. Fig. 46B depicts a table of statistically significant p values for DMSO versus compound expanded cells shown in Fig. 46A. Statistics are generated using one-way ANOVA and Tukey's multiple comparisons tests.

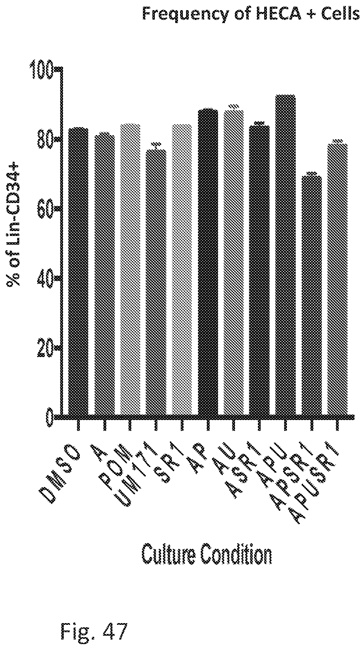

Fig. 47 depicts the frequency of Lineage-CD34+HECA+ cord blood cells. Frequency of human Lineage-CD34+ cells that are HECA+ on day 12 after culture in the indicated compounds. No statistically significant differences were detected using one-way ANOVA and Tukey's multiple comparisons tests.

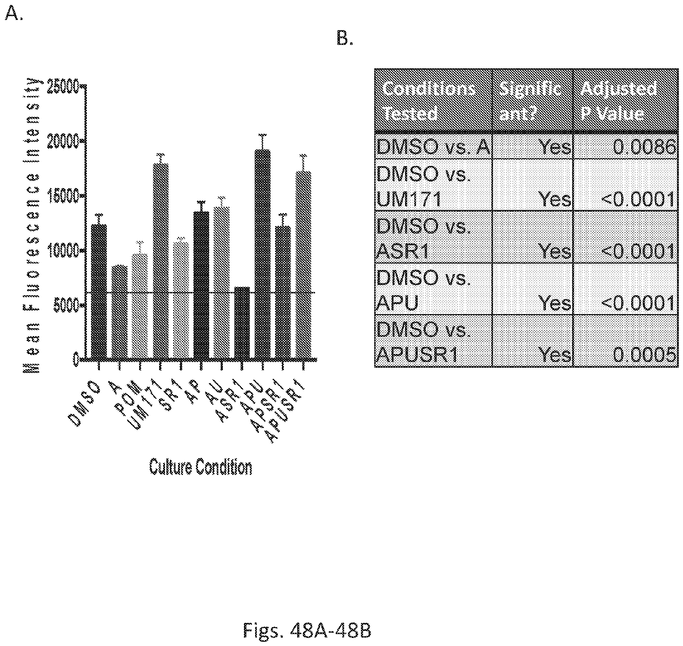

Figs. 48A-48B depict Mean Fluorescence Intensity of HECA staining on human Lineage-CD34+ cells. Data show HECA MFI staining on immunophenotypic Lin-CD34+ HSPCs. Using the antibody clone HECA-452, which recognizessialofucosylated glycans and sialyl Lewis x (sLex) on the cutaneus lymphocyte antigen. CLA binds E-selectin and has been shown to increase homing of cord blood progenitors to the bone marrow fowlloing transplantation. Fig. 48A depicts the mean flouresence intensity of HECA on the surface of Lin-34+ cells. Fig. 48B is a table of statistically significant p values for DMSO cultured cells compared to compound expanded cells. Statistics were generated using one-way ANOVA and Tukey's multiple comparisons tests.

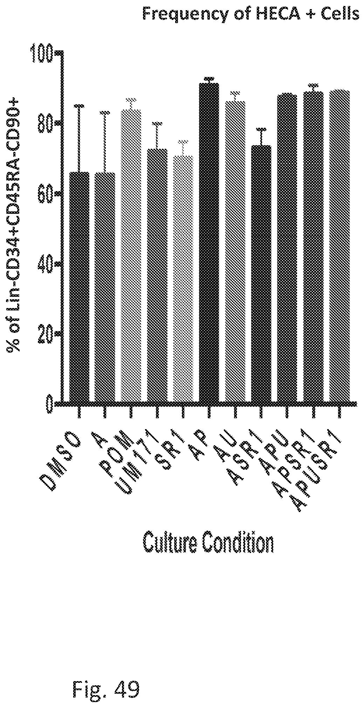

Fig. 49 depicts the frequency of Lineage-CD34+CD45RA-CD90+HECA+ HSCs. Graph shows the frequency of HECA+Lin-CD34+CD45RA-CD90+ HSCs after twelve days of culture in the indicated compounds. No statistically significant differences were detected using one-way ANOVA and Tukey's multiple comparisons tests.

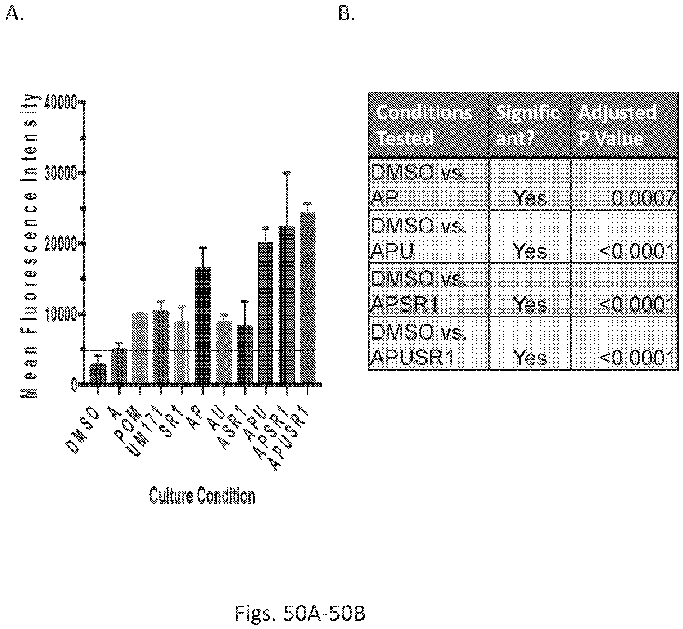

Figs. 50A-50B demonstrate significant Increase in HECA Expression on Lin-CD34+CD45RA-CD90+ HSCs from AP, APU, APSR1, APUSR1 expanded cells compared to DMSO expanded cells. Fig. 50A is a graph of the mean flouresence intensity of HECA on the surface of Lin-34+CD45RA-CD90+ cells. Fig. 50B is a table of statistically significant p values for DMSO cultured cells compared to compound expanded cells. Statistics were generated using one-way ANOVA and Tukey's multiple comparisons tests.

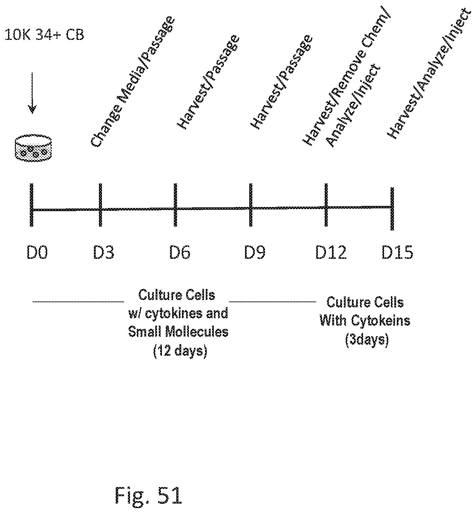

Fig. 51 depicts a schematic experimental design for compound withdrawal following expansion of CD34+ enriched cord blood cells. The schematic represents the culture protocol for the withdrawal of small molecules. Briefly, human CD34+ enriched cells from cord blood were obtained from AllCells. On D0, 10,000 fresh CD34+ cord blood cells were injected into sublethally irradiated NSG recipient mice or ten thousand cells were cultured in StemSpan SFEMII containing recombinant human SCF, TPO, IL-3 and FLT3L (all at 100 ng/ml) and vehicle control (DMSO) or the indicated small molecules. Media was changed every three days. On day twelve were harvested and washed. Half of the cells were used for counting, analysis, and for the injection of 10,000 starting cell equivalents into sublethally irradiated NSG recipient mice. The other half of the cells (10,000 starting cell equivalents/erll), were resuspended into StemSpan SFEM II media supplemented with cytokines (no small molecules or DMSO) and cultured for an additional 3 days. On Day 15, the replated cells were harvested, counted, analyzed by flow cytometry, and 10,0000s starting cell equivalents were injected into sublethally irradiated NSG mice.

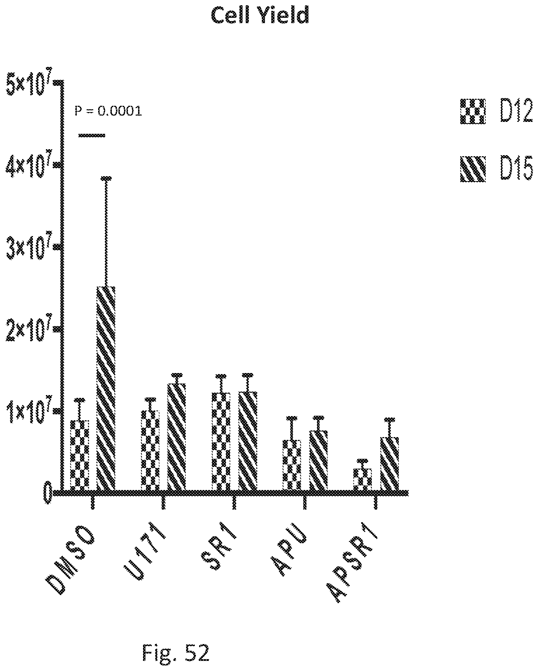

Fig. 52 depicts total live cells generated following expansion with small molecules (D12) or following removal of small molecules (D15). CD34+ cells from cord blood were obtained from AllCells. Ten thousand cells were cultured in StemSpan SFEMII containing recombinant human SCF, TPO, IL-3 and FLT3L (all at 100 ng/ml). Cells were cultured with media containing the vehicle control (DMSO) or the indicated small molecules. Media was changed every three days. On day twelve, cells harvested, counted, and analyzed by flow cytometry. Half of the cells were replated in media containing cytokines alone (no compounds) and allowed to expand for another three days. On day 15, the remaining cells were harvested, counted, and analyzed by flow cytometry. The absolute number of live cells after 12 days of culture is shown in the graph. POM, pomalidomide; SR1, StemRegenin 1; A, A83-01; APU, A+POM+UM171; APSR1, A+POM+SR1. Statistics were generated using two-way ANOVA and Tukey's multiple comparisons tests.

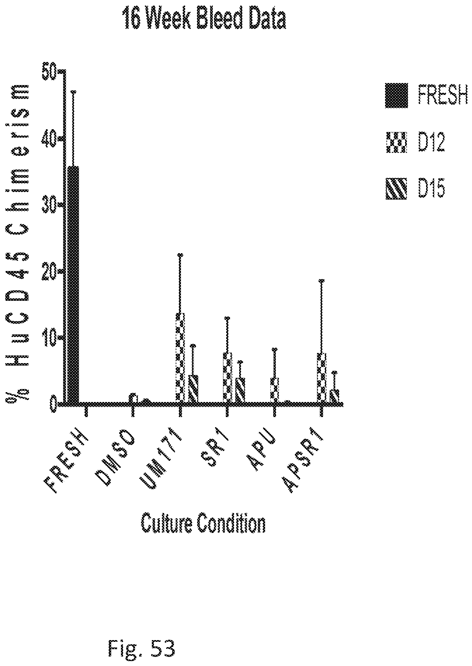

Fig. 53 depicts human CD45+ peripheral blood chimerism in NSG mice transplanted on Day 12 (compound expanded) or Day 15 (compound lift). The peripheral blood chimerism of human CD45+ cells is shown at 16 weeks post-transplant for fresh cells, cells transplanted on D12 of culture (expansion) or D15 of culture (following compound withdrawal). POM, pomalidomide; SR1, StemRegenin 1; A, A83-01; APU, A+POM+UM171; APSR1, A+POM+SR1.

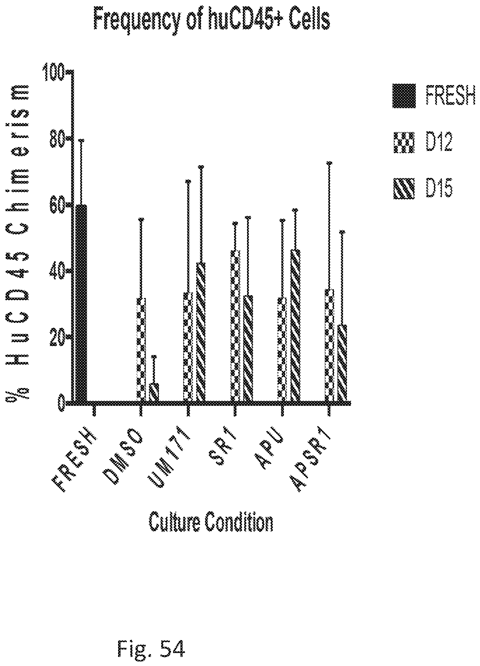

Fig. 54 depicts human CD45+ cells in the bone marrow of NSG mice transplanted on Day 12 (compound expanded) or Day 15 (compound withd).CD34+ cells from cord blood were obtained from AllCells. Ten thousand cells were cultured in StemSpan SFEMII containing recombinant human SCF, TPO, IL-3 and FLT3L (all at 100 ng/ml). Cells were cultured with media containing the vehicle control (DMSO) or the indicated small molecules. Media was changed every three days. On day twelve, cells harvested and half of the cells were replated in media containing cytokines alone (no compounds) and allowed to expand for another three days. On day 15, the remaining cells were harvested, counted, and analyzed by flow cytometry. Injections were carried out on Day 1 (10,000 fresh CD34+ CB cells), Day 12 (10,000 starting cell equivalents per mouse), and Day 15 (10,0000 starting cell equivalents, compound lift condition). The bone marrow chimerism of human CD45+ cells is shown at 18 weeks post-transplant.. POM, pomalidomide; SR1, StemRegenin 1; A, A83-01; APU, A+POM+UM171; APSR1, A+POM+SR1.

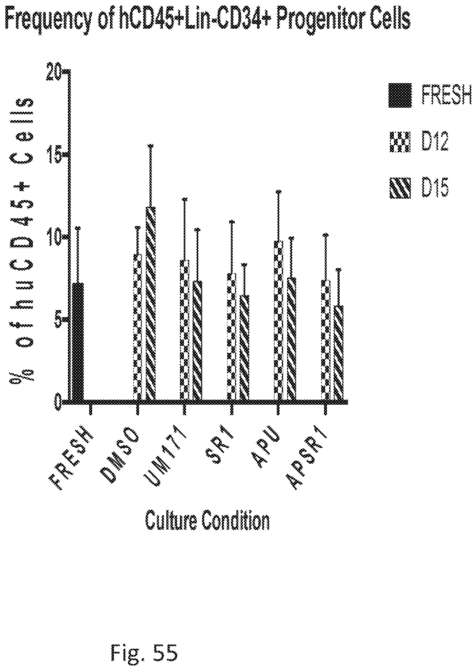

Fig. 55 depicts human CD45+CD34+ HSPCs in the bone marrow of NSG mice transplanted on Day 12 (compound expanded) or Day 15 (compound lift). The bone marrow chimerism of human CD45+Lineage-CD34+ HSCPs is shown at 18 weeks post-transplant. POM, pomalidomide; SR1, StemRegenin 1; A, A83-01; APU, A+POM+UM171; APSR1, A+POM+SR1.

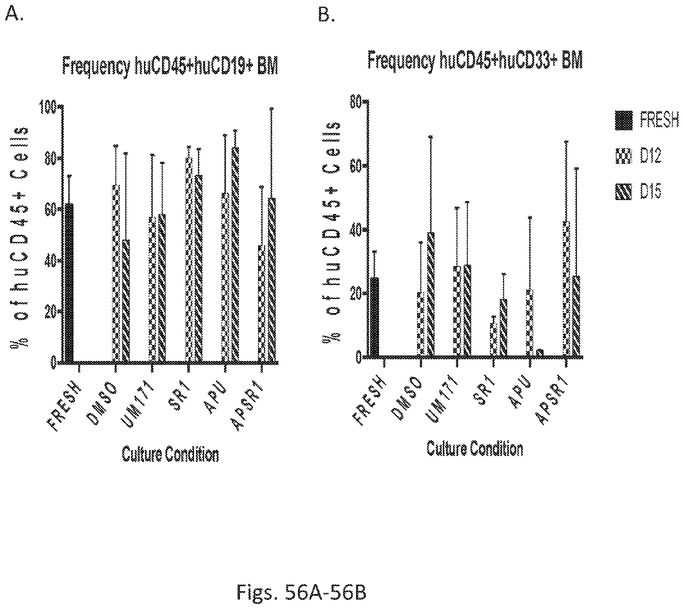

Figs. 56A-56B demonstrate Human CD45+CD19+ B cells and CD45+CD33+ myeloid cells in the bone marrow of NSG mice transplanted on Day 12 (compound expanded) or Day 15 (compound lift). Graphs depict the bone marrow chimerism of human CD45+CD19+ B cells (Fig. 56A) and human CD45+CD33+ myeloid cells (Fig. 56B) at 18 weeks post-transplant.. POM, pomalidomide; SR1, StemRegenin 1; A, A83-01; APU, A+POM+UM171; APSR1, A+POM+SR1.

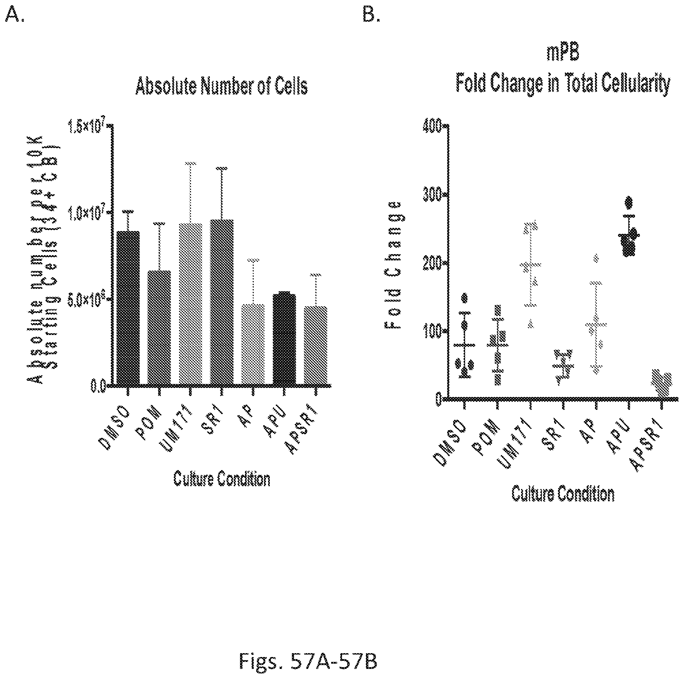

Figs. 57A-57B demonstrate absolute number and fold change in total cellularity of CD34+ mobilized peripheral blooc cells after expansion for 12 days with small molecules. CD34+ enriched human mobilized peripheral blood were obtained from AllCells. Ten thousand cells were cultured in StemSpan SFEMII containing recombinant human SCF, TPO, IL-3 and FLT3L (all at 100 ng/ml). Cells were cultured with media containing the vehicle control (DMSO) or the indicated small molecules. Media was changed every three days. On day twelve, cells harvested, counted, and analyzed by flow cytometry. Fig. 57A depicts a graph of absolute number of live cells after 12 days of culture. No statistically significant differences were detected using one-way ANOVA and Tukey's multiple comparisons tests. Fig. 57B depicts fold change in total cells compared for expanded cells compared to 10,000 starting cells. POM, pomalidomide; SR1, StemRegenin 1; A, A83-01; AP, A + POM; APU, A+POM+UM171; APSR1, A+POM+SR1.

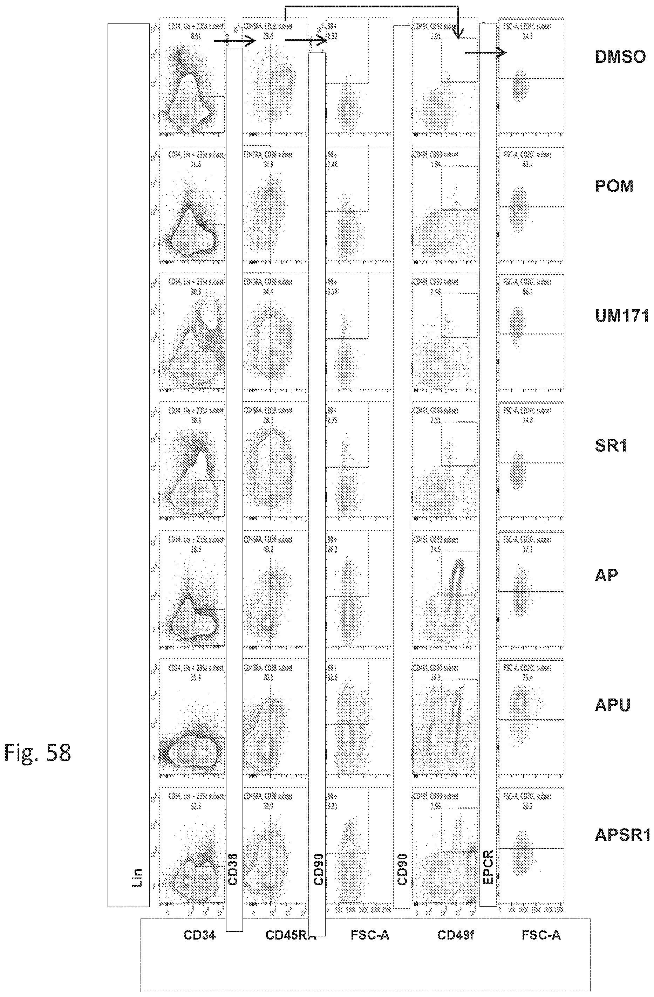

Fig. 58 depicts a representative Dot Plots and Gating Strategy for expanded CD34+ mobilized peripheral blood (mPB) HSCs. On day 12 after culture in the indicated compounds, samples were analyzed by flow cytometry. Plots show samples previously gated through live (PI-) and singlet gates. Gating strategies for immunophenotypic hematopoietic stem cells (HSCs) as Lin-CD34+CD45RA-CD90+ and Lin-CD34+CD45RA-CD90+CD49f+EPCR+ HSCs are shown. Percentages indicate the frequency of cells in the parent population.

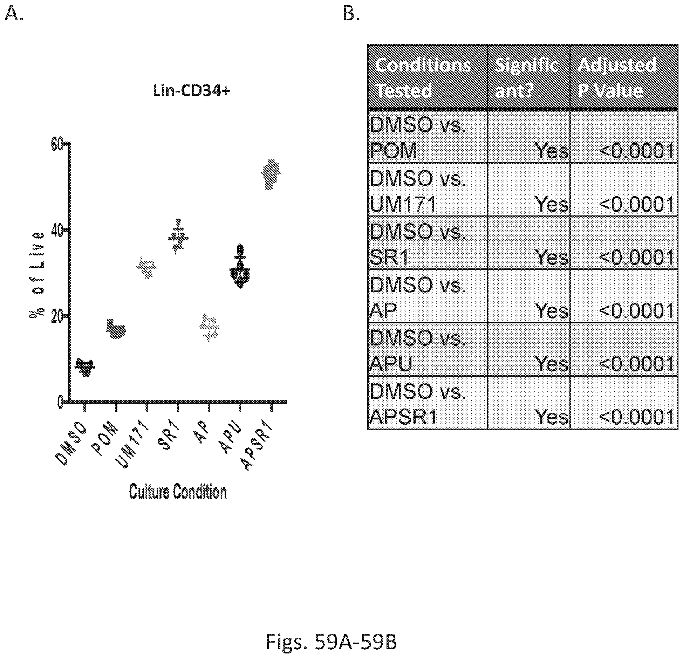

Figs. 59A-59B demonstrate the frequency of Lin-CD34+ Cells after CD34+ enriched mPB cells were expanded for 12 Days. Fig. 59A depicts a plot of the frequency of Lin-CD34+ cells arising from 10,000 CD34+ enriched mPB cells after 12 days of culture in the indicated compounds. Fig. 59B depicts a table of the statistically significant p values of compound expanded cells compared to DMSO. Statistics were generated using one-way ANOVA and Tukey's multiple comparisons tests.

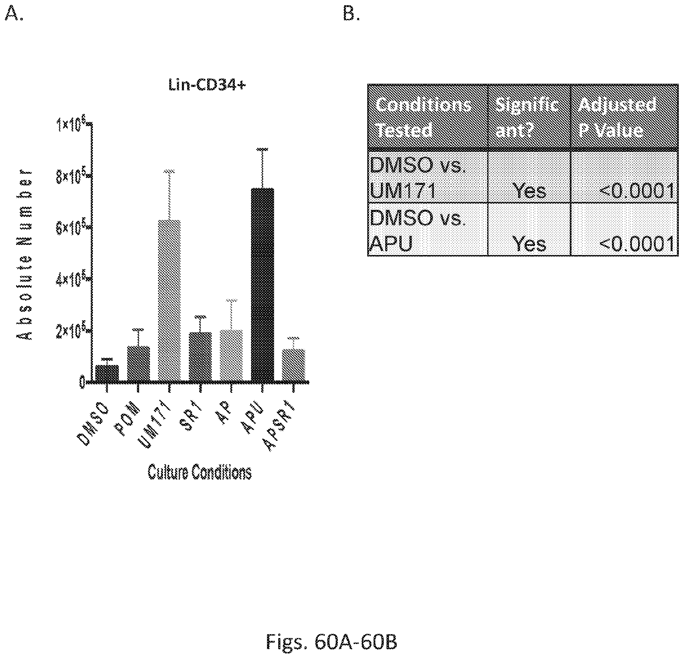

Figs. 60A-60B demonstrate the absolute number of Lin-CD34+ Cells after CD34+ enriched mPB cells were expanded for 12 Days. Plots show the (Fig. 60A) absolute number of Lin-CD34+ cells after 12 days of culture in the indicated compounds. Fig. 60B depicts the statistically significant p values of compound expanded cells compared to DMSO. Statistics were generated using one-way ANOVA and Tukey's multiple comparisons tests.

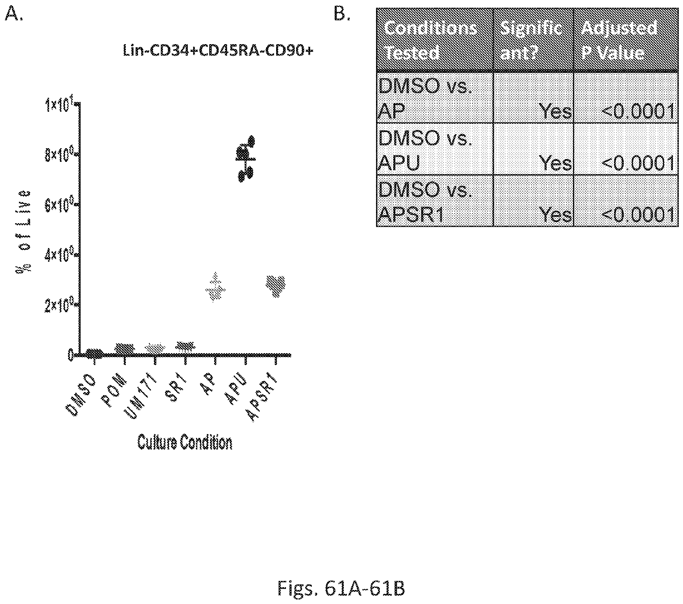

Figs. 61A-61B demonstrate the frequency of Lin-CD34+CD45RA-CD90+ HSCs after CD34+ mPB cells were cultured for 12 Days. Fig. 61A depicts the frequency of immunophenotypic HSCs (Lin-CD34+CD45RA-CD90+) arising from 10,000 CD34+ enriched mPB cells in each well after twelve days of culture in the indicated conditions. Fig. 61B depicts the statistically significant p values of compound expanded cells compared to DMSO. Statistics were generated using one-way ANOVA and Tukey's multiple comparisons tests.

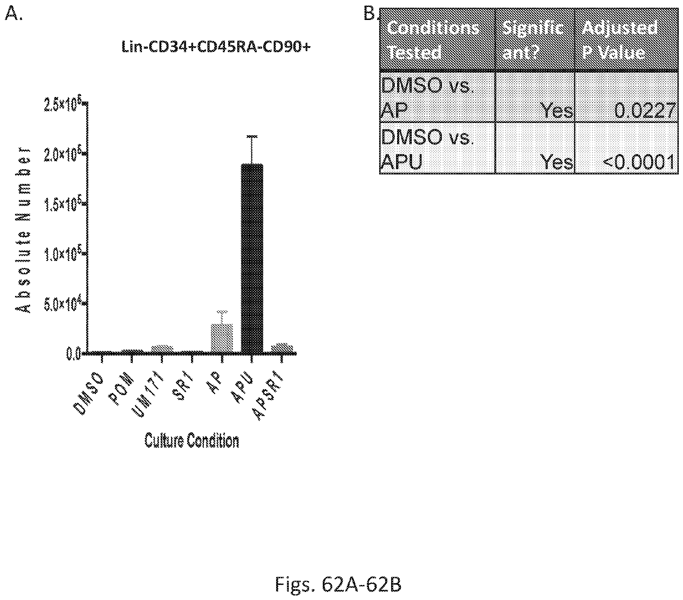

Figs. 62A-62B demonstrate the absolute number of Lin-CD34+CD45RA-CD90+ HSCs after CD34+ enriched mPB cells were cultured for 12 Days. Fig. 62A depicts a plot of absolute number of immunophenotypic HSCs (Lin-CD34+CD45RA-CD90+) arising from 10,000 CD34+ enriched mPB cells in each well after twelve days of culture in the indicated conditions. Fig. 62B depicts the statistically significant p values of compound expanded cells compared to DMSO. Statistics were generated using one-way ANOVA and Tukey's multiple comparisons tests.

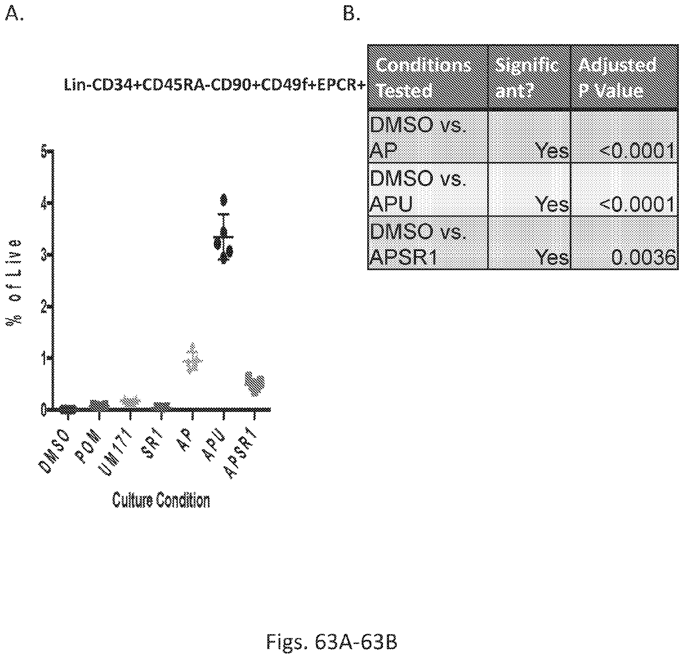

Figs. 63A-63B demonstrate the frequency of Lin-CD34+CD45RA-CD90+CD49f+EPCR+ HSCs after CD34+ enriched mPB cells were expanded for 12 Days. Fig. 63A depicts the frequency of immunophenotypic HSCs (Lin-CD34+CD45RA-CD90+CD49f+EPCR+) arising from 10,000 CD34+ enriched mPB cells in each well after twelve days of culture in the indicated conditions. Fig. 63B depicts a table of the statistically significant p values of compound expanded cells compared to DMSO. Statistics were generated using one-way ANOVA and Tukey's multiple comparisons tests.

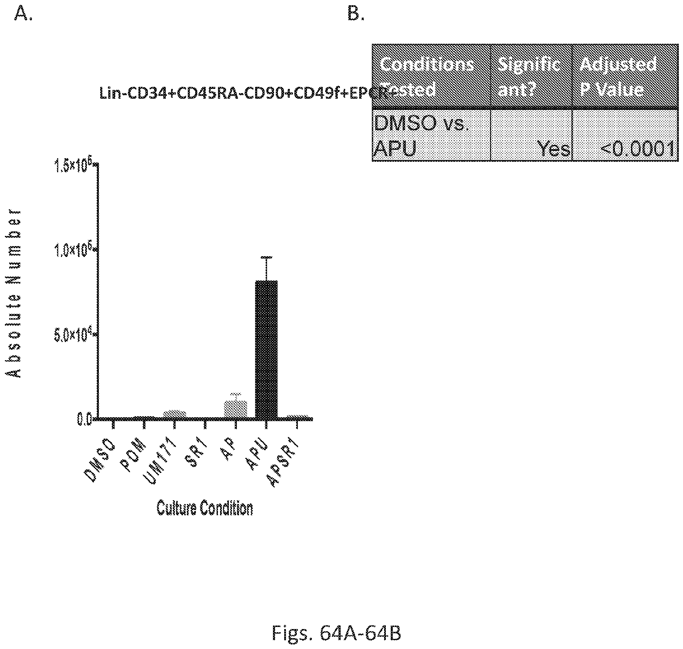

Figs. 64A-64B demonstrate the absolute number of Lin-CD34+CD45RA-CD90+CD49f+EPCR+ HSCs after CD34+ mPB cells were cultured for 12 Days. Figs. 64A depicts the absolute number of immunophenotypic HSCs (Lin-CD34+CD45RA-CD90+CD49f+EPCR+) arising from 10,000 CD34+ enriched mPB cells in each well after twelve days of culture in the indicated conditions. Fig. 64B depicts a table of the statistically significant p values of compound expanded cells compared to DMSO. Statistics were generated using one-way ANOVA and Tukey's multiple comparisons tests.

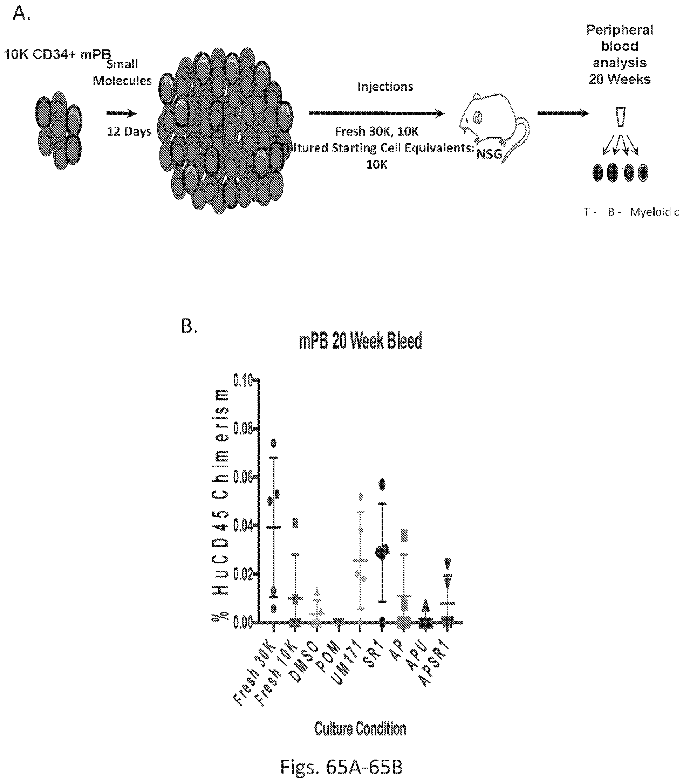

Figs. 65A-65B demonstrate a transplantation assay for expanded human CD34+ mobilized peripheral blood cells. Fig. 65A depicts a schematic showing experimental design for in vivo analysis of expanded human CD34+ mobilized peripheral blood cells. On day 0 (culture initiation), 30,000 or 10,000 CD34+ enriched mobilized peripheral blood cells were injected into sublethally irradiated NSG recipients or 10,000 cells were plated per well for expansion for 12 days in the indicated compounds. On day 12 after culture in the indicated compounds, 10,000 starting cell equivalents were injected into each sublethally irradiated NSG recipient mouse. Mice were bled every 4 weeks for 20 weeks, and engraftment was analyzed by flow cytometry. Fig. 65B depicts a plots of peripheral blood chimerism of human CD45+ cells at 20 weeks post-transplant. No significant differences were detected between 10K fresh or DMSO and compound cultured cells using one-way ANOVA and Tukey's multiple comparisons test.

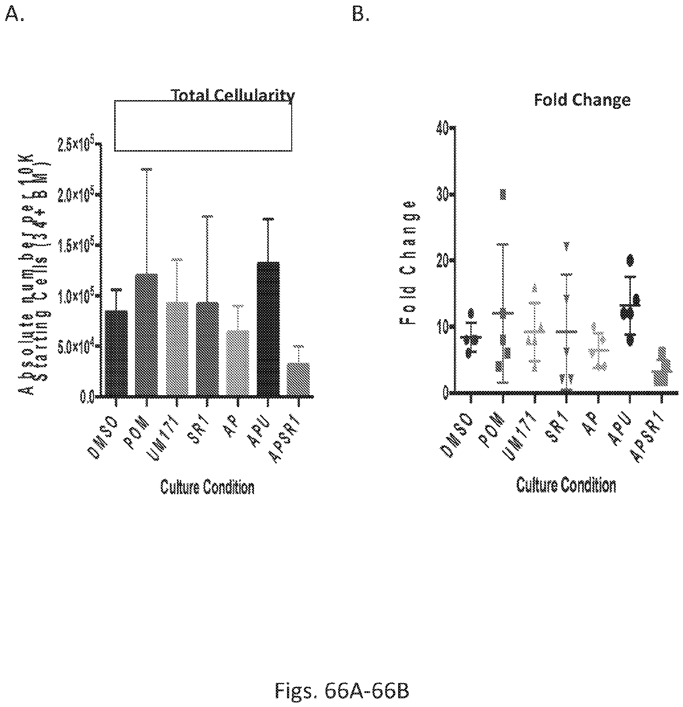

Figs. 66A-66B demonstrate the absolute number and fold change in total cellularity of CD34+ enriched bone marrow cells after expansion for 12 days with small molecules. CD34+ enriched bone marrow cells were obtained from AllCells. Ten thousand cells were cultured in StemSpan SFEMII containing recombinant human SCF, TPO, IL-3 and FLT3L (all at 100 ng/ml). Cells were cultured with media containing the vehicle control (DMSO) or the indicated small molecules. Media was changed every three days. On day twelve, cells were harvested, counted, and analyzed by flow cytometry. Fig. 66A depicts a graph of absolute number of live cells after 12 days of culture. No statistically significant differences were detected between DMSO and compound expanded cells using one-way ANOVA and Tukey's multiple comparisons tests. Fig. 66B depicts the fold expansion in total live cells compared to 10,000 starting cells in each condition. POM, pomalidomide; SR1, StemRegenin 1; A, A83-01; AP, A + POM; APU, A+POM+UM171; APSR1, A+POM+SR1.

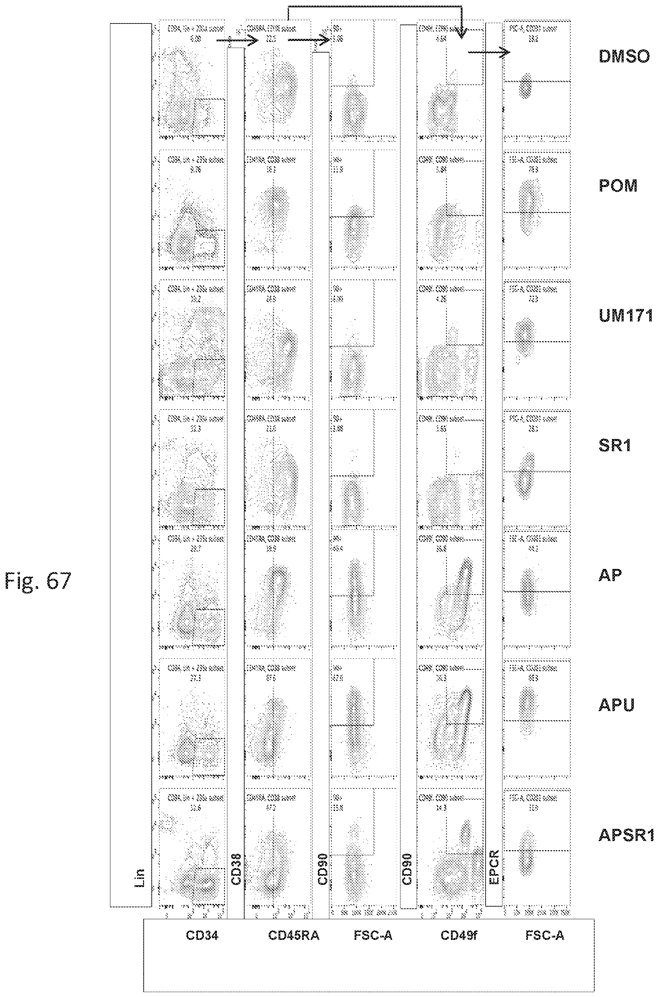

Fig. 67 depicts representative Dot Plots and Gating Strategy for expanded CD34+ mobilized peripheral blood (mPB) HSCs. On day 12 after culture in the indicated compounds, samples were analyzed by flow cytometry. Plots show samples previously gated through live (PI-) and singlet gates. Gating strategies for immunophenotypic hematopoietic stem cells (HSCs) definied as Lin-CD34+CD45RA-CD90+ and Lin-CD34+CD45RA-CD90+CD49f+EPCR+ HSCs are shown. Percentages indicate the frequency of cells in the parent population. POM, pomalidomide; SR1, StemRegenin 1; A, A83-01; AP, A + POM; APU, A+POM+UM171; APSR1, A+POM+SR1.

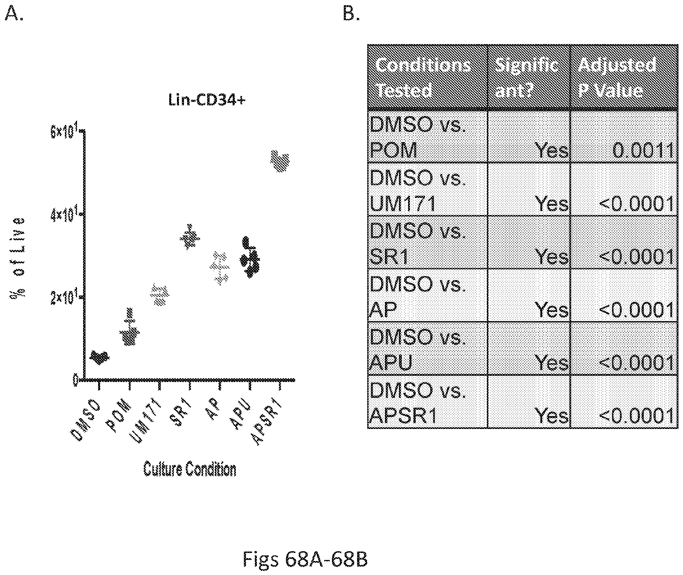

Figs. 68A-68B demonstrate the frequency of Lin-CD34+ Cells after CD34+ enriched bone marrow cells were expanded for 12 days with small molecules. Fig. 68A depicts a graph of the frequency of and of Lin-CD34+ cells arising from 10,000 CD34+ enriched bone marrow cells after 12 days of culture in the indicated compounds. Fig. 68B is a table of the statistically significant p values for compound expanded cells compared to DMSO expanded cells. Statistics were generated using one-way ANOVA and Tukey's multiple comparisons tests.

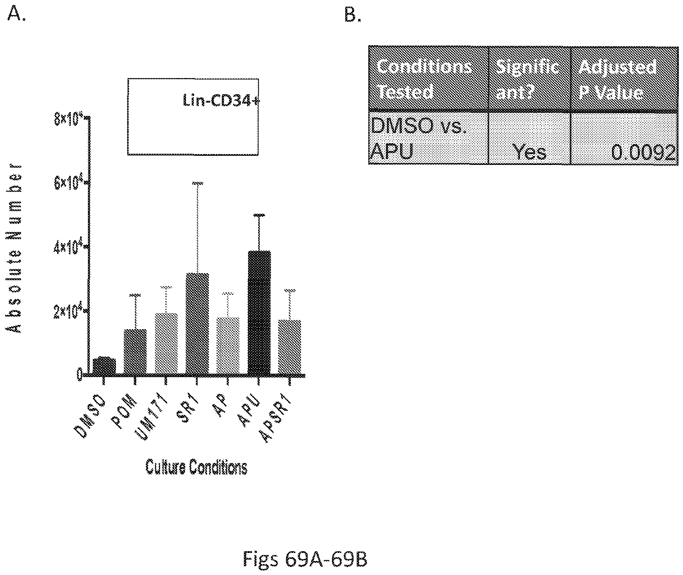

Figs. 69A-69B demonstrate the absolute number of Lin-CD34+ Cells after CD34+ bone marrow cells were cultured for 12 Days. Fig. 69A depicts the frequency of and of Lin-CD34+ cells arising from 10,000 CD34+ enriched bone marrow cells after 12 days of culture in the indicated compounds. Fig. 69B is a table of the statistically significant p values for compound expanded cells compared to DMSO expanded cells. Statistics were generated using one-way ANOVA and Tukey's multiple comparisons tests.

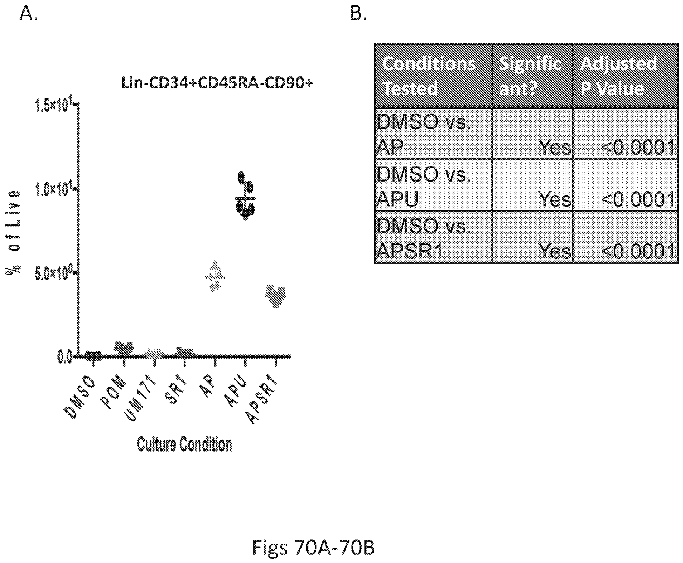

Figs. 70A-70B demonstrate the frequency of Lin-CD34+CD45RA-CD90+ HSCs after CD34+ bone marrow cells were cultured for 12 Days. Fig. 70A depicts the frequency of Lin-CD34+CD45RA-CD90+ HSCs arising from 10,000 CD34+ enriched bone marrow cells after 12 days of culture in the indicated compounds. Fig. 70B is a table of the statistically significant p values for compound expanded cells compared to DMSO expanded cells. Statistics were generated using one-way ANOVA and Tukey's multiple comparisons tests.

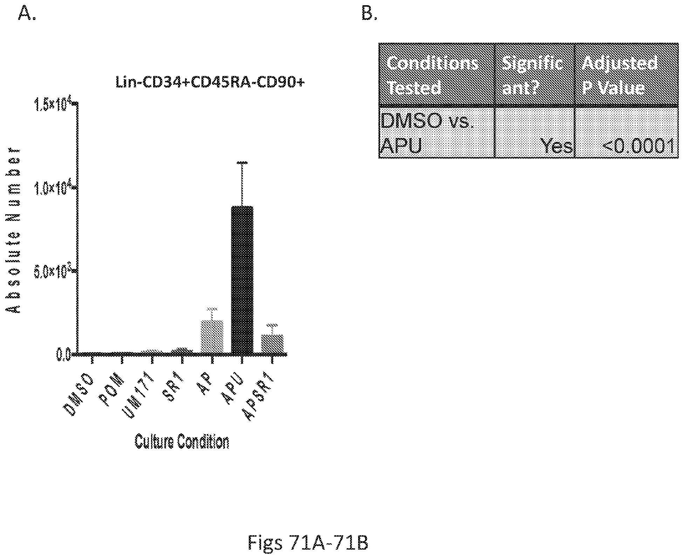

Figs. 71A-71B demonstrate the absolute number of of Lin-CD34+CD45RA-CD90+ HSCs after CD34+ bone marrow cells were cultured for 12 Days. Fig. 71A depicts the absolute number of Lin-CD34+CD45RA-CD90+ HSCs arising from 10,000 CD34+ enriched bone marrow cells after 12 days of culture in the indicated compounds. Fig. 71B is a table of the statistically significant p values for compound expanded cells compared to DMSO expanded cells. Statistics were generated using one-way ANOVA and Tukey's multiple comparisons tests.

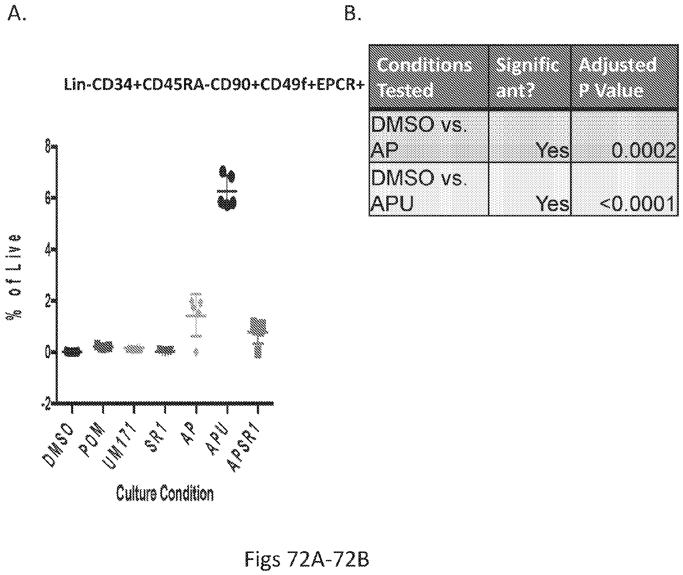

Figs. 72A-72B demonstrate the frequency of Lin-CD34+CD45RA-CD90+CD49f+EPCR+ HSCs after CD34+ bone marrow cells were cultured for 12 Days. Fig. 72A depicts the frequency of Lin-CD34+CD45RA-CD90+CD49f+EPCR+ HSCs arising from 10,000 CD34+ enriched bone marrow cells after 12 days of culture in the indicated compounds. Fig. 72B is a table of the statistically significant p values for compound expanded cells compared to DMSO expanded cells. Statistics were generated using one-way ANOVA and Tukey's multiple comparisons tests.

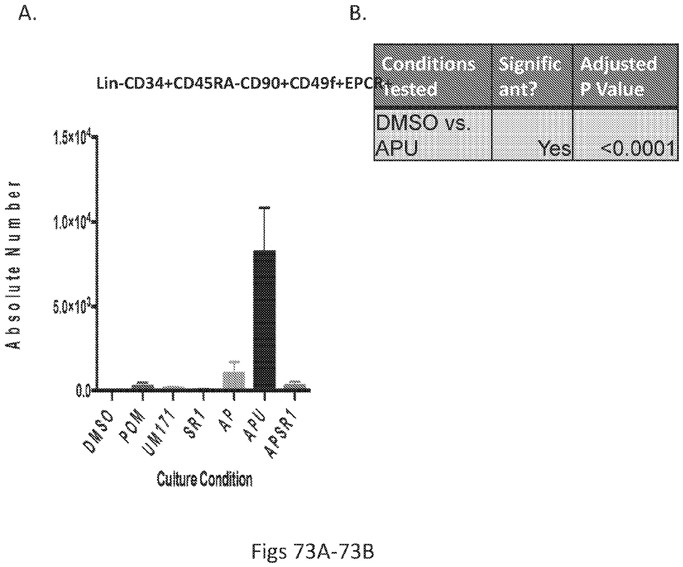

Figs. 73A-73B demonstrate the absolute number of of Lin-CD34+CD45RA-CD90+CD49f+EPCR+ HSCs after CD34+ bone marrow cells were cultured for 12 Days. Fig. 73A depicts the absolute number of Lin-CD34+CD45RA-CD90+CD49f+EPCR+ HSCs arising from 10,000 CD34+ bone marrow cells after 12 days of culture in the indicated compounds. Fig. 73B is a table of the statistically significant p values for compound expanded cells compared to DMSO expanded cells. Statistics were generated using one-way ANOVA and Tukey's multiple comparisons tests.
For Figs. 20A-73B: POM, pomalidomide; SR1, StemRegenin 1; A, A83-01; AP, A + POM; APU, A+POM+UM171; APSR1, A+POM+SR1.

### DETAILED DESCRIPTION

The present invention is based on the discovery that the *ex vivo* expansion, enrichment, and maintenance of populations of hematopoietic stem cells bearing hematopoietic stem cell functional potential can be achieved by contacting these cells with one or more agents that reduce the level of an ikaros family member transcription factor, such as a compound that activates a ubiquitin ligase that catalyzes the polyubiquitination of an ikaros transcription factor. Exemplary compounds that exhibit these biological activities include thalidomide and derivatives thereof, such as pomalidomide and lenalidomide, among others.

Using the methods described herein, hematopoietic stem cells (HSCs) may be expanded by incubating these cells with an agent that reduces the level of an ikaros family member transcription factor, optionally in combination with one or more additional agents, such as one or more agents capable of inhibiting TGFβ signaling, modulating (e.g., up-regulating or down-regulating) lysine methylation, modulating histone acetylation, inhibiting p38 signaling, and activating canonical Wnt signaling. The methods described herein can also be used to enrich a population of cells for HSCs by incubating the population with a an agent that reduces the level of an ikaros family member transcription factor, optionally in combination with one or more of the above-described additional agents. Additionally, the methods described herein can be used to maintain the hematopoietic stem cell functional potential of a population of HSCs by incubating the population with a an agent that reduces the level of an ikaros family member transcription factor, optionally in combination with one or more of the above-described additional agents.

A wide variety of structurally and mechanistically distinct agents that modulate the above biological events are known in the art. For instance, these agents may be small molecules capable of agonizing or antagonizing a particular event within a certain pathway (e.g., small molecules that inhibit enzymatic activity of proteins that propagate a signal transduction cascade). These agents may also be antibodies, such as monoclonal antibodies or antigen-binding fragments thereof, that disrupt a particular interaction (e.g., a ligand-receptor interaction) by virtue of competitively binding a particular protein and sterically precluding the association of the protein with its cognate binding partner. Other agents, such as therapeutic proteins and structurally constrained peptides, are topologically well-suited for antagonizing protein-protein interactions that occur over larger molecular surfaces and thus represent a class of inhibitors capable of intervening within signal transduction pathways at targets that have otherwise been intractable to disrupting with conventional small molecule therapeutics. Other classes of inhibitors include interfering RNA molecules, which are capable of attenuating the expression of a target gene by binding mRNA polynucleotides via complementary hydrogen bonding and, e.g., inducing the degradation of the target mRNA or sterically preventing the nucleation of ribosome assembly. The sections that follow provide examples of the types of agents useful with the compositions of the invention so as to promote hematopoietic stem cell expansion, enrichment, and maintenance of hematopoietic stem cell functional potential.

### Ikaros family member antagonists

Ikaros family transcription factor antagonists include compounds capable of reducing the concentration of an ikaros family transcription factor, such as ikaros, aiolos, and helios, in a cell (e.g., a mammalian cell, such as a human cell). Antagonists of ikaros family transcription factors also include molecules capable of directly disrupting the binding of such transcription factors to DNA, thereby attenuating the activity of the transcription factor. For instance, ikaros family transcription factors such as those listed above may function as transcription repressors, e.g., in the context of Notch target genes, such as Myc and Hes1. By disrupting the activity of ikaros family transcription factors, the transcription of these genes and expression of the encoded proteins may increase.

Agents capable of up-regulating expression of a Notch target gene by antagonizing an ikaros family transcription factor include compounds that promote the polyubiquitination, and eventual degradation, e.g., by proteolysis, of ikaros family transcription factors. Such compounds may function by activating a ubiquitin ligase that catalyzes ubiquitination of an ikaros family transcription factor. For instance, agents that can reduce the level of an ikaros family transcription factor include compounds that bind and activate cereblon, a protein that forms an E3 ubiquitin ligase and promotes the polyubiquitination of ikaros transcription factors. Exemplary cereblon ligands that may promote the activity cereblon-containing E3 ligase complexes include thalidomide, pomalidomide, lenalidomide, and derivatives thereof. These compounds and their structural analogs may be used with the methods described herein to achieve the expansion, enrichment, and maintenance of hematopoietic stem cell functional potential of populations of HSCs. Thalidomide, pomalidomide, and lenalidomide, as well as structural analogs, thereof, are described, e.g., in US 5,798,368; US 5,955,476; US 6,281,230; and US 6,335,349. According to the composition of the invention, one or more agents that reduce the level of an ikaros family member transcription factor in a cell are selected from the group consisting of pomalidomide and lenalidomide.

Exemplary compounds capable of reducing the level of an ikaros family transcription factor in a cell, e.g., by activation of a cereblon-containing E3 ubiquitin ligase, include compounds represented by formula (1): 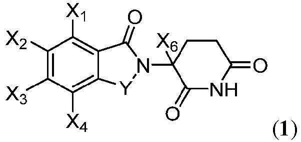
in which Y represents C=O or CH₂;
each of X₁, X₂, X₃, and X₄ is independently hydrogen or -NHX₃;
X₅ is hydrogen or an alkyl group of from 1 to 8 carbon atoms; and
X₆ is hydrogen, an alkyl group of from 1 to 8 carbon atoms, a benzyl group, or a halogen atom.

Compounds capable of reducing the level of an ikaros family transcription factor in a cell also include pharmaceutically acceptable salts of compounds of formula (1).

For instance, compounds capable of reducing the level of an ikaros family transcription factor in a cell that may be used with the methods described herein include thalidomide, pomalidomide, and lenalidomide, the structures of which are provided below.

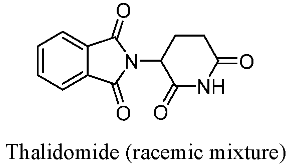

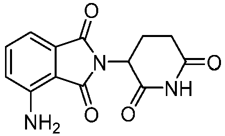

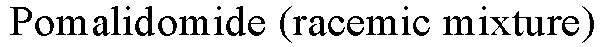

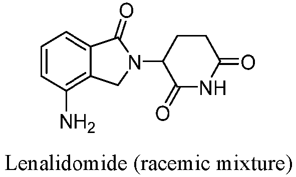

### UM171 and structural analogs thereof

Additional agents that can be used in conjunction with the methods of the disclosure include UM171, a small molecule that has been shown to induce hematopoietic stem cell expansion. UM171 is described, e.g., in Fares et al. Science 345:1509 (2014). Other agents that can be used to expand, enrich, and maintain hematopoietic stem cells include UM171 analogs, such as a UM171 structural analogs according to any one of Formulas **(I), (II), (III), (IV), (V),** and **(VI)** of US 2015/0011543. For instance, analogs of UM171 that can be used in conjunction with the compositions and methods described herein include compounds listed in Table 1, below.

**Table 1. UM171 and structural analogs thereof**

| **Compound No.** | **Molecular structure** |
|---|---|
| 5 (UM171) | 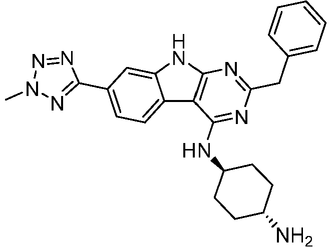 |
| 20 | 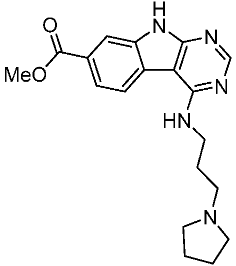 |
| 21 | 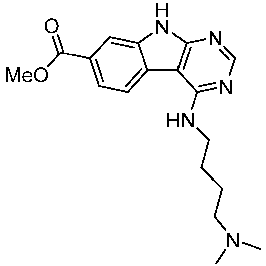 |
| 22 | 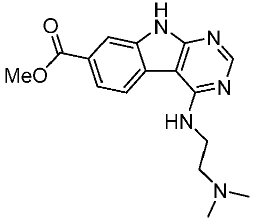 |
| 23 | 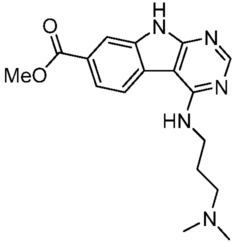 |
| 24 | 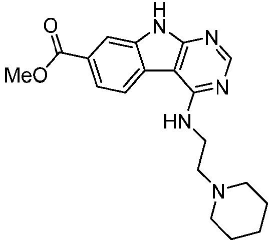 |
| 25 | 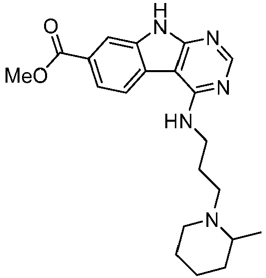 |
| 26 | 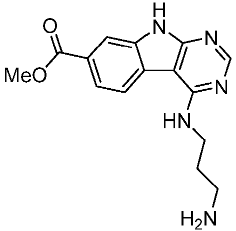 |
| 27 | 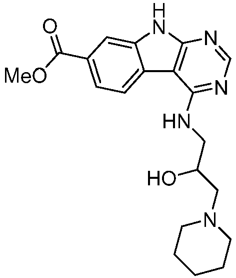 |
| 28 | 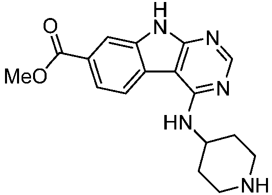 |
| 29 | 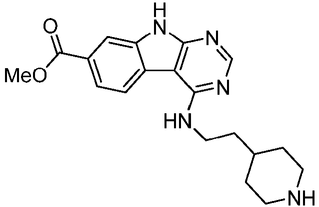 |
| 30 | 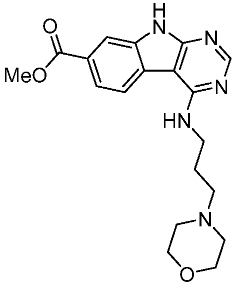 |
| 31 | 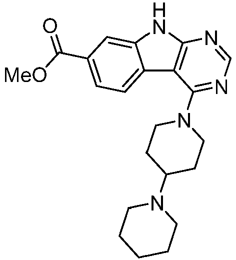 |
| 32 | 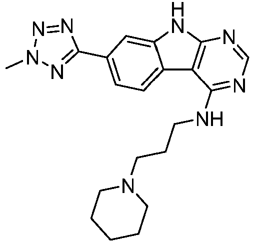 |
| 33 | 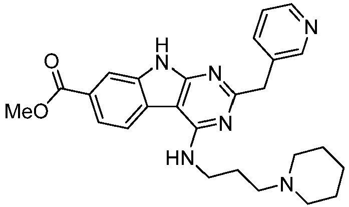 |
| 34 | 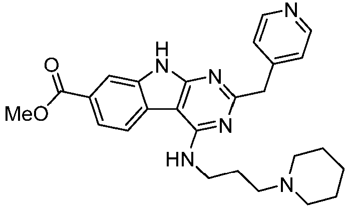 |
| 35 | 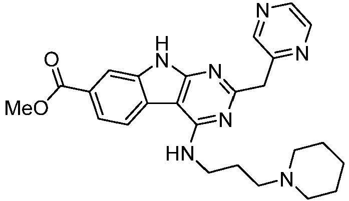 |
| 36 | 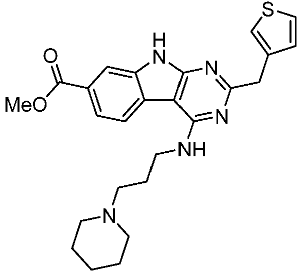 |
| 37 | 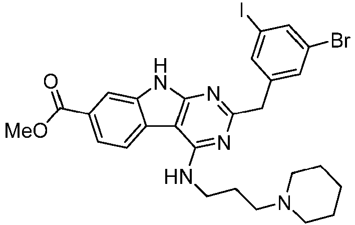 |
| 38 | 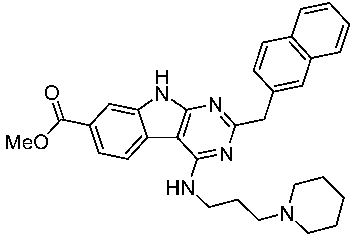 |
| 39 | 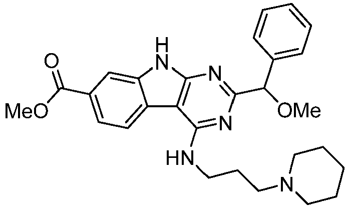 |
| 40 | 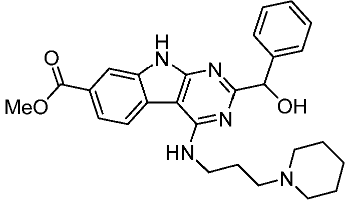 |
| 41 | 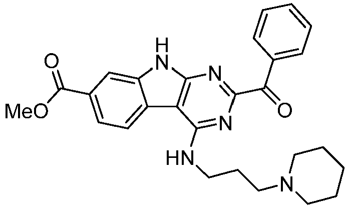 |
| 42 | 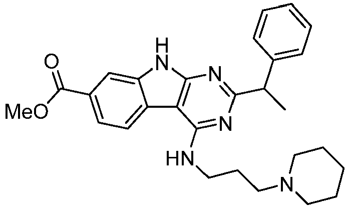 |
| 43 | 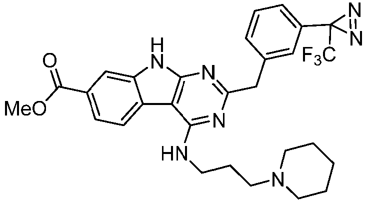 |
| 44 | 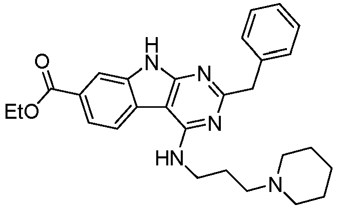 |
| 45 | 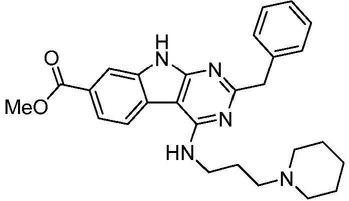 |
| 46 | 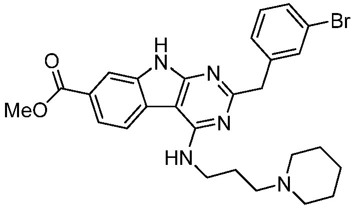 |
| 47 | 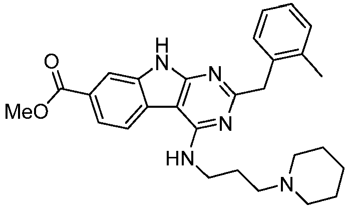 |
| 48 | 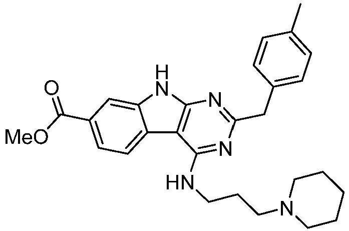 |
| 49 | 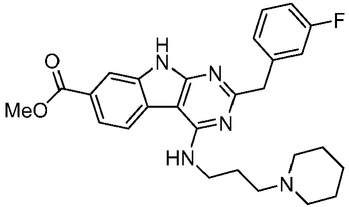 |
| 50 | 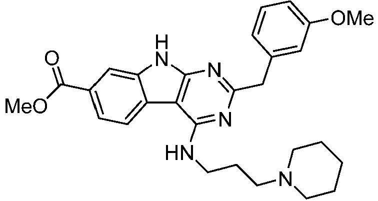 |
| 51 | 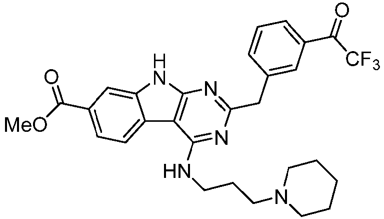 |
| 52 | 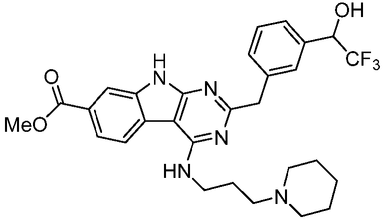 |
| 53 | 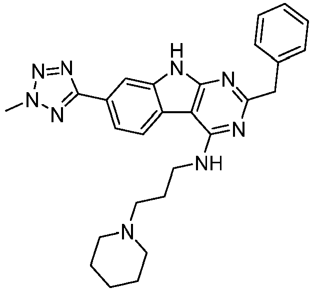 |
| 54 | 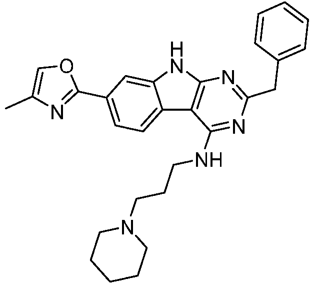 |
| 55 | 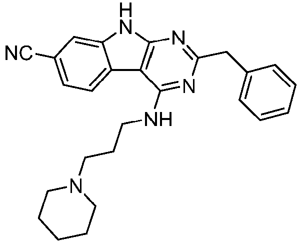 |
| 56 | 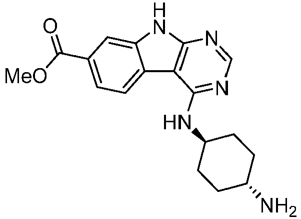 |
| 57 | 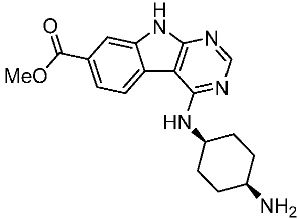 |
| 58 | 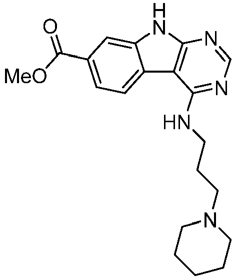 |
| 59 | 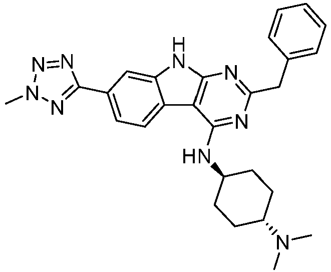 |
| 60 | 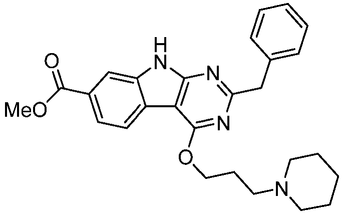 |
| 61 | 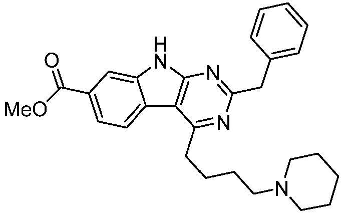 |
| 62 | 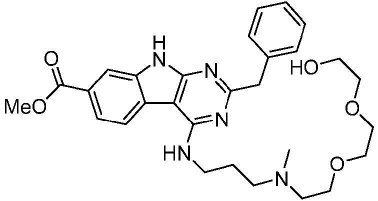 |
| 63 | 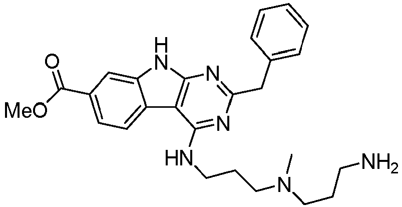 |
| 64 | 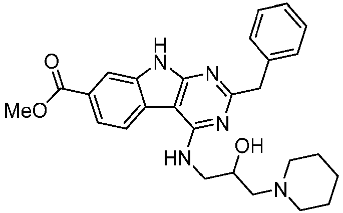 |
| 65 | 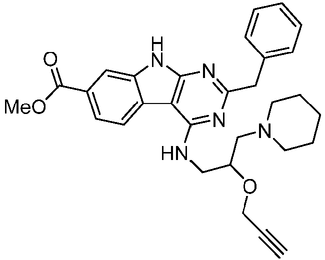 |
| 66 | 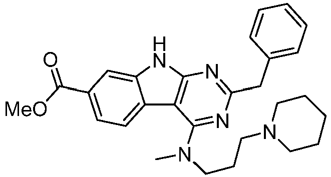 |
| 67 | 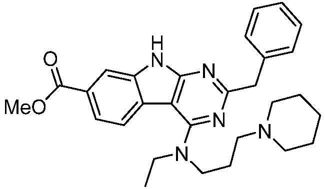 |
| 68 | 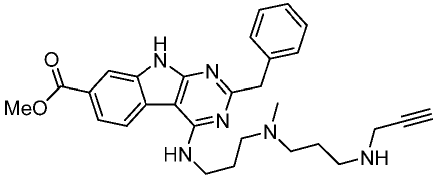 |
| 69 | 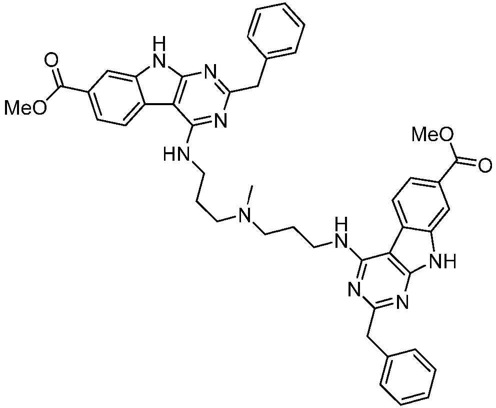 |
| 70 | 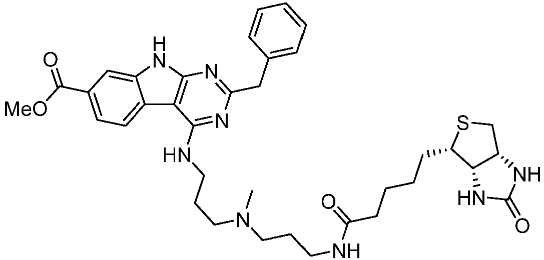 |
| 71 | 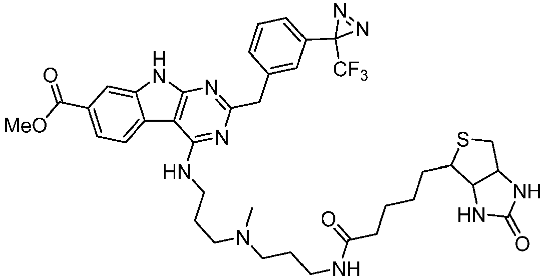 |
| 72 | 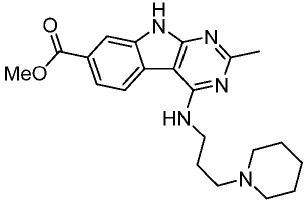 |
| 73 | 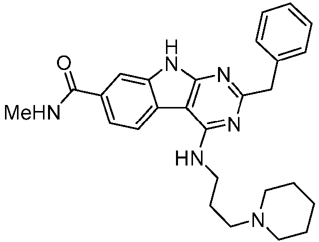 |

### Small molecules

A variety of small molecules may be used in conjunction with the methods described herein. Among these include modulators of enzyme-substrate interactions. For instance, tranylcypromine and derivatives thereof represent a robust class of inhibitors capable of irreversibly binding to and inhibiting histone demethylases such as LSD1 by virtue of forming a covalent adduct with the isoalloxazine moiety of the FAD cofactor utilized by these enzymes to catalyze oxidative demethylation of N-methylated histone tail residues. Exemplary small molecule inhibitors of histone demethylation useful with the compositions and methods of the disclosure include LSD1 inhibitor IV RN-1, LSD1 inhibitor II S2101, LSD1 inhibitor LSD1-C76, LSD1 inhibitor III CBB1007, LSD1 inhibitor I (also referred to as BHC110 Inhibitor I, Histone Lysine Demethylase Inhibitor III, and/or KDM1 Inhibitor I), and tranylcypromine, described above. Examples of small molecules useful for inhibiting histone demethylases additionally include phenelzine, propargylamine, and derivatives thereof as described in US 2013/0095067. Other tranylcypromine derivatives have been described, e.g., in US 2014/0163041.

Additional examples of small molecules that can be used to modulate histone methylation include BIX01294 (a H3K9 methylation inhibitor described in, e.g., WO 2014/057997); UNC0638 (a H3K9 methylation inhibitor described in, e.g., WO 2013/050422); and CARM1 Inhibitor (PRMT Inhibitor V, 3,5-bis(3-Bromo-4-hydroxybenzylidene)-1-benzylpiperidin-4-one, a histone arginine methyltransferase inhibitor).

Several structurally distinct classes of small molecules inhibitors of TGFβ signaling have been reported. These agents can be classified on the basis of the core molecular scaffolds of these molecules. For example, TGFβ signaling inhibitors may contain a dihydropyrrlipyrazole, imidazole, pyrazolopyridine, pyrazole, imidazopyridine, triazole, pyridopyrimidine, pyrrolopyrazole, isothiazole or oxazole functionality as the core structural fragment of the molecule. Several non-limiting examples of small molecule inhibitors of TGFβ signaling include ALK5 inhibitor II (also referred to as E-616452), LY364947 (also referred to as ALK5 Inhibitor I, TbR-I Inhibitor, Transforming Growth Factor-b Type I Receptor Kinase Inhibitor), A83-01, and DMH1. Other examples of small molecules that can be used to modulate TGFβ signaling in conjunction with the compositions and methods described herein include SB431542 (4-(5-Benzol[1,3]dioxol-5-yl-4-pyrldin-2-yl-1H-imidazol-2-yl)-benzamide hydrate, 4-[4-(1,3-Benzodioxol-5-yl)-5-(2-pyridinyl)-1H-imidazol-2-yl]-benzamide hydrate, 4-[4-(3,4-Methylenedioxyphenyl)-5-(2-pyridyl)-1H-imidazol-2-yl]-benzamide hydrate, an Alk5 inhibitor), Galunisertib (LY2157299, an Alk5 inhibitor), LY2109761 (4-[2-[4-(2-pyridin-2-yl-5,6-dihydro-4H-pyrrolo[1,2-b]pyrazol-3-yl)quinolin-7-yl]oxyethyl]morpholine, an Alk5/TGFβRII inhibitor), SB525334 (6-[2-tert-butyl-5-(6-methylpyridin-2-yl)-1H-imidazol-4-yl]quinoxaline, an Alk5 inhibitor), GW788388 (N-(oxan-4-yl)-4-[4-(5-pyridin-2-yl-1H-pyrazol-4-yl)pyridin-2-yl]benzamide, an Alk5 inhibitor), K02288 (3-[6-amino-5-(3,4,5-trimethoxyphenyl)pyridin-3-yl]phenol, an Alk4/Alk5 inhibitor), SD-208 (2-(5-chloro-2-fluorophenyl)-N-pyridin-4-ylpteridin-4-amine, an Alk5 inhibitor), EW-7197 (N-((4-([1,2,4]triazolo[1,5-a]pyridin-6-yl)-5-(6-methylpyridin-2-yl)-1H-imidazol-2-yl)methyl)-2-fluoroaniline, an Alk4/Alk5 inhibitor), and LDN-212854(5-[6-[4-(1-Piperazinyl)phenyl]pyrazolo[1,5-a]pyrimidin-3-yl]-quinoline, an Alk4/Alk5 inhibitor). According to the composition of the invention, the one or more agents that inhibit TGFβ signaling comprise a TGFβ receptor inhibitor and the TGFβ receptor inhibitor is selected from the group consisting of ALK5 inhibitor II, LY364947, DMH1, and A83-01.

Additional examples of small molecule TGFβ modulators include antagonists of TGFβ receptors, such as 2-(3-(6-Methylpyridin-2-yl)-1H-pyrazol-4-yl)-1,5 napththyridine, [3-(Pyridin-2-yl)-4-(4-quinoyl)]-1H-pyrazole, and 3-(6-Methylpyridin-2-yl)-4-(4-quinolyl)-1-phenylthiocarbamoyl-1H-pyrazole. Other small molecule inhibitors include, but are not limited to, SB-431542, **(4-[4-(1,3-**Benzodioxol-5-yl)-5-(2-pyridinyl)-1H-imidazol-2-yl]-benzamide, described in Halder et al. Neoplasia 7:509 (2005)), SM16, a small molecule inhibitor of TGFβ receptor ALK5, the structure of which is shown below (Fu et al. Arteriosclerosis, Thrombosis and Vascular Biology 28:665 (2008)), SB-505124 (an Alk4/Alk5 inhibitor, structure shown below, described in Dacosta Byfield et al. Molecular Pharmacology 65:744 (2004)), and 6-bromo-indirubin-3'-oxime (described in US 8,298,825).

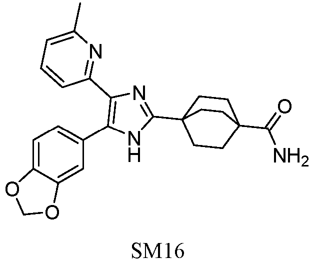

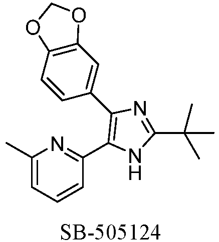

Additional examples of inhibitors of TGF-β signaling are described in, e.g., Callahan et al. Journal of Medicinal Chemistry 45:999 (2002); Sawyer et al. Journal of Medicinal Chemistry 46:3953 (2003); Gellibert et al. Journal of Medicinal Chemistry 47:4494 (2004); Tojo et al. Cancer Science 96:791 (2005); Petersen et al. Kidney International 73:705 (2008); Yingling et al. Nature Reviews Drug Discovery 3:1011 (2004); Byfield et al. Molecular Pharmacology 65:744 (2004); Dumont et al. Cancer Cell 3:531 (2003); WO 2002/094833; WO 2004/026865; WO 2004/067530; WO 2009/032667; WO 2004/013135; WO 2003/097639; WO 2007/048857; WO 2007/018818; WO 2006/018967; WO 2005/039570; WO 2000/031135; WO 1999/058128; US 6,509,318; US 6,090,383; US 6,419,928; US 7,223,766; US 6,476,031; US 6,419,928; US 7,030,125; US 6,943,191; US 2005/0245520; US 2004/0147574; US 2007/0066632; US 2003/0028905; US 2005/0032835; US 2008/0108656; US 2004/015781; US 2004/0204431; US 2006/0003929; US 2007/0155722; US 2004/0138188; and US 2009/0036382.

Another class of small molecules useful with the compositions and methods described herein include modulators of bone morphogenetic protein (BMP) signaling. BMP is a member of the TGFβ superfamily of ligands, and modulators of BMP signaling, such as inhibitors of Alk2, Alk3, and Alk6, can be used e.g., to expand, enrich, and/or maintain hematopoietic stem cells in a multi-potent state. Exemplary BMP inhibitors include DMH1 (4-[6-(4-Isopropoxyphenyl)pyrazolo[1,5-a]pyrimidin-3-yl]quinoline, 4-[6-[4-(1-Methylethoxy)phenyl]pyrazolo[1,5-a]pyrimidin-3-yl]-quinoline), K02288 (3-(6-amino-5-(3,4,5-trimethoxyphenyl)pyridin-3-yl)phenol), LDN-212854 (5-[6-[4-(1-Piperazinyl)phenyl]pyrazolo[1,5-a]pyrimidin-3-yl]-quinoline), LDN-193189 (4-[6-[4-(1-Piperazinyl)phenyl]pyrazolo[1,5-a]pyrimidin-3-yl]-quinoline), LDN-214117 (1-(4-(6-Methyl-5-(3,4,5-trimethoxyphenyl)pyridin-3-yl)phenyl)piperazine), and ML347 (5-[6-(4-methoxyphenyl)pyrazolo[1,5-a]pyrimidin-3-yl]quinoline).

Inhibitors of receptor tyrosine kinases, such as vascular endothelial growth factor (VEGF) and platelet-derived growth factor (PDGF) signaling can also be used to promote hematopoietic stem cell expansion, enrichment, and maintenance of hematopoietic stem cell functional potential. For instance, an exemplary VEGF/PDGF inhibitor useful with the methods described herein is ABT-869 (Linifanib, 1-[4-(3-amino-1H-indazol-4-yl)phenyl]-3-(2-fluoro-5-methylphenyl)urea).

Other small molecules useful with the compositions and methods described herein include inhibitors of DNA methylation, including chemical modulators of DNMT1, DNMT3a, and DNMT3B. An exemplary inhibitor of these targets that can be to expand, enrich, and maintain the hematopoietic stem cell functional potential of hematopoietic stem cells is RG108 (N-Phthalyl-L-tryptophan).

A variety of small molecule inhibitors of p38 MAPK have also been reported to date, including the pyridinylimidazole compounds SB203580 (4-(4-Fluorophenyl)-2-(4-methylsulfinylphenyl)-5-(4-pyridyl)1H-imidazole) and SB202190 (4(4-Fluorophenyl)-2-(4-hydroxyphenyl)-5-(4-pyridyl) 1H-imidazole). These compounds represent a class of inhibitors that selectively antagonize p38 MAPK α- and β-isoforms without disrupting the enzymatic activity of the γ- or δ-isoforms. These compounds are described in US 6,602,896. Other example of p38 MAPK inhibitors include SB203580, BIRB796 (Doramapimod), VX702, SB202190, LY2228820, VX745, Vinorelbine (Navelbine), PH797804, pamapimod, CMPD-1, EO1428, JX401, ML3403, RWJ67657, SB239063, SCIO469 hydrochloride, SKF86002 dihydrochloride, SX011, and TAK715, e.g., as described in US 2014/0127231. Additional examples of p38 inhibitors useful with the compositions and methods of the disclosure include Pexmetinib (ARRY-614), PH-797804 (3-(3-Bromo-4-((2,4-difluorobenzyl)oxy)-6-methyl-2-oxopyridin-1(2H)-yl)-N,4-dimethylbenzamide), Losmapimod (GW856553X), and Skepinone-L.

Small molecule agents capable of inhibiting a protein that promotes the degradation of β-catenin include those capable of attenuating the activity of proteins that promote β-catenin phosphorylation. Such inhibitors serve to increase the nuclear concentration of this transcription factor, and a variety of examples are known in the art. Inhibitors of β-catenin phosphorylation include compounds that inhibit glycogen synthase kinase 3 (GSK3), such as CHIR99021, described above, as well as 6-bromo-indirubin-3'-oxime (Meijer et al. Chemistry and Biology 10:1255 (2003); Goessling et al. Cell 136:1136 (2009)), AR-A014418 (Bhat et al. Journal of Biological Chemistry 278:45937 (2003), and the organometallic GSK-3 inhibitor DW21 (Williams et al. Angewandte Chemie International Edition 44:1984 (2005)). Other small molecule modulators of Wnt signaling useful in conjunction with the compositions and methods described herein to expand, enrich, and maintain the hematopoietic stem cell functional potential of hematopoietic stem cells include inhibitors of GSK3a and GSK3b, such as CHIR99021 and Lithium chloride.

Histone deacetylation is also amenable to targeting with small molecule therapeutics. Hydroxamic acids represent a particularly robust class of histone deacetylases that inhibit these enzymes, e.g., by virtue of hydroxamate functionality that binds cationic zinc within the active sites of these enzymes. Exemplary inhibitors include trichostatin A, described above, as well as Vorinostat (N-hydroxy-N'-phenyl-octanediamide, described in Marks et al. Nature Biotechnology 25:84 (2007) and Stenger, Community Oncology 4:384-386 (2007)). Other histone deacetylase inhibitors include Panobinostat, the structure of which is shown below.

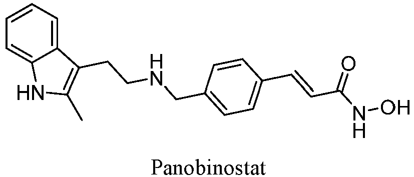

Additional examples of hydroxamic acid inhibitors of histone deacetylases include the compounds shown below, described, e.g., in Bertrand, European Journal of Medicinal Chemistry 45:2095 (2010):

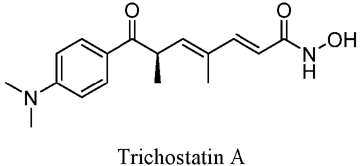

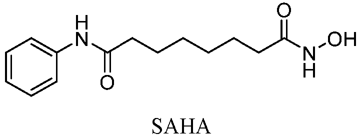

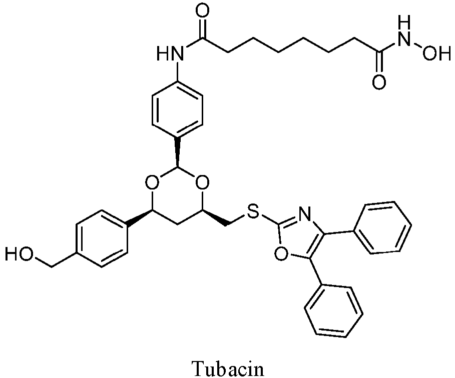

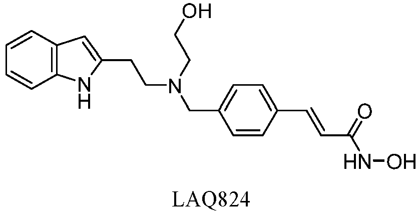

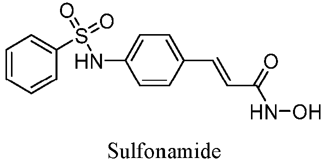

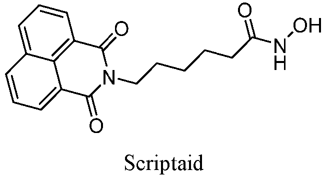

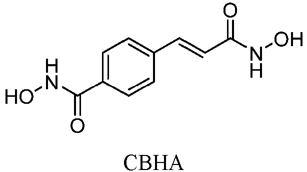

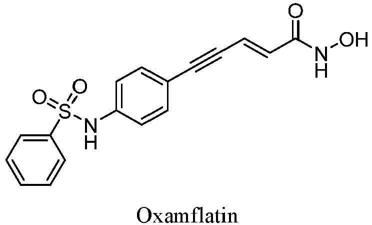

Other histone deacetylase inhibitors that do not contain a hydroxamate substituent have also been developed, including Valproic acid (Gottlicher et al. EMBO Journal 20: 6969 (2001) and Mocetinostat (N-(2-Aminophenyl)-4-[[(4-pyridin-3-ylpyrimidin-2-yl)amino]methyl]benzamide, described in Balasubramanian et al. Cancer Letters 280:211 (2009). Other small molecule inhibitors that exploit chemical functionality distinct from a hydroxamate to inhibit histone deacetylases include those shown below.

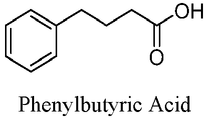

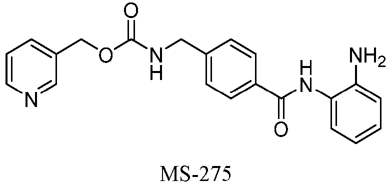

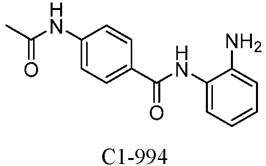

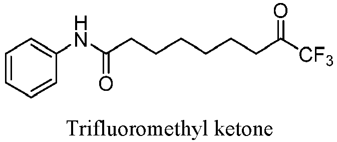

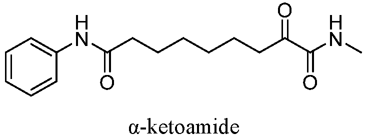

Additional examples of chemical modulators of histone acetylation useful to expand, enrich, and maintain the hematopoietic stem cell functional potential of hematopoietic stem cells include modulators of HDAC1, HDAC2, HDAC3, HDAC4, HDAC5, HDAC6, HDAC7, HDAC8, HDAC9, HDAC10, Sirt1, Sirt2, and/or HAT, such as butyrylhydroxamic acid, M344, LAQ824 (Dacinostat), AR-42, Belinostat (PXD101), CUDC-101, Scriptaid, Sodium Phenylbutyrate, Tasquinimod, Quisinostat (JNJ-26481585), Pracinostat (SB939), CUDC-907, Entinostat (MS-275), Mocetinostat (MGCD0103), Tubastatin A HCl, PCI-34051, Droxinostat, PCI-24781 (Abexinostat), RGFP966, Rocilinostat (ACY-1215), CI994 (Tacedinaline), Tubacin, RG2833 (RGFP109), Resminostat, Tubastatin A, BRD73954, BG45, 4SC-202, CAY10603, LMK-235, Nexturastat A, TMP269, HPOB, Cambinol, and Anacardic Acid.

### Antibodies and other therapeutic proteins

Antibodies represent a region of chemical space that is uniquely suited to target extracellular protein-protein interactions, such as receptor-ligand interactions. These agents possess a large molecular volume that is beneficial for inhibiting interactions that occur over vast surfaces rather than within shallow crevice. Inhibitory antibodies may function by binding an extracellular receptor in such a way that sterically precludes interaction of the receptor with the cognate ligand and thus maintains the receptor in an inactive conformation. For instance, inhibitory antibodies capable of attenuating TGFβ receptor activity include Lerdelimumab, an antibody that binds the TGFβ receptor type II. Other examples include GC-1008, an antibody that binds and antagonizes all isoforms of human TGFβ, as well as ID11, an antibody that binds all isoforms of murine TGFβ. These antibodies are described, e.g., in US 8,603,818.

Antagonist antibodies have also been developed that inhibit β-catenin phosphorylation by virtue of propagating the Wnt signal transduction cascade. Such antibodies may bind Wnt receptors such as Frizzled and LRP family proteins and trigger concomitant conformational changes that stimulate the propagation of the Wnt signaling pathway, which includes distinct molecular events that inhibit β-catenin phosphorylation by GKS3. For instance, the antibody 1D9 has been developed as an agonist of Wnt signal transduction, and is described in detail in US 2014/0044717.

Other classes of therapeutic proteins may additionally be used for inhibiting biological processes for expanding, enriching, and maintaining the hematopoietic stem cell functional potential of hematopoietic stem cells. Endogenous proteins that modulate signal transduction events may be used to attenuate these events *ex vivo,* thereby leveraging the natural affinity of these proteins for their cognate ligands in order to antagonize or stimulate important protein-protein interactions. For instance, a variety of proteins that antagonize the TGFβ signaling cascade can be used to this end, including Decorin, an extracellular matrix proteoglycan that negatively regulates TGFβ activity, as well as Lefty1, Lefty2, Follistatin, Noggin, Chordin, Cerberus, Germlin, Inhibin, Cystatin C, Recombinant Mouse Lefty-1 (an ACVR2B inhibitor), as well as the Smad proteins Smad6 and Smad7, which serve to prevent the phosphorylation of the R-Smad proteins or recruit ubiquitin ligases to the TGFβ receptor type I so as to promote the degradation of the receptor. These proteins are described in detail in US 8,298,825.

Another modulator of TGFβ signaling that may be used to expand, enrich, and maintain the hematopoietic stem cell functional potential of hematopoietic stem cells is Recombinant Amphibian TGF-β5 (an ACVR2A, ACVR2B, TGFβRII activator).

In addition to the negative feedback proteins described above, proteins capable of inducing Wnt signaling so as to inhibit β-catenin phosphorylation have also been described. For instance, Rspondin (roof plate-specific spondin) proteins are also known to activate β-catenin signaling. Rspondin proteins do not bear sequence similarity to Wnt proteins and appear to potentiate Wnt signaling through a Frizzled-independent mechanism. This protein is described in detail in Kazanskaya. O., et al., Dev. Cell 7, 525-534 (2004).

### Interfering RNA

RNA interference (RNAi) represents an inhibitory modality that exploits the ability of antagonistic RNA (e.g., double-stranded RNA containing an oligonucleotide capable of complementary hydrogen bond-mediated base pairing with an endogenous mRNA sequence) to attenuate intracellular gene expression. Mechanistically, this phenomenon often operates by way of degradation of complementary mRNA or by steric inhibition of ribosome formation at the mRNA transcript. Long stretches of dsRNAs are often cleaved in the cytoplasm of a eukaryotic cell into short 21-25 nucleotide small interfering RNAs, known as siRNAs, by the ribonuclease known Dicer. These siRNAs subsequently assemble with protein components into an RNA-induced silencing complex (RISC), unwinding in the process. Activated RISC then binds to complementary transcript by base pairing interactions between the siRNA antisense strand and the mRNA. The bound mRNA is cleaved and sequence specific degradation of mRNA results in gene silencing. The molecular events that underlie RISC-mediated gene silencing are described, e.g., in US 6,506,559; Fire et al. Nature 391:306 (1998); Timmons et al. Nature 395:854 (1998); and Hannon, RNAi: A Guide to Gene Silencing. Cold Spring Harbor Laboratory Press, Cold Spring Harbor, N.Y. (2003).

Significantly, siRNA molecules useful with the compositions and methods described herein need not be limited to those molecules containing only RNA, but may include, for example, chemically modified nucleotides and non-nucleotides that effect RNA interference, as well as molecules wherein a ribose sugar is replaced with another sugar molecule or analog thereof. Moreover, a non-natural linkage between nucleotide residues can be used, such as a phosphorothioate linkage, which is less susceptible to phosphodiesterase-mediated degradation. The RNA oligonucleotides useful with the compositions and methods described herein may also be derivatized with a reactive functional group or a reporter group, such as a fluorophore. Particularly useful derivatives are modified at a terminus or termini of an RNA strand, typically the 3' terminus of the sense strand. For example, the 2'-hydroxyl at the 3' terminus can be readily and selectively derivatized with a variety of groups by standard nucleophilic substitution techniques known in the art. Other useful RNA derivatives incorporate nucleotides having modified carbohydrate moieties, such as 2'O-alkylated residues or 2'-O-methyl ribosyl derivatives and 2'-O-fluoro ribosyl derivatives that impart oligonucleotides with enhanced structural stability. The nucleobases of siRNAs may also be chemically modified. For example, halogenated bases, such as 5-bromouracil and 5-iodouracil can be incorporated into a siRNA molecule so as to modulate the strength of hydrogen bonding interactions with target mRNA. The nucleobases may also be strategically alkylated. For instance, 7-methylguanosine can be incorporated in place of a guanosine residue. Non-natural bases that promote inhibition of target gene expression may also be incorporated into interfering RNAs. Other siRNA modifications include 2'-deoxy-2'-fluorouridine or locked nucleic acid (LNA) nucleotides and RNA duplexes containing either phosphodiester or varying numbers of phosphorothioate linkages. Such modifications are described, for example, in Braasch et al. Biochemistry 42: 7967 (2003).

Synthetic siRNA molecules can be obtained using a number of techniques known to those of skill in the art. For example, the siRNA molecule can be chemically synthesized or recombinantly produced using methods known in the art, such as using appropriately protected ribonucleoside phosphoramidites and conventional solid-phase oligonucleotide synthesis (see, e.g., Elbashir et al. Nature 411:494 (2001); Elbashir et al. Genes & Development 15:188 (2001); Harborth.et al. Journal of Cell Science 114:4557 (2001); Masters et al. Proceeding of the National Academy of Sciences USA 98:8012 (2001); and Tuschl et al. Genes & Development 13:3191 (1999)). In addition, dsRNAs can be expressed as stem loop structures encoded by plasmid vectors, retroviruses and lentiviruses (see, e.g., Paddison et al. Genes and Development 16:948 (2002); McManus et al. RNA 8:842 (2002); Paul et al. Nature Biotechnology 20:505 (2002); Miyagishi et al. Nature Biotechnology 20:497 (2002); Sui et al. Proceedings of the National Academy of Sciences USA 99:5515 (2002); Brummelkamp et al. Cancer Cell 2:243 (2002); Lee et al. Nature Biotechnology 20:500 (2002); Yu et al. Proceedings of the National Academy of Sciences USA 99:6047 (2002); Zeng et al. Molecular Cell 9:1327 (2002); Rubinson et al. Nature Genetics 33:401 (2003); Stewart et al. RNA 9:493 (2003)).

A variety of inhibitory agents that operate by a mechanism of RNA interference have been developed to antagonize the biological processes described by the methods of the disclosure. For instance, TGFβ receptor type II siRNA polynucleotides have been reported that are derived from the human TGFβRII sequence (Genbank Accession Number: M85079). The siRNA duplex sequences below were developed against particular target sequences within the TGFβ receptor type II gene and have been used to knock down expression of the receptor in a variety of whole cell models. These siRNA sequences are described in detail in US 8,067,389. Other oligonucleotide-based modulators of TGFβ signaling, such as siRNAs and antisense oligonucleotides, are described in US 5,731,424; US 6,124,449; US 2008/0015161; US 2006/0229266; US 2004/0006030; US 2005/0227936; and US 2005/0287128. siRNAs useful for targeting TGFβR or ALK5 expression can be readily designed and tested. A database of siRNA sequences and a predictor of siRNA sequences has been established (Chalk et al. (Nucleic Acids Research 33:D131 (2005)). This database can be used to predict the thermodynamic parameters of a particular siRNA-target mRNA interaction, as well as to evaluate the propensity of designed siRNA sequences for off-target interactions. The database is available as an electronic resource at www.siRNA.cgb.ki.se.

### Target Sequence

| 5' to 3' and Nucleotide number | siRNA duplex |
|---|---|
| Nt 529 | UCCUGCAUGAGCAACUGCAdTdT dTdTAGGACGUACUCGUUGACGU |
| AATCCTGCATGAGCAACTGCA (SEQ ID NO: 1) | |
| | (SEQ ID NOS: 2 and 3) |
| Nt 1113 | GGCCAAGCUGAAGCAGAACdTdT dTdTCCGGUUCGACUUCGUCUUG |
| AAGGCCAAGCTGAAGCAGAAC (SEQ ID NO: 4) | |
| | (SEQ ID NOS: 5 and 6) |
| Nt 1253 | GCAUGAGAACAUACUCCAGdTdT dTdTCGUACUCUUGUAUGAGGUC |
| AGCATGAGAACATACTCCAG (SEQ ID NO: 7) | |
| | (SEQ ID NO: 8 and 9) |
| Nt 948 | GACGCGGAAGCUCAUGGAGdTdT dTdTCUGCGCCUUCGAGUACCUC |
| AAGACGCGGAAGCTCATGGAG (SEQ ID NO: 10) | |
| | (SEQ ID NO: 11 and 12) |

### Conformationally constrained peptides

Peptide-based therapeutics represent an emerging class of compounds useful for the inhibition of protein-protein interactions that have often been intractable to inhibition by other means. Conformationally restricted peptides offer particular advantages for therapeutic applications, as these compounds often exhibit enhanced target affinity and selectivity, e.g., by virtue of presenting structurally pre-organized epitopes in which a particular pharmacophore is spatially predisposed for interacting with a protein of interest. Constrained peptides often feature additional benefits, such as enhanced protease resistivity relative to their unconstrained (e.g., linear) counterparts by restricting the access of proteases to internal amide bonds. The cell penetrating capabilities of these compounds are also frequently higher than those of linear peptides due to the sequestration of hydrogen bond donors and acceptors from aqueous solvent. Exemplary constrained peptide inhibitors useful with the compositions and methods of the disclosure include olefin "stapled" peptides, which often feature alpha helices that have been structurally rigidified by insertion of a covalent cross-link between residues on the same face of the helix. This class of constrained peptides is described, e.g., in Walensky et al. Journal of Medicinal Chemistry 57:6275 (2014). Stapled peptide inhibitors of β-catenin phosphorylation have been developed that function by disrupting the Axin/β-catenin interaction. Axin serves to anchor β-catenin to a protein complex that includes GSK3, and the association has been shown to be mediated by the insertion of an alpha helical region of Axin into a shallow pocket at the β-catenin surface. A stapled peptide of the sequence Ac-PQR₈ILDQHVS₅RVMK-NH₂ has been reported that is structurally restricted to an alpha helical conformation by virtue of an olefinic cross-link at residues R₈ ((S)-α-(7-octenyl)alanine) and S₅ ((S)-α-(4-pentenyl)alanine) (see, e.g., Cui et al. Cell Research 23:581 (2013)). This peptide competes with Axin for binding at the surface of β-catenin and serves to liberate the protein from the GSK3-containing complex, thus increasing the nuclear concentration of this transcription factor.

Constrained peptides have also been developed by virtue of covalent cyclization between the N- and C-termini. Exemplary inhibitors of this class include the depsipeptides, which feature lactone moieties that render these peptide macrocyclic. Depsipeptide inhibitors useful with the compositions and methods described herein include inhibitors of histone deacetylases, such as Romidepsin (also referred to as istodax; structure shown below), which is described in, e.g., Vinodhkumar et al. Biomedicine & Pharmacotherapy 62:85 (2008).

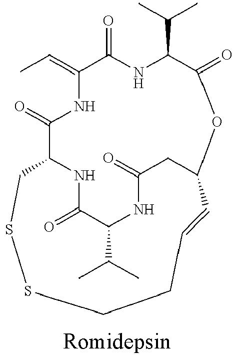

Additional examples of depsipeptide inhibitors of histone deacetylases include Apicidin, described in Bertrand, European Journal of Medicinal Chemistry 45:2095 (2010).

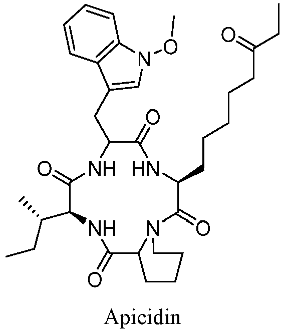

### Additional agents that can be used to induce expansion, enrichment, and maintenance of hematopoietic stem cells during ex vivo culturing

Other compounds may additionally be used to expand, enrich, and/or maintain hematopoietic stem cells during *ex vivo* culturing. Examples of these compounds include antagonists of the aryl hydrocarbon receptor (AHR), such as StemRegenin 1 (SR1), a small molecule that promotes expansion and self-renewal of human CD34+ peripheral blood and cord blood hematopoietic stem cells. SR1 has been described, e.g., in US 2014/0369973; Boitano et al. Science 1345 (2010); and Smith et al. Journal of Pharmacology and Experimental Therapeutics 338:318 (2011). Other AHR inhibitors that may be used in conjunction with the compositions and methods of the disclosure include SR1 analogs, such as those that contain various aryl and aliphatic substituents about the 6-aminopurine core (e.g., those described in US 2014/0369973). Additional examples of AHR antagonists include the stilbene derivatives (E)-l-(4'-trifluoromethylphenyl)-2-(3,5- ditrifluoromethylphenyl)-ethene, (E)-l-(4'-methoxyphenyl)-2-(3,5-dichlorophenyl)-ethene, and (E)-l-(4'-chlorophenyl)-2-(3,5-dichlorophenyl)-ethene, as described, e.g., in WO 2004/041758. Additional stilbene derivatives useful for the inhibition of the AHR include 3,5,4'-trihydroxystilbenes (e.g., resveratrols and, in particular, trans-resveratrol); 3,4,3',5-tetrahydroxystilbene (also referred to as piceatannol); 2,3',4',5'-tetrahydrostilbene (also referred to as oxyresveratrol); as well as 4,4'-dihydroxystilbenes and glycosides (e.g., galactosides, lactosides, mannosides, piceosides, and fructosides thereof) as described, e.g., in WO 1999/056737. Another exemplary AHR antagonist is 2-methyl-2H-pyrazole-3-carboxylic acid-(2-methyl-4-o-tolylazophenyl)-amide (also referred to as CH-223191, described in detail in, e.g., Kim et al. Molecular Pharmacology 69:1871 (2006); as well as in WO 2009/115807).

Additional compound that can be used in conjunction with the compositions and methods described herein include prostaglandins. Particularly useful prostaglandins include prostaglandin dmPGE2, described, e.g., in US 8,551,782 and US 8,168,428. Additional compounds that can be used in conjunction with the compositions and methods described herein include inhibitors of the Sirtuin 1 (SIRT1) protein, such as nicotinamide (described, e.g., in Peled et al. Experimental Hematology 40:342 (2012)) and cambinol (described, e.g., in Lugrin et al. Biochimica Biophysica Acta 1833:1498 (2013)).

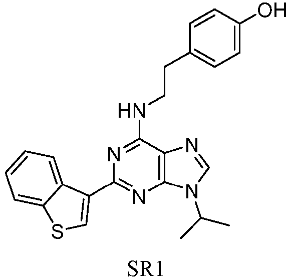

Additional agents that can be used with the compositions and methods described herein include activators of the Notch signal transduction pathway. Agonists of Notch signaling include but are not limited to proteins that contain portions of toporythmic proteins, such as Delta, Serrate, and Jagged (see, e.g., Lindsell et al. Cell 80:909 (1995)) that mediate Notch activity, and nucleic acids encoding the foregoing, as well as proteins, nucleic acids, small molecules, or derivatives thereof that regulate activity or gene expression of these proteins. Notch signaling agonists also include a protein or derivative or fragment thereof comprising a functionally active fragment, such as a fragment of a Notch ligand that mediates binding to a Notch protein.

Notch activity is promoted by the binding of Notch ligands (e.g., Delta ligands and Serrate ligands) to the extracellular portion of the Notch receptor. Endogenous Notch ligands are typically membrane-bound on adjacent cells. As such, Notch ligands for use with the compositions and methods described herein may be incubated with hematopoietic stem cells in solution as soluble protein factors or immobilized on a solid surface (e.g., a tissue culture plate, bead, or nanomatrix). For example, full length Notch ligands expressed on the surface of a cell induces the activation of the Notch signaling cascade in a neighboring cell upon contact of the ligand with the Notch receptor. Notch signaling agonists for use with the compositions and methods described herein thus include soluble, optionally truncated Delta or Serrate (e.g., Jagged) molecules that contain the extracellular domains or Notch-binding portions, as well as forms of these proteins immobilized on a surface, such as the solid surface of a tissue culture plate, water-miscible bead, or nanomatrix. Such soluble proteins can be immobilized on a solid surface by an antibody or interacting protein, for example an antibody directed to an epitope tag with which a Delta or a Serrate is expressed as a fusion protein (e.g., a myc epitope tag, which is recognized by the antibody 9E10) or a protein which interacts with an epitope tag with which a Delta or a Serrate is expressed as a fusion protein (e.g., an immunoglobulin epitope tag, which is bound by Protein A) as described in US 2014/0369973. Exemplary agonists of Notch signaling additionally include a notch polypeptide, deltex polypeptide, mastermind polypeptide, split polypeptide, hairless polypeptide, RBP-Jκ polypeptide, or hesl polypeptide as described in US 2011/0091448.

Agents such as those described above (for example, an AHR antagonist, such as SR1, optionally in combination with an AHR antagonist, dmPGE2, a Notch signaling agonist, and/or a SIRT1 inhibitor, such as nicotinamide or cambinol) may be included as additional compounds that contact hematopoietic stem cells and that are in contact with, e.g., one or more agents that reduce the level of an ikaros transcription factor member in a cell. For instance, hematopoietic stem cells in contact with one or more of these Ikaros-modulating agents may additionally be contacted with an AHR antagonist, dmPGE2, a Notch signaling agonist, and/or a SIRT1 inhibitor, such as nicotinamide or cambinol, according to distinct incubation regimens, such that one or more of these compounds are introduced to hematopoietic stem cells at various times during a culture period. Alternatively, these agents may be incubated with hematopoietic stem cells simultaneously when desired.

By way of illustrative example, Table 2 depicts certain exemplary combinations of compounds that are contemplated for use in the compositions and methods described herein. The combinations provided below herein are not meant to be limiting.

**Table 2**

| Exemplary Combinations of Compounds |
|---|
| inhibitor of an ikaros family transcription factor; |
| modulator of histone methylation; |
| inhibitor of TGFβ signaling; |
| inhibitor of p38 signaling; |
| activator of canonical Wnt signaling; |
| modulator of histone acetylation; |
| inhibitor of aryl hydrocarbon receptor signaling; and UM171 and/or a structural analog thereof |
| inhibitor of an ikaros family transcription factor; |
| inhibitor of TGFβ signaling; |
| inhibitor of aryl hydrocarbon receptor signaling; |
| UM171 and/or a structural analog thereof |
| inhibitor of an ikaros family transcription factor; |
| inhibitor of TGFβ signaling; |
| inhibitor of aryl hydrocarbon receptor signaling; |
| UM171 and/or a structural analog thereof; and one or more of: |
| modulator of histone methylation; |
| inhibitor of p38 signaling; |
| activator of canonical Wnt signaling; and |
| modulator of histone acetylation; |
| inhibitor of an ikaros family transcription factor; and |
| inhibitor of aryl hydrocarbon receptor signaling; |
| inhibitor of an ikaros family transcription factor; |
| inhibitor of TGFβ signaling; and |
| UM171 and/or a structural analog thereof |
| inhibitor of an ikaros family transcription factor; |
| inhibitor of TGFβ signaling; and |
| inhibitor of aryl hydrocarbon receptor signaling |

### Hematopoietic Stem Cell Mobilization

Hematopoietic stem cells for use with the compositions and methods of the disclosure may arise from a variety of cell types. For instance, hematopoietic cells to be used in methods of expansion, enrichment, and maintenance of hematopoietic stem cell functional potential as recited herein may be derived from mononuclear cells prior to the treatment of these cells with, e.g., one or more agents that reduce the level of an ikaros family transcription factor in a cell, such as a cereblon-activating agent (e.g.,. thalidomide, pomalidomide, or lenalidomide). Human hematopoietic stem cells may optionally be CD34+ cells prior to the treatment with one or more of these agents. For instance, human hematopoietic stem cells may be within populations with cell surface phenotypes including CD34+, CD34+CD38-, CD34+CD38-CD90+, CD34+CD38-CD90+CD45RA-, or CD34+CD38-CD90+CD45RA-CD49F+ cells prior to the treatment with one or more of these agents.

Hematopoietic stem cells may additionally are derived from human bone marrow. Alternatively, hematopoietic stem cells may be derived from human cord blood or mobilized peripheral blood. Hematopoietic stem cells obtained from human peripheral blood may be mobilized by one of a variety of strategies. Exemplary agents that can be used to induce mobilization of hematopoietic stem cells from the bone marrow into peripheral blood include chemokine (C-X-C motif) receptor 4 (CXCR4) antagonists, such as AMD3100 (also known as Plerixafor and MOZOBIL^{™} (Genzyme, Boston, MA)) and granulocyte colony-stimulating factor (GCSF), the combination of which has been shown to rapidly mobilize CD34⁺ cells in clinical experiments. Additionally, chemokine (C-X-C motif) ligand 2 (CXCL2, also referred to as GROβ) represents another agent capable of inducing hematopoietic stem cell mobilization to from bone marrow to peripheral blood. Agents capable of inducing mobilization of hematopoietic stem cells for use with the compositions and methods of the disclosure may be used in combination with one another. For instance, CXCR4 antagonists (e.g., AMD3100), CXCL2, and/or GCSF may be administered to a subject sequentially or simultaneously in a single mixture in order to induce mobilization of hematopoietic stem cells from bone marrow into peripheral blood. The use of these agents as inducers of hematopoietic stem cell mobilization is described, e.g., in Pelus, Current Opinion in Hematology 15:285 (2008).

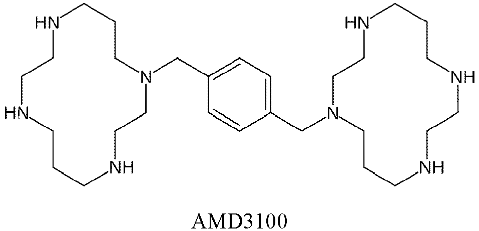

### Modulating Target Gene Expression in Hematopoietic Stem Cells

The compositions and methods described herein provide strategies for regulating the expression of target genes in populations of hematopoietic stem cells. For instance, a population of hematopoietic stem cells may be expanded, enriched or maintained *ex vivo* according to the methods described herein and may additionally be genetically modified, e.g., so as to exhibit an altered gene expression pattern. Alternatively, a population of cells may be enriched with hematopoietic stem cells, or a population of hematopoietic stem cells may be maintained in a multi-potent state, and the cells may further be modified using established genome editing techniques known in the art. For instance, one may use a genome editing procedure to promote the expression of an exogenous gene or inhibit the expression of an endogenous gene within a hematopoietic stem cell. Populations of hematopoietic stem cells may be expanded, enriched, or maintained in a multi-potent state according to the methods described herein and subsequently genetically modified so as to express a desired target gene, or populations of these cells may be genetically modified first and then expanded, enriched, or maintained in a multi-potent state. A wide array of methods has been established for the incorporation of target genes into the genome of a cell (e.g., a mammalian cell, such as a murine or human cell) so as to facilitate the expression of such genes.

### Polynucleotides encoding target genes

One example of a platform that can be used to facilitate the expression of a target gene in a hematopoietic stem cell is by the integration of the polynucleotide encoding a target gene into the nuclear genome of the cell. A variety of techniques have been developed for the introduction of exogenous genes into a eukaryotic genome. One such technique involves the insertion of a target gene into a vector, such as a viral vector. Vectors for use with the compositions and methods of the disclosure can be introduced into a cell by a variety of methods, including transformation, transfection, direct uptake, projectile bombardment, and by encapsulation of the vector in a liposome. Examples of suitable methods of transfecting or transforming cells include calcium phosphate precipitation, electroporation, microinjection, infection, lipofection and direct uptake. Such methods are described in more detail, for example, in Green, et al., Molecular Cloning: A Laboratory Manual, Fourth Edition, Cold Spring Harbor University Press, New York (2014); and Ausubel, et al., Current Protocols in Molecular Biology, John Wiley & Sons, New York (2015).

Exogenous genes can also be introduced into a mammalian cell through the use of a vector containing the gene of interest to cell membrane phospholipids. For example, vectors can be targeted to the phospholipids on the extracellular surface of the cell membrane by linking the vector molecule to a VSV-G protein, a viral protein with affinity for all cell membrane phospholipids. Viral vectors containing the VSV-G protein are described in further detail, e.g., in US 5,512,421; and in US 5,670,354.

Recognition and binding of the polynucleotide encoding a target gene by mammalian RNA polymerase is an important molecular event for gene expression to occur. As such, one may include sequence elements within the polynucleotide that exhibit a high affinity for transcription factors that recruit RNA polymerase and promote the assembly of the transcription complex at the transcription initiation site. Such sequence elements include, e.g., a mammalian promoter, the sequence of which can be recognized and bound by specific transcription initiation factors and ultimately RNA polymerase. Alternatively, promoters derived from viral genomes can be used for the stable expression of target genes in mammalian cells. Examples of functional viral promoters that can be used to promote mammalian expression of these enzymes include adenovirus late promoter, vaccinia virus 7.5K promoter, SV40 promoter, cytomegalovirus promoter, mouse mammary tumor virus (MMTV) promoter, LTR promoter of HIV, promoter of moloney virus, Epstein barr virus (EBV) promoter, Rous sarcoma virus (RSV) promoter, and the cytomegalovirus (CMV) promoter. Additional viral promoters include the SV40 late promoter from simian virus 40, the Baculovirus polyhedron enhancer/promoter element, Herpes Simplex Virus thymidine kinase (HSV tk) promoter, and the 35S promoter from Cauliflower Mosaic Virus. Suitable phage promoters for use with the compositions and methods of the disclosure include, but are not limited to, the E. coli T7 and T3 phage promoters, the S. typhimurium phage SP6 promoter, B. subtilis SP01 phage and B. subtilis phage phi 29 promoters, and N4 phage and K11 phage promoters as described in US 5,547,892.

Upon incorporation of a polynucleotide encoding a target gene has been incorporated into the genome of a cell (e.g., the nuclear genome of a hematopoietic stem cell), the transcription of this polynucleotide can be induced by methods known in the art. For example expression can be induced by exposing the mammalian cell to an external chemical reagent, such as an agent that modulates the binding of a transcription factor and/or RNA polymerase to the mammalian promoter and thus regulate gene expression. The chemical reagent can serve to facilitate the binding of RNA polymerase and/or transcription factors to the mammalian promoter, e.g., by removing a repressor protein that has bound the promoter. Alternatively, the chemical reagent can serve to enhance the affinity of the mammalian promoter for RNA polymerase and/or transcription factors such that the rate of transcription of the gene located downstream of the promoter is increased in the presence of the chemical reagent. Examples of chemical reagents that potentiate polynucleotide transcription by the above mechanisms include tetracycline and doxycycline. These reagents are commercially available (Life Technologies, Carlsbad, CA) and can be administered to a mammalian cell in order to promote gene expression according to established protocols.

Other DNA sequence elements that may be included in polynucleotides for use with the compositions and methods of the disclosure include enhancer sequences. Enhancers represent another class of regulatory elements that induce a conformational change in the polynucleotide comprising the gene of interest such that the DNA adopts a three-dimensional orientation that is favorable for binding of transcription factors and RNA polymerase at the transcription initiation site. Thus, polynucleotides for use with the compositions and methods of the disclosure include those that encode a target gene and additionally include a mammalian enhancer sequence. Many enhancer sequences are now known from mammalian genes, and examples include enhancers from the genes that encode mammalian globin, elastase, albumin, α-fetoprotein, and insulin. Enhancers for use with the compositions and methods of the disclosure also include those that are derived from the genetic material of a virus capable of infecting a eukaryotic cell. Examples include the SV40 enhancer on the late side of the replication origin (bp 100-270), the cytomegalovirus early promoter enhancer, the polyoma enhancer on the late side of the replication origin, and adenovirus enhancers. Additional enhancer sequences that induce activation of eukaryotic gene transcription are disclosed in Yaniv et al. Nature 297:17 (1982). An enhancer may be spliced into a vector containing a polynucleotide encoding a target gene, for example, at a position 5' or 3' to this gene. In a preferred orientation, the enhancer is positioned at the 5' side of the promoter, which in turn is located 5' relative to the polynucleotide encoding the target gene.

In addition to promoting high rates of transcription and translation, stable expression of an exogenous gene in a hematopoietic stem cell can be achieved by integration of the polynucleotide comprising the gene into the nuclear DNA of the cell. A variety of vectors for the delivery and integration of polynucleotides encoding exogenous proteins into the nuclear DNA of a mammalian cell have been developed. Examples of expression vectors are disclosed in, e.g., WO 1994/11026. Expression vectors for use with the compositions and methods of the disclosure contain a polynucleotide sequence that encodes a target gene, as well as, e.g., additional sequence elements used for the expression of these enzymes and/or the integration of these polynucleotide sequences into the genome of a mammalian cell. Certain vectors that can be used for the expression of target genes include plasmids that contain regulatory sequences, such as promoter and enhancer regions, which direct gene transcription. Other useful vectors for expression of target genes contain polynucleotide sequences that enhance the rate of translation of these genes or improve the stability or nuclear export of the mRNA that results from gene transcription. These sequence elements often encode features within RNA transcripts that enhance the nuclear export, cytosolic half-life, and ribosomal affinity of these molecules, e.g., 5' and 3' untranslated regions, an internal ribosomal entry site (IRES), and polyadenylation signal site in order to direct efficient transcription of the gene carried on the expression vector. Exemplary expression vectors may also contain a polynucleotide encoding a marker for selection of cells that contain such a vector. Non-limiting examples of a suitable marker include genes that encode resistance to antibiotics, such as ampicillin, chloramphenicol, kanamycin, or nourseothricin.

### Vectors for the expression of target genes

Viral genomes provide a rich source of vectors that can be used for the efficient delivery of exogenous genes into a mammalian cell. Viral genomes are particularly useful vectors for gene delivery because the polynucleotides contained within such genomes are typically incorporated into the nuclear genome of a mammalian cell by generalized or specialized transduction. These processes occur as part of the natural viral replication cycle, and often do not require added proteins or reagents in order to induce gene integration. Examples of viral vectors include a retrovirus, adenovirus (e.g., Ad5, Ad26, Ad34, Ad35, and Ad48), parvovirus (e.g., adeno-associated viruses), coronavirus, negative strand RNA viruses such as orthomyxovirus (e.g., influenza virus), rhabdovirus (e.g., rabies and vesicular stomatitis virus), paramyxovirus (e.g. measles and Sendai), positive strand RNA viruses, such as picornavirus and alphavirus, and double stranded DNA viruses including herpes virus (e.g., Herpes Simplex virus types 1 and 2, Epstein-Barr virus, cytomegalovirus), and poxvirus (e.g., vaccinia, modified vaccinia Ankara (MVA), fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D-type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996). Other examples of viral vectors include murine leukemia viruses, murine sarcoma viruses, mouse mammary tumor virus, bovine leukemia virus, feline leukemia virus, feline sarcoma virus, avian leukemia virus, human T-cell leukemia virus, baboon endogenous virus, Gibbon ape leukemia virus, Mason Pfizer monkey virus, simian immunodeficiency virus, simian sarcoma virus, Rous sarcoma virus and lentiviruses. Other examples of vectors are described in, e.g., US 5,801,030.

### Additional transfection methods

Other techniques that can be used to introduce a polynucleotide, such as DNA or RNA (e.g., mRNA, tRNA, siRNA, miRNA, shRNA, chemically modified RNA) into a mammalian cell are well known in the art. For instance, electroporation can be used to permeabilize mammalian cells by the application of an electrostatic potential. Mammalian cells, such as hematopoietic stem cells, subjected to an external electric field in this manner are subsequently predisposed to the uptake of exogenous nucleic acids. Electroporation of mammalian cells is described in detail, e.g., in Chu et al. Nucleic Acids Research 15:1311 (1987). A similar technique, Nucleofection^{™}, utilizes an applied electric field in order to stimulate the update of exogenous polynucleotides into the nucleus of a eukaryotic cell. Nucleofection^{™} and protocols useful for performing this technique are described in detail, e.g., in Distler et al. Experimental Dermatology 14:315 (2005), as well as in US 2010/0317114.

Additional techniques useful for the transfection of hematopoietic stem cells include the squeeze-poration methodology. This technique induces the rapid mechanical deformation of cells in order to stimulate the uptake of exogenous DNA through membranous pores that form in response to the applied stress. This technology is advantageous in that a vector is not required for delivery of nucleic acids into a cell, such as a hematopoietic stem cell. Squeeze-poration is described in detail, e.g., in Sharei et al. Journal of Visualized Experiments 81:e50980 (2013).

Lipofection represents another technique useful for transfection of hematopoietic stem cells. This method involves the loading of nucleic acids into a liposome, which often presents cationic functional groups, such as quaternary or protonated amines, towards the liposome exterior. This promotes electrostatic interactions between the liposome and a cell due to the anionic nature of the cell membrane, which ultimately leads to uptake of the exogenous nucleic acids, e.g., by direct fusion of the liposome with the cell membrane or by endocytosis of the complex. Lipofection is described in detail, e.g., in US 7,442,3 86. Similar techniques that exploit ionic interactions with the cell membrane to provoke the uptake of foreign nucleic acids include contacting a cell with a cationic polymer-nucleic acid complex. Exemplary cationic molecules that associate with polynucleotides so as to impart a positive charge favorable for interaction with the cell membrane include activated dendrimers (described, e.g., in Dennig, Topics in Current Chemistry 228:227 (2003)) and diethylaminoethyl (DEAE)-dextran, the use of which as a transfection agent is described in detail, e.g., in Gulick et al. Current Protocols in Molecular Biology 40:I:9.2:9.2.1 (1997). Magnetic beads are another tool that can be used to transfect hematopoietic stem cells in a mild and efficient manner, as this methodology utilizes an applied magnetic field in order to direct the uptake of nucleic acids. This technology is described in detail, e.g., in US 2010/0227406.

Another useful tool for inducing the uptake of exogenous nucleic acids by hematopoietic stem cells is laserfection, a technique that involves exposing a cell to electromagnetic radiation of a particular wavelength in order to gently permeabilize the cells and allow polynucleotides to penetrate the cell membrane. This technique is described in detail, e.g., in Rhodes et al. Methods in Cell Biology 82:309 (2007).

Microvesicles represent another potential vehicle that can be used to modify the genome of a hematopoietic stem cell according to the methods of the disclosure described herein. For instance, microvesicles that have been induced by the co-overexpression of the glycoprotein VSV-G with, e.g., a genome-modifying protein, such as a nuclease, can be used to efficiently deliver proteins into a cell that subsequently catalyze the site-specific cleavage of an endogenous polynucleotide sequence so as to prepare the genome of the cell for the covalent incorporation of a polynucleotide of interest, such as a gene or regulatory sequence. The use of such vesicles, also referred to as Gesicles, for the genetic modification of eukaryotic cells is described in detail, e.g., in Quinn et al. Genetic Modification of Target Cells by Direct Delivery of Active Protein [abstract]. In: Methylation changes in early embryonic genes in cancer [abstract], in: Proceedings of the 18th Annual Meeting of the American Society of Gene and Cell Therapy; 2015 May 13, Abstract No. 122.

### Incorporation of target genes by gene editing techniques

In addition to viral vectors, a variety of additional tools have been developed that can be used for the incorporation of exogenous genes into hematopoietic stem cells. One such method that can be used for incorporating polynucleotides encoding target genes into hematopoietic stem cells involves the use of transposons. Transposons are polynucleotides that encode transposase enzymes and contain a polynucleotide sequence or gene of interest flanked by 5' and 3' excision sites. Once a transposon has been delivered into a cell, expression of the transposase gene commences and results in active enzymes that cleave the gene of interest from the transposon. This activity is mediated by the site-specific recognition of transposon excision sites by the transposase. In certain cases, these excision sites may be terminal repeats or inverted terminal repeats. Once excised from the transposon, the gene of interest can be integrated into the genome of a mammalian cell by transposase-catalyzed cleavage of similar excision sites that exist within the nuclear genome of the cell. This allows the gene of interest to be inserted into the cleaved nuclear DNA at the complementary excision sites, and subsequent covalent ligation of the phosphodiester bonds that join the gene of interest to the DNA of the mammalian cell genome completes the incorporation process. In certain cases, the transposon may be a retrotransposon, such that the gene encoding the target gene is first transcribed to an RNA product and then reverse-transcribed to DNA before incorporation in the mammalian cell genome. Exemplary transposon systems include the piggybac transposon (described in detail in, e.g., WO 2010/085699) and the sleeping beauty transposon (described in detail in, e.g., US2005/0112764).

Another useful tool for the integration of target genes into the genome of a hematopoietic stem cell is the clustered regularly interspaced short palindromic repeats (CRISPR)/Cas system, a system that originally evolved as an adaptive defense mechanism in bacteria and archaea against viral infection. The CRISPR/Cas system includes palindromic repeat sequences within plasmid DNA and an associated Cas9 nuclease. This ensemble of DNA and protein directs site specific DNA cleavage of a target sequence by first incorporating foreign DNA into CRISPR loci. Polynucleotides containing these foreign sequences and the repeat-spacer elements of the CRISPR locus are in turn transcribed in a host cell to create a guide RNA, which can subsequently anneal to a target sequence and localize the Cas9 nuclease to this site. In this manner, highly site-specific cas9-mediated DNA cleavage can be engendered in a foreign polynucleotide because the interaction that brings cas9 within close proximity of the target DNA molecule is governed by RNA:DNA hybridization. As a result, one can theoretically design a CRISPR/Cas system to cleave any target DNA molecule of interest. This technique has been exploited in order to edit eukaryotic genomes (Hwang et al. Nature Biotechnology 31:227 (2013) and can be used as an efficient means of site-specifically editing hematopoietic stem cell genomes in order to cleave DNA prior to the incorporation of a gene encoding a target gene. The use of CRISPR/Cas to modulate gene expression has been described in, e.g., US 8,697,359. Alternative methods for site-specifically cleaving genomic DNA prior to the incorporation of a gene of interest in a hematopoietic stem cell include the use of zinc finger nucleases (ZFNs) and transcription activator-like effector nucleases (TALENs). Unlike the CRISPR/Cas system, these enzymes do not contain a guiding polynucleotide to localize to a specific target sequence. Target specificity is instead controlled by DNA binding domains within these enzymes. The use of ZFNs and TALENs in genome editing applications is described, e.g., in Urnov et al. Nature Reviews Genetics 11:636 (2010); and in Joung et al. Nature Reviews Molecular Cell Biology 14:49 (2013).

Additional genome editing techniques that can be used to incorporate polynucleotides encoding target genes into the genome of a hematopoietic stem cell include the use of ARCUS^{™} meganucleases that can be rationally designed so as to site-specifically cleave genomic DNA. The use of these enzymes for the incorporation of genes encoding target genes into the genome of a mammalian cell is advantageous in view of the defined structure-activity relationships that have been established for such enzymes. Single chain meganucleases can be modified at certain amino acid positions in order to create nucleases that selectively cleave DNA at desired locations, enabling the site-specific incorporation of a target gene into the nuclear DNA of a hematopoietic stem cell. These single-chain nucleases have been described extensively in, e.g., US 8,021,867 and US 8,445,251.

### Inducing the differentiation of hematopoietic stem cells

In certain cases, it may be desirable to expand, enrich, or maintain a population of hematopoietic stem cells according to the methods of the disclosure and subsequently induce the differentiation of these cells into a blood cell of the hematopoietic repertoire prior to infusion of the resulting cells into a recipient. This represents a useful paradigm for administering a specific blood cell type to a recipient in need thereof. Populations of hematopoietic stem cells that have been expanded, enriched, and/or maintained according to the methods of the disclosure may be subjected to various conditions in order to stimulate the differentiation of these cells into cells of the hematopoietic lineage, such as conditions that are known in the art. For instance, using established protocols, hematopoietic stem cells can be induced to differentiate into one of a multitude of blood cell types, such as common lymphoid progenitor cells, common myeloid progenitor cells, megakaryocyte-erythroid progenitor cells, granulocyte-megakaryocyte progenitor cells, granulocytes, promyelocytes, neutrophils, eosinophils, basophils, erythrocytes, reticulocytes, thrombocytes, megakaryoblasts, platelet-producing megakaryocytes, platelets, monocytes, macrophages, dendritic cells, microglia, osteoclasts, and lymphocytes, such as NK cells, B-cells and T-cells.

### Indications for Hematopoietic Stem Cell Therapy

Hematopoietic stem cells produced (e.g., expanded, enriched, or maintained in a multi-potent state) through the use of the compositions and methods of the disclosure can be used to treat a variety of human diseases. Hematopoietic stem cells or progeny thereof administered to a patient may be autologous, syngeneic, or allogeneic, and may be administered in conjunction with one or more agents that promote the expansion of a hematopoietic stem cell *in vivo.* For instance, hematopoietic stem cells or progeny thereof may be administered to a patient (e.g., a human patient) in order to treat such diseases as Acute Lymphoblastic Leukemia (ALL), Acute Myelogenous Leukemia (AML), Chronic Myelogenous Leukemia (CML), Chronic Lymphocytic Leukemia (CLL), Hodgkin Lymphoma (HL), Non-Hodgkin Lymphoma (NHL), Myelodysplastic Syndrome (MDS), Multiple myeloma, Aplastic anemia, Bone marrow failure, Myeloproliferative disorders such as Myelofibrosis, Essential thrombocytopenia or Polycythemia vera, Fanconi anemia, Dyskeratosis congenita, Common variable immune deficiency (CVID, such as CVID 1, CVID 2, CVID 3, CVID 4, CVID 5, and CVID 6), Hemophagocytic lymphohistiocystosis, Solid tumors such as Neuroblastoma, Germ cell tumors, Breast cancer, Wilms' tumor, Medulloblastoma, and Neuroectodermal tumors, Autoimmune diseases such as Scleroderma, Multiple sclerosis, Ulcerative colitis, Systemic lupus erythematosus or Type I diabetes, Human immunodeficiency virus (HIV), or protein deficiencies such as Adrenoleukodystrophy (ALD), Metachromatic leukodystrophy (MLD), Hemophilia A & B, Hurler syndrome, Hunter syndrome, Fabry disease, Gaucher disease, Epidermolysis bullosa, Amyloidosis, Globoid Cell Leukodystrophy, Sanfillipo syndrome, and Morquio syndrome.

Hematopoietic stem cells or progeny thereof can also be administered to a human patient in order to treat a genetic blood disorder, such as Sickle cell anemia, Alpha thalassemia, Beta thalassemia, Delta thalassemia, Hemoglobin E/thalassemia, Hemoglobin S/thalassemia, Hemoglobin C/thalassemia, Hemoglobin D/thalassemia, Chronic granulomatous disease (X-linked Chronic granulomatous disease, autosomal recessive (AR) chronic granulomatous disease, chronic granulomatous disease AR I NCF1,, Chronic granulomatous disease AR CYBA, Chronic granulomatous disease AR II NCF2, Chronic granulomatous disease AR III NCF4), X-linked Severe Combined Immune Deficiency (SCID), ADA SCID, IL7-RA SCID, CD3 SCID, Rag1/Rag2 SCID, Artemis SCID, CD45 SCID, Jak3 SCID, Congenital agranulocytosis,, Congenital agranulocytosis-congenital neutropenia- SCN1, Congenital agranulocytosis-congenital neutropenia-SCN2, Familial hemophagocytic lymphohistiocystosis (FHL), Familial hemophagocytic lymphohistiocytosis type 2 (FHL2, perforin mutation), Agammaglobulinemia (X-linked Agammaglobulinemia), Wiskott-Aldrich syndrome, Chediak-Higashi syndrome, Hemolytic anemia due to red cell pyruvate kinase deficiency, Paroxysmal nocturnal hemoglobinuria, X-linked Adrenoleukodystrophy (X-ALD), X-linked lymphoproliferative disease, Unicentric Castleman's Disease, Multicentric Castleman's Disease, Congenital amegakaryocytic thrombocytopenia (CAMT) type I, Reticular dysgenesis, Fanconi anemia, Acquired idiopathic sideroblastic anemia, Systemic mastocytosis, Von willebrand disease (VWD), Congenital dyserythropoietic anemia type 2, Cartilage-hair hypoplasia syndrome, Hereditary spherocytosis, Blackfan-Diamond syndrome, Shwachman-Diamond syndrome, Thrombocytopenia-absent radius syndrome, Osteopetrosis, Infantile osteopetrosis, Mucopolysaccharidoses, Lesch-Nyhan syndrome, Glycogen storage disease, Congenital mastocytosis, Omenn syndrome, X-linked Immunodysregulation, polyendocrinopathy, and enteropathy (IPEX), IPEX characterized by mutations in FOXP3, X-linked syndrome of polyendocrinopathy, immune dysfunction, and diarrhea (XPID), X-Linked Autoimmunity-Allergic Dysregulation Syndrome (XLAAD), IPEX-like syndrome, Hyper IgM type 1, Hyper IgM type 2, Hyper IgM type 3, Hyper IgM type 4, Hyper IgM type 5, X linked hyperimmunoglobulin M, Bare lymphocyte Syndrome type I, and Bare lymphocyte Syndrome type II (Bare lymphocyte Syndrome type II, MHC class I deficiency; Bare lymphocyte Syndrome type II, complementation group A; Bare lymphocyte Syndrome type II, complementation group C; Bare lymphocyte Syndrome type II complementation group D; Bare lymphocyte Syndrome type II, complementation group E). Populations of hematopoietic stem cells expanded, enriched, or maintained by the compositions and/or methods of the disclosure, as well as progeny thereof, can also be used to treat a patient suffering from a hematolymphoid malignancy, a non-hematolymphoid malignancy, or a protein deficiency. In other examples, the patient may be tissue or cell transplantation recipient, and the hematopoietic stem cells or progeny thereof are administered in order to induce tolerance to the transplanted tissue or cells.

All patents and other publications; including literature references, issued patents, published patent applications, and co-pending patent applications, cited throughout this application are for the purpose of describing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. The disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

### Examples

### Example 1. Cell culture protocol for the expansion of CD34+ cells ex vivo

Hematopoietic stem cells, such as CD34+ hematopoietic stem cells, can be expanded, enriched, and maintained ex vivo using the compositions and methods described herein. This example provides a sample protocol that can be used in conjunction with these compositions and methods.

On day 1, CD34 enriched cord blood cells (AllCells) are thawed, and 10,000 cells are plated per well (48 well tissue culture plate) in 300 µl of media composed of Stem Span SFEM II (Stemcell Technologies) supplemented with penicillin/streptomycin (100U/ml, Gibco), hSCF(100ng/ml), hTPO (100ng/ml), hIL3 (100ng/ml), hFLT3L (100ng/ml), and various combinations of small molecules at the following final concentrations (all stock solutions were prepared with DMSO as the solvent): A83-01 (1 µM, Tocris), Pomalidomide (2 µM, Selleckchem), UM171 (35 nM, ApexBio), Tranylcypromine hydrochloride (6 µM, Cayman Chemical), Trichostatin A (25 nM, Cayman Chemical), and DMSO (0.1%, Sigma).

On day 4, every well is split across 4 wells (in a 24 well tissue culture plate), each well containing a final volume of 500 µl respective media.

On day 7, day 9, and day 12, every well is split across 2 wells (in a 24 well tissue culture plate), each well containing a final volume of 500 µl respective media.

On day 14, all cells derived from each starting well (total progeny of 10,000 CD34 enriched cord blood cells) are independently combined (hereinafter "Sample"), washed, and suspended in FACS buffer (DPBS without calcium, 2 mM EDTA, 1% BSA). Approximately 1/175 of each Sample is saved for immunophenotyping and cell counting. For transplantation, one Sample is injected per sublethally irradiated NSG recipient.

Hematopoietic cells cultured according to the above protocol may be expanded, enriched for hematopoietic stem cells, exhibit maintained hematopoietic stem cell functional potential. Figures 1-19 describe the results of FACS experiments obtained from the analysis of hematopoietic cells cultured according to the methods described herein.

### Example 2.

CD34+ cord blood cells were cultured in vitro in the presence of different combinations of compounds as described herein. The percentage and total number of Lin-CD34+and Lin- cells was increased (Figs. 21; Fig. 22A-22B; Fig. 23A-23B) in the presence of POM, (pomalidomide); SR1( StemRegenin 1); A (A83-01), U (UM171); AP (A + POM); APU (A+POM+UM171) and APSR1 (A+POM+SR1). All conditions tested increase CD34+ cell number, which includes both HSCs and progenitor cells. The percentage and total numberof Lin-CD45RA-CD90+ HSCs was increased in the presence of POM; AP; APU; and APSR1 (Fig. 24, 25). The percentage of Lin-CD45RA-CD90+CD49f+EPCR+ HSCs was increased in the presence of POM; AP; APU; and APSR1 (Fig. 26, 27). Importantly, these cells retain immunophenotypic markers associated with HSCs and expand HSC numbers after twelve days of culture in vitro. As seen in the vehicle control cells (DMSO), these cells limited in number after twelve days of in vitro culture. Furthermore, the expansion of immunophenotpyic HSCs was significant in the presence of both POM and A.

The colony-forming potential of the cells in the presence of various compounds is depicted in Fig. 28A-28B. Colony forming protential measures the functional capacity of the expanded cells. Importatly, this data demonstrates that cultured cells are able to differentiate into all blood lineages, thus function is not impaired.

### Example 3.

Transplantation assays were conducted to measure HSC function of human CD34+ cord blood cells expanded in the presence of various compounds and combinations thereof as described herein (Figs. 29-38) herein. These figures demonstrate that the expanded HSCs function as they provide long-term multilineage (B cells, T cells, and myeloid cells) reconsistution of human cells in the xenotransplantation model. There were no adverse events noted during transplantation assays.

Culture in the presence of AP expands functional human hematopoietic stem cells (Fig. 39A-39B). The limit dilution assay measures the number of functional HSCs in the compound treated cells. This data demonstrates that we have a two-fold expansion in functional HSCs compared to fresh cells, indicating that AP expands HSCs. There were no adverse events noted in these assays.

### Example 4.

CD34+ cord blood cells were expanded in the presence of certain compounds and combinations thereof as described herein. The number of certain cell types after 12 days in culture was determined (Figs. 40-47). HECA staining of immunophenotypic Lin-CD34+ HSPCs was conducted, demonstrating that there was a significant Increase in HECA Expression in UM171, APU and APSR1 treated cells compared to DMSO treated cells. Also, HECA was significantly incrased on Lin-CD34+CD45RA-CD90+ HSCs from AP, APU, APSR1, APUSR1 expanded cells compared to DMSO expanded cells (Fig. 49). Increased HECA expression has been shown to increase the short-term homing of cord blood cells to the bone marrow. This is important for progenitor cells and HSCs to engraft. This data shows that cultured cells have similar or better expression of HECA compared to fresh cells, suggesting that they are able to home to the bone marrow effieciently.

### Example 5.

Compound withdrawal was performed following expansion of CD34+ enriched cord blood cells (Fig. 51) . On the day of withdrawl and 3 days after compound withdrawal, transplantation experiments were conducted. Figs. 52-56 demonstrate that following compound withdrawal, the expanded cells generally retained the activity observed in the cells which did not undergo compound withdrawal. This suggests that the compounds are not adversely affecting the engraftment and multilineage reconstitution potential of the cultured cells. There were no adverse events noted during the transplantation assays.

### Example 6.

Expansion of Human CD34+ Cells from Mobilized Peripheral Blood was demonstrated (Fig. 57A-57B, 58) The frequency of certain cell types in the expanded cell populations were determined (Figs. 59A-64B). A transplantation assay was conducted with the expanded cells (Fig. 65A-65B). This shows that the coupounds can expand progenitor cells HSCs from multiple donor sources relevant to human hematopoietic stem cell transplantation therapies. Also, these compounds do not inhibit HSC function as assayed by the in vivo reconstitution assays. There were no adverse events noted during the transplantation assays.

### Example 7.

Expansion of Human CD34+ Cells from bone marrow was demonstrated (Figs. 66A-67). The frequency of certain cell types in the expanded cell populations were determined (Figs. 68A-73B). This shows that the coupounds can expand progenitor cells and HSCs from multiple sources relevant for hematopoietic stem cell transplantation treatments in the clinic.

## Claims

1. A composition comprising:
a. one or more agents that reduce the level of an ikaros family member transcription factor in a cell, wherein the one or more agents are selected from the group consisting of pomalidomide and lenalidomide; and
b. one or more agents that inhibit TGFβ signaling, wherein the one or more agents that inhibit TGFβ signaling comprise a TGFβ receptor inhibitor, and wherein said TGFβ receptor inhibitor is selected from the group consisting of ALK5 inhibitor II, LY364947, DMH1, and A83-01.

2. The composition of claim 1, wherein the composition is for producing an expanded population of hematopoietic stem cells *ex vivo.*

3. The composition of any one of claims 1-2, wherein the composition further comprises one or more of:
c. one or more compounds listed in Table 1;
d. one or more agents that inhibit histone demethylation;
e. one or more agents that inhibit histone deacetylation; and
f. one or more agents that inhibit aryl hydrocarbon receptor signaling.

4. The composition of claim 3, wherein said one or more compounds listed in Table 1 comprise UM171.

5. The composition of claim 3 or 4, wherein said one or more agents that inhibit histone demethylation comprise a histone demethylase inhibitor, and wherein said histone demethylase inhibitor is a LSD1 inhibitor selected from the group consisting of LSD1 inhibitor IV RN-1, LSD1 inhibitor II S2101, LSD1 inhibitor LSD1-C76, LSD1 inhibitor III CBB1007, LSD1 inhibitor I, and Tranylcypromine.

6. The composition of any of claims 3-5, wherein said one or more agents that inhibit histone deacetylation comprise a histone deacetylase inhibitor selected from the group consisting of Trichostatin A, valproic acid, butyrylhydroxamic acid, and istodax.

7. The composition of any one of claims 3-6, wherein said one or more agents that inhibit aryl hydrocarbon receptor signaling comprise SR1.

8. The composition of any one of claim 1-7, said composition further comprising one or more agents selected from the group consisting of:
a. a compound that inhibits a protein that propagates p38 signaling comprising SB203580 and SB202190; and
b. a compound that inhibits a protein that promotes β-catenin degradation selected from the group consisting of CHIR99021, lithium chloride, BIO, and FGF2.

9. The composition of any one of claims 1-8, wherein said one or more agents or compounds are present in amounts that are sufficient to (i) produce a population of cells enriched with hematopoietic stem cells, or (ii) maintain the hematopoietic stem cell functional potential of said population of hematopoietic stem cells for at least two days.

10. The composition of any one of claims 1-9, wherein said one or more agents or compounds are present in an aqueous solution; or as a lyophilized solid.

11. The composition of any one of claims 1-10, wherein said one or more agents or compounds are present in amounts that are sufficient to:
(i) stimulate expansion of said population of cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture;
(ii) enrich the population of cells with hematopoietic stem cells by 10% or more relative to a population of hematopoietic stem cells that have been contacted with a prostaglandin, an agonist of Notch signaling, or an inhibitor of SIRT1 such as nicotinamide, cambinol, or an analog thereof, after seven or more days of culture;
(iii) maintain long term engraftment potential of said hematopoietic stem cells post-transplantation after having contacted said cells in culture for two or more days; or
(iv) increase expression of a Notch target gene in said population of hematopoietic stem cells, relative to a control population of hematopoietic stem cells cultured under, same conditions but without the one or more agents in any of claims 1-11, and for the same time, optionally wherein the Notch target gene is Hes1 or Myc.

## Patentansprüche

1. Zusammensetzung, umfassend:
a. ein oder mehrere Mittel, das/die den Spiegel eines Transkriptionsfaktors eines Mitglieds der Ikarosfamilie in einer Zelle reduziert/reduzieren, wobei das eine oder die mehreren Mittel ausgewählt ist/sind aus der Gruppe bestehend aus Pomalidomid und Lenalidomid; und
b. ein oder mehrere Mittel, das/die TGFβ-Signalisierung inhibiert/inhibieren, wobei das eine oder die mehreren Mittel, das/die TGFβ-Signalisierung inhibiert/inhibieren, einen TGFβ-Rezeptorinhibitor umfasst/umfassen, und wobei der TGFβ-Rezeptorinhibitor ausgewählt ist aus der Gruppe bestehend aus ALKS-Inhibitor II, LY364947, DMH1 und A83-01.

2. Zusammensetzung nach Anspruch 1, wobei die Zusammensetzung zum Produzieren einer expandierten Population hämatopoetischer Stammzellen *ex vivo* dient.

3. Zusammensetzung nach einem der Ansprüche 1 bis 2, wobei die Zusammensetzung des Weiteren einen oder mehrere der folgenden umfasst:
c. eine oder mehrere Verbindungen, die in Tabelle 1 aufgeführt ist/sind;
d. ein oder mehrere Mittel, das/die Histondemethylierung inhibiert/inhibieren;
e. ein oder mehrere Mittel, das/die Histondeacetylierung inhibiert/inhibieren; und
f. ein oder mehrere Mittel, das/die Arylkohlenwasserstoffrezeptorsignalisierung inhibiert/inhibieren.

4. Zusammensetzung nach Anspruch 3, wobei die eine oder mehreren Verbindungen, die in Tabelle 1 aufgeführt ist/sind, UM171 umfasst/umfassen.

5. Zusammensetzung nach Anspruch 3 oder 4, wobei das eine oder die mehreren Mittel, das/die Histondemethylierung inhibiert/inhibieren, einen Histondemethylaseinhibitor umfasst/umfassen, und wobei der Histondemethylaseinhibitor ein LSD1-Inhibitor ausgewählt aus der Gruppe bestehend aus LSD1-Inhibitor IV RN-1, LSD1-Inhibitor II S2101, LSD1-Inhibitor LSD1-C76, LSD1-Inhibitor III CBB1007, LSD1-Inhibitor I und Tranylcypromin ist.

6. Zusammensetzung nach einem der Ansprüche 3 bis 5, wobei das eine oder die mehreren Mittel, das/die Histondeacetylierung inhibiert/inhibieren, einen Histondeacetylaseinhibitor ausgewählt aus der Gruppe bestehend aus Trichostatin A, Valproinsäure, Butyrylhydroxamsäure und Istodax umfasst/umfassen.

7. Zusammensetzung nach einem der Ansprüche 3 bis 6, wobei das eine oder die mehreren Mittel, das/die Arylkohlenwasserstoffrezeptorsignalisierung inhibiert/inhibieren, SR1 umfasst/umfassen.

8. Zusammensetzung nach einem der Ansprüche 1 bis 7, wobei die Zusammensetzung des Weiteren ein oder mehrere Mittel ausgewählt aus der Gruppe bestehend aus folgenden umfasst:
a. einer Verbindung, die ein Protein inhibiert, das p38-Signalisierung weiterleitet, umfassend SB203580 und SB202190; und
b. einer Verbindung, die ein Protein inhibiert, das Abbau von β-Catenin fördert, ausgewählt aus der Gruppe bestehend aus CHIR99021, Lithiumchlorid, BIO und FGF2.

9. Zusammensetzung nach einem der Ansprüche 1 bis 8, wobei das eine oder die mehreren Mittel oder Verbindungen in Mengen vorhanden ist/sind, die ausreichen, um (i) eine Population von Zellen zu produzieren, die mit hämatopoetischen Stammzellen angereichert ist, oder (ii) das funktionelle Potential der hämatopoetischen Stammzelle von jener Population von hämatopoetischen Stammzellen mindestens zwei Tage lang aufrechtzuerhalten.

10. Zusammensetzung nach einem der Ansprüche 1 bis 9, wobei das eine oder die mehreren Mittel oder Verbindungen in einer wässrigen Lösung oder als lyophilisierter Feststoff vorliegt/vorliegen.

11. Zusammensetzung nach einem der Ansprüche 1 bis 10, wobei das eine oder die mehreren Mittel oder Verbindungen in Mengen vorliegt/vorliegen, die ausreichen zum:
(i) Stimulieren der Expansion der Population von Zellen um 10 % oder mehr nach sieben oder mehr Kulturtagen relativ zu einer Population von hämatopoetischen Stammzellen, die mit einem Prostaglandin, einem Agonisten der Notch-Signalisierung oder einem Inhibitor von SIRT1, wie Nicotinamid, Cambinol oder einem Analogon davon kontaktiert worden sind;
(ii) Anreichern der Population von Zellen mit hämatopoetischen Stammzellen um 10 % oder mehr nach sieben oder mehr Kulturtagen relativ zu einer Population von hämatopoetischen Stammzellen, die mit einem Prostaglandin, einem Agonisten der Notch-Signalisierung oder einem Inhibitor von SIRT1, wie Nicotinamid, Cambinol oder einem Analogon davon kontaktiert worden sind;
(iii) Aufrechterhalten des langfristigen Anwachspotentials der hämatopoetischen Stammzellen nach der Transplantation, nachdem die Zellen in Kultur zwei oder mehr Tage lang kontaktiert worden sind; oder
(iv) Steigern der Expression eines Notch-Zielgens in der Population der hämatopoetischen Stammzellen relativ zu einer Kontrollpopulation von hämatopoetischen Stammzellen, die unter denselben Bedingungen, jedoch ohne das eine oder die mehreren Mittel in einem der Ansprüche 1 bis 11 und für die gleiche Zeit kultiviert worden sind, wobei das Notch-Zielgen gegebenenfalls Hes1 oder Myc ist.

## Revendications

1. Composition comprenant :
a. un ou plusieurs agents qui réduisent le niveau d'un facteur de transcription d'un membre de la famille ikaros dans une cellule, les un ou plusieurs agents étant choisis parmi le groupe constitué du pomalidomide et du lénalidomide ; et
b. un ou plusieurs agents qui inhibent la signalisation du TGFβ, dans laquelle le ou les agents qui inhibent la signalisation du TGFβ comprennent un inhibiteur des récepteurs du TGFβ, et dans laquelle ledit inhibiteur des récepteurs du TGFβ est sélectionné parmi le groupe constitué de l'inhibiteur ALKS II, LY364947, DMH1 et A83-01.

2. Composition selon la revendication 1, dans laquelle la composition est destinée à la production d'une population expansée de cellules souches hématopoïétiques *ex vivo.*

3. Composition selon l'une quelconque des revendications 1 et 2, dans laquelle la composition est constituée en outre d'un ou plusieurs parmi :
c. un ou plusieurs composés énumérés dans le tableau 1 ;
d. un ou plusieurs agents qui inhibent la déméthylation de l'histone ;
e. un ou plusieurs agents qui inhibent la déacétylation de l'histone ; et
f. un ou plusieurs agents qui inhibent la signalisation du récepteur des hydrocarbures d'aryle.

4. Composition selon la revendication 3, dans laquelle lesdits un ou plusieurs composés énumérés dans le Tableau 1 comprennent l'UM171.

5. Composition selon la revendication 3 ou 4, dans laquelle lesdits un ou plusieurs agents qui inhibent la déméthylation de l'histone comprennent un inhibiteur de l'histone déméthylase, et dans laquelle ledit inhibiteur de l'histone déméthylase est un inhibiteur de LSD1 sélectionné à partir du groupe constitué de l'inhibiteur de LSD1 IV RN-1, de l'inhibiteur de LSD1 II S2101, de l'inhibiteur de LSD1 LSD1-C76, de l'inhibiteur de LSD1 III CBB1007, de l'inhibiteur de LSD1 I et de la tranylcypromine.

6. Composition selon l'une quelconque des revendications 3 à 5, dans laquelle lesdits un ou plusieurs agents qui inhibent la désacétylation de l'histone comprennent un inhibiteur de l'histone désacétylase sélectionné dans le groupe constitué de la trichostatine A, de l'acide valproïque, de l'acide butyrylhydroxamique et de l'istodax.

7. Composition selon l'une quelconque des revendications 3 à 6, dans laquelle lesdits un ou plusieurs agents qui inhibent la signalisation du récepteur des hydrocarbures d'aryle comprennent le SR1.

8. Composition selon l'une quelconque des revendications 1 à 7, ladite composition comprenant en outre un ou plusieurs agents sélectionnés par le groupe constitué :
a. d'un composé qui inhibe une protéine qui propage la signalisation de p38 comprenant le SB203580 et le SB202190 ; et
b. un composé qui inhibe une protéine qui favorise la dégradation de la β-caténine sélectionnée dans le groupe constitué de CHIR99021, du chlorure de lithium, de BIO et de FGF2.

9. Composition selon l'une quelconque des revendications 1 à 8, dans laquelle lesdits un ou plusieurs agents ou composés sont présents en quantités suffisantes pour (i) produire une population de cellules enrichies de cellules souches hématopoïétiques, ou (ii) maintenir le potentiel fonctionnel des cellules souches hématopoïétiques de ladite population de cellules souches hématopoïétiques pendant au moins deux jours.

10. Composition selon l'une quelconque des revendications 1 à 9, dans laquelle lesdits un ou plusieurs agents ou composés sont présents dans une solution aqueuse ou sous la forme d'un solide lyophilisé.

11. Composition selon l'une quelconque des revendications 1 à 10, dans laquelle lesdits un ou plusieurs agents ou composés sont présents en quantités qui sont suffisantes pour :
(i) stimuler l'expansion de ladite population de cellules par 10 % ou plus par rapport à une population de cellules souches hématopoïétiques qui ont été mises en contact avec une prostaglandine, un agoniste de la signalisation Notch ou un inhibiteur de SIRT1 tel que le nicotinamide, le cambinol ou un analogue de ceux-ci, après sept jours ou plus de culture ;
(ii) enrichir la population de cellules avec des cellules souches hématopoïétiques par 10 % ou plus par rapport à une population de cellules souches hématopoïétiques qui ont été mises en contact avec une prostaglandine, un agoniste de la signalisation Notch ou un inhibiteur de SIRT1 tel que le nicotinamide, le cambinol ou un analogue de ceux-ci, après sept jours ou plus de culture ;
(iii) maintenir le potentiel de prise de greffe à long terme desdites cellules souches hématopoïétiques après la greffe, après avoir été en contact avec lesdites cellules en culture pendant deux jours ou plus ; ou
(iv) augmenter l'expression d'un gène cible Notch dans ladite population de cellules souches hématopoïétiques, par rapport à une population témoin de cellules souches hématopoïétiques cultivées dans les mêmes conditions, mais sans les un ou plusieurs agents selon l'une quelconque des revendications 1 à 11, et pour la même durée, éventuellement dans laquelle le gène cible Notch est Hes1 ou Myc.
